(19) Europäisches Patentamt
European Patent Office
Office européen des brevets

(11) EP 3 954 395 A1

# (12) EUROPEAN PATENT APPLICATION
published in accordance with Art. 153(4) EPC

(43) Date of publication:
**16.02.2022 Bulletin 2022/07**

(21) Application number: **20788494.1**

(22) Date of filing: **08.04.2020**

(51) International Patent Classification (IPC):
***A61K 48/00*** (2006.01) ***A61P 3/10*** (2006.01)
***A61P 7/00*** (2006.01) ***A61P 9/00*** (2006.01)
***A61P 9/06*** (2006.01) ***A61P 21/00*** (2006.01)
***A61P 21/04*** (2006.01) ***A61P 25/28*** (2006.01)
***A61P 29/00*** (2006.01) ***A61K 47/56*** (2017.01)
***C12N 15/113*** (2010.01) ***A61K 31/713*** (2006.01)

(52) Cooperative Patent Classification (CPC):
**A61K 31/713; A61K 47/56; A61K 48/00; A61P 3/10; A61P 7/00; A61P 9/00; A61P 9/06; A61P 21/00; A61P 21/04; A61P 25/28; A61P 29/00; C12N 15/113**

(86) International application number:
**PCT/JP2020/015818**

(87) International publication number:
**WO 2020/209285 (15.10.2020 Gazette 2020/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2019 JP 2019073832**

(71) Applicant: **National University Corporation Tokyo Medical and Dental University**
**Tokyo 113-8510 (JP)**

(72) Inventors:
- **YOKOTA, Takanori**
  **Tokyo 113-8510 (JP)**
- **NAGATA, Tetsuya**
  **Tokyo 113-8510 (JP)**

(74) Representative: **Mewburn Ellis LLP**
**Aurora Building**
**Counterslip**
**Bristol BS1 6BX (GB)**

## (54) PHARMACEUTICAL COMPOSITION FOR MUSCLE DISEASE TREATMENT

(57) A nucleic acid complex that exhibits an excellent antisense effect in the skeletal muscle and/or heart muscle, and a composition for treating or preventing a muscle disease that develops in the skeletal muscle, heart muscle, and the like comprising the nucleic acid complex as an active ingredient is to be developed. A double-stranded nucleic acid complex in which a first nucleic acid strand that hybridizes to the transcription product of a target gene and has an antisense effect on the transcription product is annealed with a second nucleic acid strand that comprises a base sequence complementary to the first nucleic acid strand and is bound to cholesterol or analog thereof is provided.

EP 3 954 395 A1

## Description

### Technical Field

The present invention relates to a double-stranded nucleic acid complex capable of specifically suppressing the expression of a target gene expressed in the skeletal muscle and heart muscle, and a pharmaceutical composition for treating or preventing muscle disease comprising the same as an active ingredient.

### Background Art

Muscular dystrophy, which is a type of muscle disease, is a progressive hereditary muscle disease that causes muscle atrophy and muscular weakness due to degeneration or necrosis of muscle fibers in skeletal muscles. Increase in severity will lead to motor function impairment such as difficulty in walking, or in many cases even to death due to respiratory failure and cardiac failure. Various disease types of muscular dystrophy are known, such as Duchenne type, Becker type, limb-girdle type, and facioscapulohumeral type, depending on the mode of inheritance and clinical manifestations (Non-Patent Literature 1).

There has been so far no radical treatment for muscular dystrophy, and most cases are treated symptomatically. For example, in the case of Duchenne muscular dystrophy, steroids have been used conventionally to treat skeletal muscle disorders. In February 2017, the U.S. Food and Drug Administration (FDA) approved deflazacort (product name: Emflaza®) as a therapeutic drug for Duchenne muscular dystrophy (DMD) in children of age 5 and older and adults. This therapeutic drug is a corticosteroid agent that reduces the immune system activity and suppresses inflammation. Also in September 2016, the U.S. FDA further approved eteplirsen (product name: EXONDYS 51) as a therapeutic drug for Duchenne muscular dystrophy. This therapeutic drug is a nucleic acid medicine designed to induce exon skipping in pre-mRNA splicing at the time of expression of a dystrophin gene such that mRNA lacking the 51st exon is synthesized.

The prevalence rate of muscular dystrophy is said to be 17 to 20 per a population of 100,000 people. The market size of related therapeutic drugs is growing every year, and is estimated to reach 78.5 billion dollars by 2022.

In recent years, oligonucleotides have attracted much interest in the development of a pharmaceutical, called nucleic acid medicine. In particular, because of high selectivity for a target gene and low toxicity, the development of nucleic acid medicine utilizing an antisense method is being actively promoted. The antisense method is a method in which expression of the protein encoded by a target gene is selectively modified or inhibited by introducing an oligonucleotide complementary to a partial sequence of mRNA or miRNA transcribed from the target gene as the target sense strand (antisense oligonucleotide: herein often referred to as "ASO") into a cell.

As a nucleic acid utilizing the antisense method, the present inventors have so far developed a double-stranded nucleic acid complex in which an antisense oligonucleotide and a complementary strand thereto are annealed. For example, Patent Literature 1 discloses that an antisense oligonucleotide annealed with a tocopherol-bound complementary strand is efficiently delivered to the liver, and exhibits a high antisense effect. Further, in Patent Literature 2 a double-stranded antisense oligonucleotide having an exon skipping effect as a short gapmer antisense oligonucleotide in which additional nucleotide(s) is (are) added to 5' end, 3' end, or both 5' end and 3' end of a gapmer (antisense oligonucleotide) is developed. In addition, in Patent Literature 3 a double-stranded agent for delivering a therapeutic oligonucleotide is also developed.

As mentioned above, most deaths from muscular dystrophy are caused by respiratory failure or cardiac failure due to expression of a mutated gene. If the expression of such a gene expressed in skeletal muscles such as the diaphragm, or in the heart muscle can be controlled by the aforedescribed nucleic acid medicine, it might be possible to decrease mortality from muscular dystrophy. Further, the same approach may be used to treat or prevent other muscle diseases such as myopathy and cardiac myopathy.

However, a nucleic acid complex that is efficiently delivered to the skeletal muscle or heart muscle, and exhibits an excellent antisense effect at the site, has not been developed to date.

### Citation List

### Patent Literature

Patent Literature 1: WO2013/089283
Patent Literature 2: WO2014/203518
Patent Literature 3: WO2014/192310

Non-Patent Literature

Non-Patent Literature 1: Principle of Myology, edited by Sugita Hideo, Ozawa Eijiro, and Nonaka Ikuya, 1995, Nankodo Co., Ltd., Tokyo: pp469-550

Summary of Invention

Technical Problem

An object of the present invention is to develop a nucleic acid complex that is efficiently delivered to a skeletal muscle and/or a heart muscle and exhibits an excellent antisense effect at the site. Another object is to develop a composition for treating or preventing a muscle disease that occurs in a skeletal muscle, a heart muscle, and the like comprising such a nucleic acid complex as an active ingredient.

Solution to Problem

In order to achieve the aforedescribed objects, the inventors have diligently conducted investigations and found that a double-stranded nucleic acid complex consisting of an antisense oligonucleotide and a complementary strand to which a lipid, especially cholesterol, is bound, which was previously thought to be mainly delivered to the liver, can also be efficiently delivered to the skeletal muscle and heart muscle to exhibit a supreme antisense effect at the sites.

Therefore, by designing the target gene of a double-stranded nucleic acid complex (double-stranded nucleic acid agent) having the above configuration for the causative gene of a muscle disease expressed in a skeletal muscle and/or a heart muscle, the antisense oligonucleotide can be efficiently delivered to the skeletal muscle and/or heart muscle to regulate the expression of the target gene, thereby also providing a composition for treating or preventing a muscle disease. The present invention is based on the above findings and development results, and provides the following.

(1) A double-stranded nucleic acid complex for suppressing or increasing the expression level of a transcription product or a translation product of a target gene, or inhibiting the function of a transcription product or a translation product of a target gene in the skeletal muscle or heart muscle of a subject, the complex comprising a first nucleic acid strand and a second nucleic acid strand, wherein said first nucleic acid strand comprises a base sequence that is capable of hybridizing to all or part of said transcription product of the target gene, and has an antisense effect on said transcription product, said second nucleic acid strand comprises a base sequence complementary to said first nucleic acid strand, and is bound to cholesterol or analog thereof, and said first nucleic acid strand is annealed to said second nucleic acid strand.
(2) The double-stranded nucleic acid complex according to (1), wherein said first nucleic acid strand comprises at least four consecutive deoxyribonucleosides.
(3) The double-stranded nucleic acid complex according to (2), wherein said first nucleic acid strand is a gapmer.
(4) The double-stranded nucleic acid complex according to (1) or (2), wherein said first nucleic acid strand is a mixmer.
(5) The double-stranded nucleic acid complex according to any one of (1) to (4), wherein said second nucleic acid strand comprises at least four consecutive ribonucleosides complementary to at least four consecutive deoxyribonucleosides in said first nucleic acid strand.
(6) The double-stranded nucleic acid complex according to any one of (1) to (5), wherein said second nucleic acid strand does not comprise a natural ribonucleoside.
(7) The double-stranded nucleic acid complex according to any one of (1) to (6), wherein the nucleic acid portion of said second nucleic acid strand consists of deoxyribonucleosides and/or sugar-modified nucleosides linked by modified or unmodified internucleoside linkages.
(8) The double-stranded nucleic acid complex according to any one of (1) to (7), wherein said second nucleic acid strand is bound to cholesterol or analog thereof.
(9) The double-stranded nucleic acid complex according to any one of (1) to (8), wherein said cholesterol or analog thereof is bound to 5' end and/or 3' end of said second nucleic acid strand.
(10) The double-stranded nucleic acid complex according to any one of (1) to (9), wherein said second nucleic acid strand is bound to a ligand via a cleavable or uncleavable linker.

(11) The double-stranded nucleic acid complex according to any one of (1) to (10), wherein said first nucleic acid

strand is bound to said second nucleic acid strand via said linker.

(12) The double-stranded nucleic acid complex according to (10) or (11), wherein said linker consists of nucleic acids.

(13) A pharmaceutical composition comprising the double-stranded nucleic acid complex according to any one of (1) to (12) as an active ingredient.

(14) The pharmaceutical composition according to (13) for treating skeletal muscle dysfunction, or cardiac dysfunction of a subject.

(15) The pharmaceutical composition according to (13) or (14), wherein the skeletal muscle dysfunction or cardiac dysfunction is a disease selected from the group consisting of muscular dystrophy, myopathy, inflammatory myopathy, polymyositis, dermatomyositis, Danon disease, myasthenic syndrome, mitochondrial disease, myoglobinuria, glycogen storage disease, periodic paralysis, hereditary cardiomyopathy, hypertrophic cardiomyopathy, dilated cardiomyopathy, hereditary arrhythmia, neurodegenerative disorder, sarcopenia, and cachexia,.

(16) The pharmaceutical composition according to any one of (13) to (15) wherein the pharmaceutical composition is administered by intravenous, intramuscular, or subcutaneous administration.

(17) The pharmaceutical composition according to any one of (13) to (16), wherein a single dose of said double-stranded nucleic acid complex is 0.1 mg/kg or more.

(18) The pharmaceutical composition according to any one of (13) to (17), wherein a single dose of said double-stranded nucleic acid complex is from 0.01 mg/kg to 200 mg/kg.

(19) The pharmaceutical composition according to any one of (13) to (18), wherein the transcription product of the target gene is an RNA selected from the group consisting of mRNA, microRNA, pre-mRNA, long non-coding RNA, and natural antisense RNA.

(20) The pharmaceutical composition according to any one of (13) to (19), wherein the first nucleic acid strand is an RNA selected from the group consisting of steric blocking, splicing switch, exon skipping, and exon inclusion.

(21) The pharmaceutical composition according to any one of (13) to (20), wherein the base sequence of the first nucleic acid strand in said double-stranded nucleic acid complex is represented by SEQ ID NO: 24.

(22) A double-stranded nucleic acid complex for inducing RNA editing, exon skipping, or exon inclusion of a target gene, or causing steric blocking of a target RNA in the skeletal muscle or heart muscle of a subject, the complex comprising a first nucleic acid strand and a second nucleic acid strand, wherein said first nucleic acid strand comprises a base sequence that is capable of hybridizing to all or part of the transcription product of said target gene, and has an antisense effect on said transcription product, said second nucleic acid strand comprises a base sequence that is complementary to said first nucleic acid strand, and said first nucleic acid strand is annealed to said second nucleic acid strand.

(23) The double-stranded nucleic acid complex according to (22), wherein said first nucleic acid strand comprises at least one morpholino nucleic acid or nucleic acid modified at the 2'-position of the ribose.

(24) The double-stranded nucleic acid complex according to (22) or (23), wherein 50% or more of bases in said first nucleic acid strand are morpholino nucleic acids or nucleic acids modified at the 2'-position of the ribose.

(25) The double-stranded nucleic acid complex according to any one of (22) to (24), wherein said first nucleic acid strand is a mixmer.

(26) The double-stranded nucleic acid complex according to any one of (22) to (25), wherein 100% of bases in said first nucleic acid strand are morpholino nucleic acids or nucleic acids modified at the 2'-position of the ribose.

(27) The double-stranded nucleic acid complex according to any one of (22) to (26), wherein said second nucleic acid strand does not comprise a natural ribonucleoside.

(28) The double-stranded nucleic acid complex according to any one of (22) to (27), wherein the nucleic acid portion of said second nucleic acid strand consists of deoxyribonucleosides and/or sugar-modified nucleosides linked by modified or unmodified internucleoside linkages.

(29) The double-stranded nucleic acid complex according to any one of (22) to (28), wherein said second nucleic acid strand is bound to a functional moiety.

(30) The double-stranded nucleic acid complex according to any one of (22) to (28), wherein said functional moiety is selected from the group consisting of cholesterol or analog thereof, tocopherol or analog thereof, phosphatidylethanolamine or analog thereof, a substituted or unsubstituted C1-C30 alkyl group, a substituted or unsubstituted C2-C30 alkenyl group, and a substituted or unsubstituted C1-C30 alkoxy group.

(31) The double-stranded nucleic acid complex according to (30), wherein said functional moiety is cholesterol or analog thereof.

(32) The double-stranded nucleic acid complex according to any one of (22) to (31), wherein said cholesterol or analog thereof is bound to 5' end and/or 3' end of said second nucleic acid strand.

(33) The double-stranded nucleic acid complex according to any one of (22) to (32), wherein said second nucleic acid strand is bound to a ligand via a cleavable or uncleavable linker.

(34) A pharmaceutical composition comprising the double-stranded nucleic acid complex according to any one of (22) to (33) as an active ingredient.

(35) The pharmaceutical composition according to (34) for treating muscular dystrophy of a subject.

(36) The pharmaceutical composition according to (35), wherein said muscular dystrophy is myotonic dystrophy or Duchenne muscular dystrophy.

(37) The pharmaceutical composition according to any one of (34) to (36) wherein the pharmaceutical composition is administered by intravenous or subcutaneous administration.

(38) The pharmaceutical composition according to any one of (34) to (37), wherein a single dose of said double-stranded nucleic acid complex is 0.1 mg/kg or more.

(39) The pharmaceutical composition according to any one of (34) to (38), wherein a single dose of said double-stranded nucleic acid complex is from 0.01 mg/kg to 200 mg/kg.

(40) The pharmaceutical composition according to any one of (34) to (39), wherein the base sequence of the first nucleic acid strand in said double-stranded nucleic acid complex is represented by any one of SEQ ID NOs: 25 to 28.

The entire contents of the disclosures in Japanese Patent Application No. 2019-073832 which forms the basis for priority of the present application are incorporated herein.

## Advantageous Effects of Invention

The present invention provides a double-stranded nucleic acid complex that enables delivery of a double-stranded nucleic acid complex agent to the skeletal muscle and heart muscle, and exhibits an antisense effect at the sites. The antisense effect allows for suppression or enhancement of expression, functional inhibition, or induction of exon skipping for a target gene.

## Brief Description of Drawings

[Figure 1] Figure 1 is schematic diagrams of a representative example of the double-stranded nucleic acid complex of the present invention. Figure 1a shows a double-stranded nucleic acid complex in which tocopherol is bound to the 5' end of the second nucleic acid strand. Figure 1b shows a double-stranded nucleic acid complex in which cholesterol is bound to the 5' end of the second nucleic acid strand. Figure 1c shows a self-annealed product of a single-stranded nucleic acid obtained by linking the double-stranded nucleic acid complex in Figure 1b with an RNA linker. Although not illustrated here, cholesterol or analog thereof may be bound to the 3' end of the second nucleic acid strand. Also, cholesterol or analog thereof may be bound to both the ends of the second nucleic acid strand. Further, cholesterol or analog thereof may be bound to a nucleotide in the interior part of the second nucleic acid strand, or to the RNA region of the single-stranded nucleic acid.

[Figure 2] Figure 2 shows the structures of various bridged nucleic acids.

[Figure 3] Figure 3 shows inhibitory effects of double-stranded nucleic acid complexes of the present invention (Toc#1HDO(mSR-B1) and Chol#1HDO(mSR-B1), respectively), in which tocopherol or cholesterol is bound to the second nucleic acid strand, on the expression of the target SR-B1 gene in the heart muscle (Heart), quadriceps muscle (Quadriceps), diaphragm (Diaphragm), and musculi dorsi proprii (Back). In the Figure, ASO means ASO(mSR-B1), which is a single-stranded nucleic acid molecule used as a positive control, and PBS means PBS used as a solvent, which is used as a negative control.

[Figure 4] Figure 4 shows inhibitory effects of the double-stranded nucleic acid complexes of the present invention (Toc#1HDO(mMalat1) and Chol#1HDO(mMalat1), respectively), in which tocopherol or cholesterol is bound to the second nucleic acid strand, on the expression of the target Malat1 gene in the heart muscle (Heart), quadriceps muscle (Quadriceps), diaphragm (Diaphragm), and musculi dorsi proprii (Back). In the Figure, ASO means ASO(mMalat1), which is a single-stranded nucleic acid molecule used as a positive control, and PBS means PBS used as a solvent, which is used as a negative control.

[Figure 5] Figure 5 shows inhibitory effects of double-stranded nucleic acid complexes of the present invention administered at 12.5 mg/kg, in which tocopherol or cholesterol is bound to the second nucleic acid strand (Toc#1HDO(mDMPK) and Chol#1HDO(mDMPK), respectively), on the expression of the target DMPK gene in the gastrocnemius muscle (GC), tibialis anterior muscle (TA), triceps brachii muscle (TB), quadriceps muscle (Quadriceps), diaphragm (Diaphragm), musculi dorsi proprii (Back), and heart muscle (Heart). In the Figure, ASO means ASO(mDMPK), which is a single-stranded nucleic acid molecule used as a positive control, and PBS means PBS used as a solvent, which is used as a negative control.

[Figure 6] Figure 6 shows inhibitory effects of double-stranded nucleic acid complexes of the present invention administered at 25 mg/kg in which tocopherol or cholesterol is bound to the second nucleic acid strand (Toc#1HDO(mDMPK), and Chol#1HDO(mDMPK), respectively), on the expression of the target DMPK gene in the gastrocnemius muscle (GC), tibialis anterior muscle (TA), triceps brachii muscle (TB), quadriceps muscle (Quadriceps), diaphragm (Diaphragm), musculi dorsi proprii (Back), and heart muscle (Heart). In the Figure, ASO means ASO(mDMPK), which is a single-stranded nucleic acid molecule used as a positive control, and PBS means PBS used as a solvent, which is used as a negative control.

[Figure 7] Figure 7 shows inhibitory effects of double-stranded nucleic acid complexes of the present invention administered at 50 mg/kg, in which tocopherol or cholesterol is bound to the second nucleic acid strand (Toc#1HDO(mDMPK) and Chol#1HDO(mDMPK), respectively), on the expression of the target DMPK gene in the gastrocnemius muscle (GC), tibialis anterior muscle (TA), triceps brachii muscle (TB), quadriceps muscle (Quadriceps), diaphragm (Diaphragm), musculi dorsi proprii (Back), and heart muscle (Heart). In the Figure, ASO means ASO(mDMPK), which is a single-stranded nucleic acid molecule used as a positive control, and PBS means PBS used as a solvent, which is used as a negative control.

[Figure 8] Figure 8 shows inhibitory effects of double-stranded nucleic acid complexes of the present invention, in which tocopherol or cholesterol is bound to the second nucleic acid strand (Toc#1DNA/DNA(mMalat1) and Chol#1DNA/DNA(mMalat1), respectively), on the expression of the target malat1 gene in the heart muscle (Heart), quadriceps muscle (Quadriceps), and diaphragm (Diaphragm). The second nucleic acid strand constituting the double-stranded nucleic acid complex also consists solely of DNA. In the Figure, ASO means ASO(mMalat1), which is a single-stranded nucleic acid molecule used as a positive control, and PBS means PBS used as a solvent, which is used as a negative control.

[Figure 9] Figure 9 shows inhibitory effects of double-stranded nucleic acid complexes of the present invention comprising Chol#1-cDNA(mMalat1) (PS) in which all of internucleoside linkages are composed of phosphorothioate linkages, or Chol#1-cDNA(mMalat1) (PO) in which all of internucleoside linkages are composed of phosphodiester linkages, both of which are bound to cholesterol and composed solely of DNA, as the second nucleic acid strand, on the expression of the target malat1 gene in the heart muscle (Heart), diaphragm (Diaphragm), and musculi dorsi proprii (Back). In the Figure, PBS means PBS used as a solvent, which is used as a negative control.

[Figure 10] Figure 10 shows inhibitory effects of double-stranded nucleic acid complexes of the present invention comprising Chol#1HDO(mMalat1) (PO), Chol#1HDO (5'PS), or Chol#1HDO (3'PS), which comprises phosphodiester linkages or phosphorothioate linkages between nucleosides in the second nucleic acid strand, to which cholesterol is bound at 5' end, on the expression of the target malat1 gene in the heart muscle (Heart), quadriceps muscle (Quadriceps), diaphragm (Diaphragm), and musculi dorsi proprii (Back). In the Figure, PBS means PBS used as a solvent, which is used as a negative control.

[Figure 11] Figure 11 shows inhibitory effects of double-stranded nucleic acid complexes of the present invention administered in multiple doses, in which tocopherol or cholesterol is bound to the second nucleic acid strand (Toc#1HDO(mMalat1) and Chol#1HDO(mMalat1), respectively), on the expression of the target Malat1 gene in the heart muscle (Heart), quadriceps muscle (Quadriceps), diaphragm (Diaphragm), and musculi dorsi proprii (Back). In the Figure, ASO means ASO(mMalat1), which is a single-stranded nucleic acid molecule used as a positive control, and PBS means PBS used as a solvent, which is used as a negative control.

[Figure 12] Figure 12 shows inhibitory effects of a double-stranded nucleic acid complex of the present invention, in which cholesterol is bound to the second nucleic acid strand (Chol#1HDO(mDMPK)), on the expression of the target DMPK gene in the heart muscle (Heart), quadriceps muscle (Quadriceps), diaphragm (Diaphragm), and musculi dorsi proprii (Back). In the Figure, PBS means PBS used as a solvent, which is used as a negative control.

[Figure 13] Figure 13 shows inhibitory effects of a double-stranded nucleic acid complex of the present invention, in which cholesterol is bound to the second nucleic acid strand (Chol#1HDO(mMalat1)), on the expression of the target gene (malat1) in (a) heart muscle (Heart), (b) musculi dorsi proprii (Back), (c) quadriceps muscle (QF), and (d) diaphragm (Dia). The vertical axis represents the relative expression level of malat1 non-coding RNA, and the horizontal axis represents time (days) after administration. In the Figure, PBS means PBS used as a solvent, which is used as a negative control.

[Figure 14] Figure 14 shows relative expression levels of the malat1 non-coding RNA in each tissue at 8 weeks (56 days) after administration. In the Figure, PBS means PBS used as a solvent, which is used as a negative control.

[Figure 15] Figure 15 shows inhibitory effects of a double-stranded nucleic acid complex of the present invention, i.e., Chol#1HDO(mMalat1), administered in a single dose in various doses, on the expression of the target malat1 gene in (a) quadriceps muscle (Quadriceps), (b) heart muscle (Heart), (c) musculi dorsi proprii (Back), and (d) diaphragm (Diaphragm).

[Figure 16] Figure 16 shows inhibitory effects of a double-stranded nucleic acid complex of the present invention, in which a linker consisting of a C6 hexyl group is bound between cholesterol bound to the second nucleic acid strand and the nucleic acid end, on expression of the target malat1 gene in the heart muscle (Heart), quadriceps muscle (Quadriceps), diaphragm (Diaphragm), and musculi dorsi proprii (Back). In the Figure, PBS means PBS used as a solvent, which is used as a negative control.

[Figure 17] Figure 17 shows inhibitory effects of double-stranded nucleic acid complex agents comprising a first nucleic acid strand having a different length of complementary strand with respect to the target gene, on the expression of the target gene (malat1) in the heart muscle (Heart), quadriceps muscle (Quadriceps), diaphragm (Diaphragm), and musculi dorsi proprii (Back). In the Figure, PBS means PBS used as a solvent, which is used as a negative control.

[Figure 18] Figure 18 shows inhibitory effects on the expression of the target gene (malat1) in (a) heart muscle (Heart), (b) quadriceps muscle (Quadriceps), and (c) diaphragm (Diaphragm), when a double-stranded nucleic acid complex agent of the present invention is subcutaneously administered.

[Figure 19] Figure 19 shows inhibitory effects of a single-stranded nucleic acid complex agent, to which cholesterol is bound at the end, a double-stranded nucleic acid complex agent, i.e., Chol#1HDO(mMalat1), of the present invention, and PBS as a negative control, on the expression of the malat1 gene in the heart muscle (Heart), quadriceps muscle (Quadriceps), diaphragm (Diaphragm), and musculi dorsi proprii (Back).

[Figure 20] Figure 20 shows platelet count in the mouse blood 72 hours after the administration of a single-stranded nucleic acid complex agent to which cholesterol is bound at the end (5'Chol-ASO-DNA and 3'Chol-ASO-DNA), a double-stranded nucleic acid complex agent of the present invention, i.e., Chol#1HDO(mMalat1), and PBS as a negative control.

[Figure 21] Figure 21 shows a representative result for electrophoresis with a Bioanalyzer 2100 (manufactured by Agilent Technologies) among PCR results. (a) shows PCR products from the heart (Heart), and (b) shows PCR products from the quadriceps muscle (Quadriceps). In the Figure, the arrowhead indicates a band in which exon 23 has not been skipped (Unskipped band), and the arrow indicates a band in which exon 23 has been skipped (Skipped band).

[Figure 22] Figure 22 shows the exon skipping efficiency of the dystrophin gene in mdx mice (Duchenne muscular dystrophy model mice) with a single-stranded nucleic acid complex agent (PMO), a double-stranded nucleic acid complex agent of the present invention to which tocopherol is bound at the end, i.e., Toc#1HDO(PMO), and PBS as a negative control respectively. (a) shows results for the heart muscle (Heart), (b) for the diaphragm (Diaphragm), (c) for the musculi dorsi proprii (Back), (d) for the quadriceps muscle (Quadriceps), (e) for the tibialis anterior muscle (Tibialis anterior), and (f) for the triceps brachii muscle (Triceps).

[Figure 23] Figure 23 shows Western blotting for the expression of the dystrophin protein. It shows dystrophin after administration of a single-stranded nucleic acid complex agent (PMO), a double-stranded nucleic acid complex agent of the present invention to which tocopherol is bound at the end, i.e., Toc#1HDO(Toc-HDO), and PBS as a negative control in (a) heart muscle (Heart), and (b) musculi dorsi proprii (Back) of mdx mice. B10 (normal mouse) shows dystrophin as a positive control.

[Figure 24] Figure 24 shows immunostaining for the expression of the dystrophin protein in the heart muscle and musculi dorsi proprii of mdx mice after administration of a single-stranded nucleic acid complex agent (PMO), and a double-stranded nucleic acid complex agent of the present invention to which tocopherol is bound at the end, i.e., Toc#1HDO(Toc-HDO).

[Figure 25] Figure 25 shows exon skipping in the dystrophin gene by a single-stranded nucleic acid complex agent (PMO), a double-stranded nucleic acid complex agent of the present invention to which tocopherol is bound at the end, i.e., Toc#1HDO(Toc-HDO) and Chol#1HDO(Chol-HDO), and PBS as a negative control in the heart muscle, diaphragm, quadriceps muscle, tibialis anterior muscle, and triceps brachii muscle of mdx mice.

[Figure 26] Figure 26 shows exon skipping in the dystrophin gene by a single-stranded nucleic acid complex agent (Mixmer), a double-stranded nucleic acid complex agent of the present invention to which tocopherol is bound at the end, i.e., Toc#1HDO(Toc-Mixmer), and PBS as a negative control in the heart muscle, quadriceps muscle, tibialis anterior muscle, triceps brachii muscle, and musculi dorsi proprii of mdx mice.

[Figure 27] Figure 27 shows inhibitory effects of the double-stranded nucleic acid complex of the present invention (Chol#1HDO(mMalat1)), and a nucleic acid molecule Chol-sHDO formed by self-annealing of a single-stranded nucleic acid in which Chol-HDO is bound by an RNA linker as shown in Figure 1c, on the expression of the target Malat1 gene in the heart muscle (Heart), quadriceps muscle (Quadriceps), diaphragm (Diaphragm), and musculi dorsi proprii (Back). PBS means PBS used as a solvent, which is used as a negative control.

[Figure 28] Figure 28 shows inhibitory effects of the double-stranded nucleic acid complex (Chol#1HDO(mMalat1)) of the present invention, in which cholesterol is bound to the second nucleic acid strand, and a nucleic acid (3'Chol(TEG)HDO(mMalat1)) having a structure in which the second nucleic acid strand is a strand having a sequence complementary to the first nucleic acid strand and cholesterol bound to the 3' end, and a linker (TEG) consisting of

tetraethylene glycol links the cholesterol and the end of the second nucleic acid strand, on the expression of the target Malat1 gene in the heart muscle (Heart), quadriceps muscle (Quadriceps), diaphragm (Diaphragm), and musculi dorsi proprii (Back). PBS means PBS used as a solvent, which is used as a negative control.

[Figure 29] Figure 29 shows running duration in an exercise tolerance test of normal mice (B 10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 30] Figure 30 shows measurements of (a) grip power and (b) holding impulse of normal mice (B10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 31] Figure 31 shows measurements of (a) creatine kinase (CK), (b) aspartate aminotransferase (AST), and (c) alanine aminotransferase (ALT) in the serum of normal mice (B 10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 32] Figure 32 shows corrected QT interval (QTc) in the measurement of electrocardiogram for normal mice (B10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 33] Figure 33 shows Western blotting for the expression of the dystrophin protein in the heart muscle. They show the expression of (a) dystrophin protein, and (b) vinculin protein in the heart muscle of normal mice (B10), and mdx mice administered with PBS only (mdx), a single-stranded nucleic acid complex agent (PMO), a double-stranded nucleic acid complex agent to which end tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 34] Figure 34 shows Western blotting for the expression of the dystrophin protein in the quadriceps muscle. It shows (a) dystrophin protein and (b) vinculin protein in the quadriceps muscle of normal mice (B10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO).

[Figure 35] Figure 35 shows immunostaining for the expression of the dystrophin protein in the heart muscle. It shows the expression of the dystrophin protein in the heart muscle of normal mice (B10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO). The scale bars indicate 200 μm.

[Figure 36] Figure 36 shows immunostaining for the expression of the dystrophin protein in the quadriceps muscle. It shows the expression of a dystrophin protein in the quadriceps muscle of normal mice (B10), and mdx mice administered with PBS only (mdx), a single-stranded nucleic acid complex agent (PMO), a double-stranded nucleic acid complex agent to which tocopherol is bound at the end, i.e., Toc#1HDO (Toc-HDO), or a double-stranded nucleic acid complex agent to which cholesterol is bound at the end, i.e., Chol#1HDO (Chol-HDO). The scale bars indicate 200 μm.

## Description of Embodiments

### 1. Double-stranded nucleic acid complex

#### 1-1. Overview

The first aspect of the present invention relates to a double-stranded nucleic acid complex. The double-stranded nucleic acid complex of the present invention can suppress or increase the expression level of the transcription product or translation product of a target gene in the skeletal muscle or heart muscle of a subject, inhibit the function of the transcription product or translation product of a target gene, or induce steric blocking, splicing switching, RNA editing, exon skipping, or exon inclusion by an antisense effect.

The double-stranded nucleic acid complex of the present invention comprises a first nucleic acid strand and a second nucleic acid strand that are annealed to each other. This first nucleic acid strand comprises a base sequence capable of hybridizing to all or part of the transcription product of the target gene, and has an antisense effect on the transcription product. The second nucleic acid strand comprises a base sequence complementary to the first nucleic

acid strand and has a functional moiety bound to the 5' end and/or 3' end.

1-2. Definitions of terms

A "target gene" means herein a gene, for which the expression level of the transcription product or translation product thereof can be suppressed or enhanced, the function of the transcription product or translation product can be inhibited, or steric blocking, splicing switching, RNA editing, exon skipping, or exon inclusion can be induced by the antisense effect of the double-stranded nucleic acid complex of the present invention. There is no particular restriction on the kind of a target gene, as long as it is expressed *in vivo.* Examples thereof include a gene which has been derived from an organism into which a double-stranded nucleic acid complex of the present invention is to be introduced, such as a gene whose expression is increased in various diseases. Specific examples thereof include a scavenger receptor B1 (herein often referred to as "SR-B1") gene, a metastasis-associated lung adenocarcinoma associated lung adenocarcinoma transcript 1 (herein often referred to as "Malat1") gene, a DMPK (dystrophia myotonica-protein kinase) gene, and a dystrophin gene.

Here, all of the scavenger receptors are receptor membrane proteins for denatured lipoproteins, and are known to be involved in cholesterol and lipoprotein metabolism. SR-B 1 is a double-pass transmembrane protein belonging to the evolutionarily conserved CD36 family, and has a long extracellular domain and two short intracellular domains comprising the amino terminal and the carboxyl terminal respectively.

It is known that Malat1 is a long non-coding RNA (IncRNA) which is highly expressed in malignant tumors such as lung cancer, and is localized in the nucleus of myocytes.

The DMPK gene encodes a myotonin-protein kinase, and is known to be a causative gene for myotonic dystrophy, which is the most frequent type of muscular dystrophy in adults. It is believed that abnormal elongation of the CTG repeat sequence existing in the 3' untranslated region of the DMPK gene is the cause of the disease.

A "target transcription product" means herein any RNA that is synthesized by an RNA polymerase and is a direct target of the nucleic acid complex of the present invention. In general, it is a "transcription product of a target gene". Specifically, mRNA transcribed from a target gene (comprising mature mRNA, mRNA precursor, mRNA without base modification, and so on), non-coding RNA (ncRNA) such as miRNA, long non-coding RNA (lncRNA), and natural antisense RNA can be included. Examples of a transcription product of a target gene may comprise SR-B1 mRNA which is a transcription product of the SR-B1 gene, Malat1 non-coding RNA which is a transcription product of the Malat1 gene, and DMPK mRNA which is a transcription product of the DMPK gene.

As specific examples, the base sequence of a murine SR-B1 mRNA is shown in SEQ ID NO: 1, and the base sequence of a human SR-B1 mRNA is shown in SEQ ID NO: 2. Further, the base sequence of a murine malat1 non-coding RNA is shown in SEQ ID NO: 3, and a human malat1 non-coding RNA is shown in SEQ ID NO: 4. Furthermore, the base sequence of a murine DMPK mRNA is shown in SEQ ID NO: 5, and the base sequence of a human DMPK mRNA is shown in SEQ ID NO: 6. In this regard, in all of SEQ ID NOs: 1 to 6, the base sequences of mRNA are replaced with the base sequences of DNA. The base sequence information for these genes and transcription products can be obtained from publicly known databases, such as the database of NCBI (The U.S. National Center for Biotechnology Information).

The base sequence of a publicly known antisense medicine may be also utilized. For example, the base sequence shown in SEQ ID NO: 24 constituting ISIS 598769 (IONIS) which is a therapeutic drug for myotonic dystrophy and related to its causative gene, namely DMPK gene, the base sequence shown in SEQ ID NO: 25 constituting Eteplirsen (Exondys 51, Sarepta Therapeutics), which is known as a therapeutic drug for Duchenne muscular dystrophy and induces exon skipping in pre-mRNA of the dystrophin gene, the base sequence shown in SEQ ID NO: 26 constituting Golodirsen (Sarepta Therapeutics), the base sequence shown in SEQ ID NO: 27 constituting NS-065/NCNP-01 (Nippon Shinyaku Co., Ltd.), or the base sequence shown in SEQ ID NO: 28 constituting Casimersen (Sarepta Therapeutics) may be used.

An "antisense oligonucleotide (ASO)" means herein a single-stranded oligonucleotide that comprises a complementary base sequence capable of hybridizing to all or part, such as any target region, of a target transcription product, and can regulate and suppress the expression of a transcription product of the target gene or the level of the target transcription product by an antisense effect. In the double-stranded nucleic acid complex of the present invention, the first nucleic acid strand functions as ASO, and its target region may comprise 3'UTR, 5'UTR, exon, intron, coding region, translation initiation region, translation termination region, or any other nucleic acid region. The target region of a target transcription product may be at least 8 base in length, for example, 10 to 35 base in length, 12 to 25 base in length, 13 to 20 base in length, 14 to 19 base in length, or 15 to 18 base in length.

An "antisense effect" means an effect of regulating expression or editing of a target transcription product by hybridization of ASO to the target transcription product (*e.g.* RNA sense strand). The phrase "regulating expression or editing of a target transcription product" means suppression or reduction of the expression of a target gene or the expression amount of a target transcription product ("expression amount of a target transcription product" is herein often referred to as "expression level of a target transcription product"), inhibition of translation, RNA editing, splicing function

modification effect *(e.g.,* splicing switching, exon inclusion, and exon skipping), or degradation of a transcription product. For example, in the case of post-transcriptional inhibition of a target gene, when an RNA oligonucleotide is introduced into a cell as ASO, the ASO forms a partial double strand by annealing to mRNA which is a transcription product of a target gene. This partial double strand serves as a cover to prevent translation by ribosomes, so as to inhibit the expression of the target protein encoded by the target gene at the translation level (steric blocking). On the other hand, when an oligonucleotide comprising DNA is introduced into a cell as ASO, a partial DNA-RNAheteroduplex is formed. This hetero double-strand structure is recognized by RNase H, and as a result mRNA of the target gene is degraded and the expression of the protein encoded by the target gene is inhibited at the expression level. In addition, an antisense effect can also be produced for an intron in an mRNA precursor as a target. Further, an antisense effect can also be produced for miRNA as a target. In this case, as a result of functional inhibition of the miRNA, the expression of the gene whose expression is normally regulated by the miRNA may be increased. In an embodiment, expression regulation of a target transcription product may be decrease of the amount of a target transcription product.

A "translation product of the target gene" means herein any polypeptide or protein that is a direct target of the nucleic acid complex of the present invention and is synthesized by translation of the target transcription product or a transcription product of the target gene. Examples of a translation product of the target gene comprise a SR-B1 protein, which is a translation product of the SR-B1 gene, a Malat1 protein, which is a translation product of the Malat1 gene, and a DMPK protein, which is a translation product of the DMPK gene.

The term "nucleic acid" or "nucleic acid molecule" used herein means a nucleoside or a nucleotide in the case of a monomer, an oligonucleotide in the case of an oligomer, and a polynucleotide in the case of a polymer.

A "nucleoside" generally means a molecule consisting of a combination of a base and a sugar. The sugar moiety of a nucleoside is usually, but not limited to, composed of pentofuranosyl sugar, and specific examples thereof include ribose and deoxyribose. The base moiety of nucleoside (nucleobase) is usually a heterocyclic base moiety. Without limitation, examples thereof include adenine, cytosine, guanine, thymine, and uracil as well as other modified nucleobases (modified bases).

A "nucleotide" refers to a molecule in which a phosphate group is covalently bonded to the sugar moiety of a nucleoside. In the case of a nucleotide comprising pentofuranosyl sugar, a phosphate group is usually linked to a hydroxyl group at the 2', 3', or 5' position of the sugar.

An "oligonucleotide" refers to a linear oligomer formed by linking several to dozens of neighboring nucleotides through a covalent bond between a hydroxyl group and a phosphate group in the sugar moiety. Meanwhile, a "polynucleotide" refers to a linear polymer formed by linking with covalent bonds dozens or more, preferably hundreds or more of nucleotides, namely more nucleotides than in an oligonucleotide. It is considered that the phosphate group generally forms an internucleoside linkage inside the structure of an oligonucleotide or a polynucleotide.

A "nucleic acid strand" or simply "strand" herein means an oligonucleotide or a polynucleotide. A full length strand or a partial length strand of a nucleic acid strand can be produced by a chemical synthesis using an automated synthesizer, or by an enzymatic process using a polymerase, a ligase, or a restricted reaction. A nucleic acid strand may comprise a natural nucleotide and/or a non-natural nucleotide.

A "natural nucleoside" refers herein to a nucleoside that exists in nature. Examples thereof include a ribonucleoside consisting of a ribose and the aforementioned base such as adenine, cytosine, guanine, or uracil, or a deoxyribonucleoside consisting of a deoxyribose and the aforementioned base such as adenine, cytosine, guanine, or thymine. In this regard, a ribonucleoside found in RNA, and a deoxyribonucleoside found in DNA are herein often referred to as "DNA nucleoside" and "RNA nucleoside", respectively.

A "natural nucleotide" means herein a nucleotide that exists in nature, namely a molecule in which a phosphate group is covalently bound to the sugar moiety of the aforementioned natural nucleoside. Examples thereof include a ribonucleotide which is known as a constituent of RNA, and in which a phosphate group is bound to a ribonucleoside, and a deoxyribonucleotide, which is known as a constituent of DNA, and in which a phosphate group is bound to a deoxyribonucleoside.

A "non-natural nucleoside" means herein any nucleoside other than a natural nucleoside. For example, it comprises a modified nucleoside and a nucleoside mimic. A "modified nucleoside" means herein a nucleoside having a modified sugar moiety and/or a modified nucleobase. A nucleic acid strand comprising a non-natural oligonucleotide is in many cases more preferable than a natural type owing to desirable properties, such as enhanced cellular uptake, enhanced affinity for a target nucleic acid, increased stability in the presence of a nuclease, and increase in inhibitory activity.

A "mimic" refers herein to a functional group that replaces a sugar, a nucleobase, and/or an internucleoside linkage. In general, a mimic is used in place of a sugar or a combination of a sugar-internucleoside linkage, and a nucleobase is maintained for hybridization to a target to be selected. The term "nucleoside mimic" used herein comprises a structure to be used for replacing a sugar at one or more sites of an oligomer compound, or replacing a sugar and a base, or replacing a bond between monomer subunits constituting an oligomer compound. An "oligomer compound" means a polymer composed of linked monomer subunits that can at least hybridize to a region of a nucleic acid molecule.

Examples of a nucleoside mimic comprise a morpholino, cyclohexenyl, cyclohexyl, tetrahydropyranyl, bicyclic or tricyclic sugar mimic, such as a nucleoside mimic having a non-furanose sugar unit.

A "bicyclic nucleoside" herein means a modified nucleoside comprising a bicyclic sugar moiety. A nucleic acid comprising a bicyclic sugar moiety is commonly referred to as bridged nucleic acid (BNA). A nucleoside comprising a bicyclic sugar moiety is herein sometimes referred to as "bridged nucleoside." Some examples of a bridged nucleic acid are shown in Figure 2.

A bicyclic sugar may be a sugar in which the carbon atom at the 2' position and the carbon atom at the 4' position are bridged with two or more atoms. Examples of a bicyclic sugar are known to those skilled in the art. A subgroup of nucleic acids comprising a bicyclic sugar (BNA) may be so described that they have a carbon atom at the 2' position and a carbon atom at the 4' position which are bridged with 4'-$(CH_2)_p$-O-2', 4'-$(CH_2)_p$-$CH_2$-2', 4'-$(CH_2)_p$-S-2', 4'-$(CH_2)_p$-O $CH_2$O-2', 4'-$(CH_2)_n$-N($R_3$)-O-$(CH_2)_m$-2' [wherein p, m, and n represent integers from 1 to 4, from 0 to 2, and from 1 to 3, respectively; and $R_3$ represents a hydrogen atom, an alkyl group, an alkenyl group, a cycloalkyl group, an aryl group, an aralkyl group, an acyl group, a sulfonyl group, and a unit substituent (*e.g.*, a fluorescently or chemiluminescently labeled molecule, a functional group with nucleic acid cleavage activity, and an intracellular or intranuclear localization signal peptide)]. Furthermore, with respect to a BNA in a certain embodiment, in the $OR_2$ substituent of the carbon atom at the 3' position and the $OR_1$ substituent of the carbon atom at the 5' position, $R_1$ and $R_2$ are typically hydrogen atoms, but may be the same or different from each other, or may also be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or P($R_4$)$R_5$ [wherein $R_4$ and $R_5$, may be the same or different from each other, and respectively represent a hydroxyl group, a hydroxyl group protected by a protecting group for nucleic acid synthesis, a mercapto group, a mercapto group protected by a protecting group for nucleic acid synthesis, an amino group, a C1-C5 alkoxy group, a C1-C5 alkylthio group, a C1-C6 cyanoalkoxy group, or an amino group substituted with a C1-C5 alkyl group]. Non-limiting examples of such BNA comprise methyleneoxy(4'-$CH_2$-O-2') BNA(LNA, Locked Nucleic Acid®, also known as 2',4'-BNA), e.g., α-L-methyleneoxy(4'-$CH_2$O-2') BNA or β-D-methyleneoxy(4'-$CH_2$-O-2') BNA, ethyleneoxy(4'-$(CH_2)_2$-O-2') BNA (also known as ENA), β-D-thio(4'-$CH_2$-S-2') BNA, aminooxy(4'-$CH_2$-O-N($R_3$)-2') BNA, oxyamino(4'-$CH_2$-N($R_3$)-O-2') BNA (also known as 2',4'-$BNA^{NC}$), 2',4'-$BNA^{coc}$, 3'-amino-2',4'-BNA, 5'-methyl BNA, (4'-CH(CH3)-O-2') BNA (also known as cEt BNA), (4'-CH($CH_2OCH_3$)-O-2') BNA (also known as cMOE BNA), amido BNA (4'-C(O)-N(R)-2') BNA (R=H, or Me) (also known as AmNA), 2'-O,4'-C-spirocyclopropylene-bridged nucleic acid (also known as scpBNA), and other BNA known to those skilled in the art. A bicyclic nucleoside having a methyleneoxy(4'-$CH_2$-O-2') bridge is herein often referred to as "LNA nucleoside".

A "non-natural nucleotide" means herein any nucleotide other than a natural nucleotide. For example, it comprises a modified nucleotide and a nucleotide mimic. A "modified nucleotide" means herein a nucleotide having at least one of a modified sugar moiety, a modified internucleoside linkage, and a modified nucleobase. The term "nucleotide mimic" herein comprises a structure used to substitute a nucleoside and a bond (linkage) at one or more positions in an oligomer compound. Examples of the nucleotide mimic comprise a peptide nucleic acid, and a morpholino nucleic acid (morpholino linked with -N(H)-C(=O)-O- or another non-phosphodiester linkage). The peptide nucleic acid (PNA) is a nucleotide mimic having a main chain in which *N*-(2-aminoethyl)glycine in place of a sugar is linked with an amide linkage. A nucleic acid strand comprising a non-natural oligonucleotide herein has in many cases preferable properties, such as enhanced cellular uptake, enhanced affinity for a target nucleic acid, increased stability in the presence of a nuclease, and increase in inhibitory activity. Therefore, it is more preferable than a natural nucleotide.

A "modified internucleoside linkage" means herein an internucleoside linkage that has a substitution or any change from a naturally occurring internucleoside linkage (*i.e.,* phosphodiester linkage). The modified internucleoside linkage comprises, but not limited to, a phosphorus-containing internucleoside linkage that comprises a phosphorus atom, and a phosphorus-free internucleoside linkage that does not comprise a phosphorus atom. Typical phosphorus-containing internucleoside linkages comprise, but not limited to, a phosphodiester linkage, a phosphorothioate linkage, a phosphorodithioate linkage, a phosphotriester linkage, an alkylphosphonate linkage, an alkylthiophosphonate linkage, a boranophosphate linkage, and a phosphoroamidate linkage. The phosphorothioate linkage is an internucleoside linkage in which an unbridged oxygen atom in a phosphodiester linkage is substituted with a sulfur atom. A method for preparing a phosphorus-containing and phosphorus-free linkage is well known. It is preferable that a modified internucleoside linkage is a linkage having a higher resistance to a nuclease than a naturally occurring internucleoside linkage.

A "modified nucleobase" or a "modified base" means herein any nucleobase other than adenine, cytosine, guanine, thymine, and uracil. Examples of a modified nucleobase comprise, but not limited to, 5-methylcytosine, 5-fluorocytosine, 5-bromocytosine, 5-iodocytosine, N4-methylcytosine, N6-methyladenine, 8-bromoadenine, N2-methylguanine, or 8-bromoguanine. A preferred modified nucleobase is 5-methylcytosine.

The term "unmodified nucleobase" or "unmodified base" is synonymous with a natural nucleobase, and means adenine (A) and guanine (G), which are purine bases, and thymine (T), cytosine (C), and uracil (U), which are pyrimidine bases.

A "modified sugar" refers herein to a sugar in which a natural sugar moiety (*i.e.,* sugar moiety found in DNA(2'-H) or RNA(2'-OH)) has undergone a substitution and/or any change. A nucleic acid strand herein may comprise in some cases one or more modified nucleosides comprising a modified sugar. A sugar-modified nucleoside can confer a beneficial biological property such as enhanced nuclease stability, increased binding affinity, or the like to a nucleic acid strand. A nucleoside may comprise a chemically modified ribofuranose ring moiety. Examples of a chemically modified ribofuranose ring comprise, but not limited to, addition of a substituent (comprising 5' and 2' substituents), formation of a bicyclic nucleic acid (bridged nucleic acid, BNA) through bridge formation of a non-geminal ring atom, substitution of a ribosyl ring oxygen atom with S, N(R), or C(R1)(R2) (wherein R, R1, and R2 independently represent H, a $C_1$-$C_{12}$ alkyl, or a protecting group), and a combination thereof. Examples of a nucleoside having a modified sugar moiety herein comprise, but not limited to, a nucleoside comprising a substituent such as 5'-vinyl, 5'-methyl (R or S), 4'-S, 2'-F (2'-fluoro group), 2'-$OCH_3$ (2'-OMe group or 2'-O-methyl), and 2'-$O(CH_2)_2OCH_3$. A substituent at the 2' position may be selected from allyl, amino, azide, thio, -O-allyl, -O-$C_1$-$C_{10}$ alkyl, -$OCF_3$, -$O(CH_2)_2SCH_3$, -$O(CH_2)_2$-O-N(Rm)(Rn), and O-$CH_2$-C(=O)-N(Rm)(Rn), wherein Rm and Rn are independently H or a substituted or unsubstituted $C_1$-$C_{10}$ alkyl. A "2'-modified sugar" means herein a furanosyl sugar modified at the 2' position.

In general, a modification can be performed such that nucleotides in the same strand can independently undergo different modifications. In addition, to provide resistance to enzymatic cleavage, the same nucleotide can have a modified internucleoside linkage (*e.g.,* phosphorothioate linkage) and also a modified sugar *(e.g.,* a 2'-O-methyl modified sugar or a bicyclic sugar). The same nucleotide can also have a modified nucleobase *(e.g.,* 5-methylcytosine) and can also have a modified sugar *(e.g.,* a 2'-O-methyl modified sugar, or a bicyclic sugar).

The number, kind, and position of a non-natural nucleotide in a nucleic acid strand may influence the antisense effect and the like provided by the nucleic acid complex of the present invention. The choice of a modification may vary depending on the sequence of a target gene or the like, but those skilled in the art can determine a suitable embodiment by referring to the descriptions in the literature related to the antisense method *(e.g.,* WO 2007/143315, WO 2008/043753, and WO 2008/049085). Furthermore, when the antisense effect of a nucleic acid complex after the modification is measured and the obtained measured value is not significantly lower than the measured value of the nucleic acid complex before the modification (*e.g.*, when the measured value obtained after the modification is 70% or more, 80% or more, or 90% or more of the measured value of the nucleic acid complex before the modification), a relevant modification may be evaluated.

The term "complementary" as used herein refers to the relationship that nucleobases can form via hydrogen bonds so-called Watson-Crick base pairs (natural base pairs) or non-Watson-Crick base pairs (Hoogsteen base pairs, etc.). The first nucleic acid strand is not necessarily required to be completely complementary to all or part of a target transcription product *(e.g.,* the transcription product of a target gene), and it is acceptable if the base sequence has at least 70%, preferably at least 80%, and further preferably at least 90% *(e.g.,* 95%, 96%, 97%, 98%, or 99% or more) in the complementarity. Similarly, the complementary region in the second nucleic acid strand is not necessarily required to be completely complementary to all or part of the first nucleic acid strand, and it is acceptable if the base sequence has a complementarity of at least 70%, preferably at least 80%, and further preferably at least 90% *(e.g.,* 95%, 96%, 97%, 98%, or 99% or more).

In the present invention, a "muscle disease" refers to a disease that causes atrophy of muscles leading to muscular weakness. Example thereof include muscular dystrophy, myopathy, inflammatory myopathy (including polymyositis, and dermatomyositis), Danon disease, myasthenic syndrome, mitochondrial disease, myoglobinuria, glycogen storage disease, and periodic paralysis. In the present invention, a suitable muscle disease is muscular dystrophy. With respect to muscular dystrophy various disease types are known comprising Duchenne type, Becker type, Emery-Dreyfus type, limb-girdle type, facioscapulohumeral type, and oculopharyngeal type, and the muscular dystrophy herein may be any of these disease types. Similarly, with respect to myopathy various types are known comprising congenital, distal, hypothyroid, and steroidal myopathy types and the myopathy herein may be any of these disease types.

A "functional moiety" is herein a moiety that binds to a double-stranded nucleic acid complex to enable efficient delivery of the double-stranded nucleic acid complex to the skeletal muscle, heart muscle, etc. There is no particular restriction on the functional moiety, and it may be a lipid ligand, a lipid derivative ligand, a peptide ligand, an antibody ligand, an aptamer, a small molecule ligand, a ligand molecule to be incorporated into the heart muscle or skeletal muscle, or the like. For example, a functional moiety may be cholesterol or analog thereof, tocopherol or analog thereof, phosphatidyl ethanolamine or analog thereof, a substituted or unsubstituted C1-C30 alkyl group, a substituted or unsubstituted C2-C30 alkenyl group, or a substituted or unsubstituted C1-C30 alkoxy group.

"Tocopherol" is herein a methylated derivative of tocorol, and a liposoluble vitamin (vitamin E) having a cyclic structure called chroman. Tocopherol has a strong antioxidant effect, and therefore functions *in vivo* as an antioxidant substance to scavenge free radicals produced by metabolism and protect cells from damage.

A plurality of different types of tocopherol are known based on the position of the methyl group bound to chroman comprising $\alpha$-tocopherol, $\beta$-tocopherol, $\gamma$-tocopherol, and $\delta$-tocopherol. The tocopherol herein may be any types of tocopherol. In addition, examples of the analog of tocopherol comprise various unsaturated analogs of tocopherol, such

as α-tocotrienol, β-tocotrienol, γ-tocotrienol, and δ-tocotrienol. Preferably, tocopherol is α-tocopherol.

"Cholesterol" is herein a kind of sterol, also called steroid alcohol, which is especially abundant in animals. Cholesterol exerts an important function in the metabolic process *in vivo,* and in animal cells, it is also a major constituent of the membrane system of a cell, together with phospholipid. In addition, the cholesterol analog refers to various cholesterol metabolism products and their analogs, which are alcohols with a sterol backbone. Examples thereof include, but not limited to, cholestanol, lanosterol, cerebrosterol, dehydrocholesterol, and coprostanol.

An "analog" herein refers to a compound having a similar structure and property having the same or a similar basic backbone. The analog comprises, for example, a biosynthetic intermediate, a metabolism product, and a compound having a substituent. Those skilled in the art can determine whether or not a compound is an analog of another compound based on common general technical knowledge.

A "subject" herein refers to the object to which the double-stranded nucleic acid complex or pharmaceutical composition of the present invention is applied. A subject comprises an individual as well as an organ, a tissue, and a cell. When the subject is an individual, any animal including a human may be applicable. For example, in addition to a human, a variety of domestic animals, domestic fowls, pets, and laboratory animals are included. Without limitation, a subject may be an individual who needs reduction of the expression level of a target transcription product in the skeletal muscle or heart muscle, or an individual who needs a treatment for or prevention from a muscle disease.

1-3. Configuration

The double-stranded nucleic acid complex of the present invention comprises a first nucleic acid strand and a second nucleic acid strand. The specific configuration of each nucleic acid strand is described below.

The first nucleic acid strand is a single-stranded oligonucleotide strand that comprises a base sequence capable of hybridizing to all or part of the transcription product of a target gene and produces an antisense effect on the target transcription product.

The second nucleic acid strand is a single-stranded oligonucleotide strand that comprises a base sequence complementary to the first nucleic acid strand. The second nucleic acid strand is bound to cholesterol or analog thereof. Also, in a double-stranded nucleic acid complex, the second nucleic acid strand is annealed to the first nucleic acid strand via hydrogen bonds of complementary base pairs.

There is no particular restriction on the base lengths of the first nucleic acid strand and the second nucleic acid strand, and may be at least 8 base in length, at least 9 base in length, at least 10 base in length, at least 11 base in length, at least 12 base in length, at least 13 base in length, at least 14 base in length, or at least 15 base in length. Further, the base length of the first nucleic acid strand and the second nucleic acid strand may be 35 base in length or less, 30 base in length or less, 25 base in length or less, 24 base in length or less, 23 base in length or less, 22 base in length or less, 21 base in length or less, 20 base in length or less, 19 base in length or less, 18 base in length or less, 17 base in length or less, or 16 base in length or less. The first nucleic acid strand and the second nucleic acid strand may have the same length or different lengths (for example, one of them is shorter or longer by 1 to 3 bases). The double-stranded structure formed by the first nucleic acid strand and the second nucleic acid strand may comprise a bulge. The length can be determined according to the balance between the strength of the antisense effect and the specificity of the nucleic acid strand to the target, among other factors such as cost, and synthesis yield.

The internucleoside linkage in the first nucleic acid strand and second nucleic acid strand may be a naturally occurring internucleoside linkage and/or a modified internucleoside linkage. Without limitations, at least one, at least two, or at least three internucleoside linkages from an end (5' end, 3' end, or both ends) of the first nucleic acid strand and/or the second nucleic acid strand are preferably modified internucleoside linkages. In this regard, for example, two internucleoside linkages from an end of a nucleic acid strand means the internucleoside linkage closest to the end of the nucleic acid and the internucleoside linkage positioned next thereto and on the opposite side of the end. The modified internucleoside linkage in the terminal region of a nucleic acid strand is preferred because it can reduce or inhibit undesired degradation of the nucleic acid strand. In an embodiment, all the internucleoside linkages of the first nucleic acid strand and/or second nucleic acid strand may be modified internucleoside linkages. The modified internucleoside linkage may be a phosphorothioate linkage.

At least one *(e.g.,* three) internucleoside linkage from the 3' end of the second nucleic acid strand may be a modified internucleoside linkage such as a phosphorothioate linkage with a high resistance to an RNase. It is preferable that the second nucleic acid strand comprises a modified internucleoside linkage such as a phosphorothioate modification at the 3' end, because the gene suppression activity of the double-stranded nucleic acid complex is enhanced.

At the 5' end and 3' end of the second nucleic acid strand, internucleoside linkages for 2 to 6 bases from the end, to which cholesterol or analog thereof is not bound, may be modified internucleoside linkages (*e.g.*, phosphorothioate linkages).

At least one *(e.g.,* three) nucleoside from the 3' end of the second nucleic acid strand may be a modified nucleoside, such as 2'F-RNA, 2'-OMe, or the like having a high resistance to an RNase. It is preferable that the second

nucleic acid strand comprises a modified nucleoside such as 2'F-RNA, or 2'-OMe at the 3' end, because the gene suppression activity of the double-stranded nucleic acid complex is enhanced.

At the 5' end and 3' end of the second nucleic acid strand, 1 to 5 nucleosides from the end, to which cholesterol or analog thereof is not bound, may be modified nucleosides, such as 2'F-RNA or the like having a high resistance to an RNase.

A nucleoside in the first nucleic acid strand and the second nucleic acid strand may be a natural nucleoside (deoxyribonucleoside, ribonucleoside, or both) and/or a non-natural nucleoside.

The base sequence of the first nucleic acid strand herein is complementary to all or part of the base sequence of a target transcription product, and therefore can hybridize (or anneal) to the target transcription product. The complementarity of a base sequence can be determined by using a BLAST program or the like. Those skilled in the art can easily determine the conditions (temperature, salt concentration, and the like) under which two strands can be hybridized, taking into consideration the complementarity between the strands. In addition, those skilled in the art can easily design an antisense nucleic acid that is complementary to a target transcription product, for example, for example, based on the information on the base sequence of the target gene.

Hybridization conditions may be a variety of stringent conditions, for example, low-stringent conditions or high stringent conditions. The low-stringent conditions may be relatively low temperature and high salt concentration, for example, 30°C, 2 × SSC, and 0.1% SDS. The high stringent conditions may be relatively high temperature and low salt concentration, for example, 65°C, 0.1 × SSC, and 0.1% SDS. By changing the conditions such as temperature and salt concentration, the stringency of hybridization can be adjusted. Here, 1 × SSC contains 150 mM sodium chloride and 15 mM sodium citrate.

The first nucleic acid strand may comprise at least four, at least five, at least six, or at least seven consecutive nucleosides that are recognized by RNase H when hybridized to a target transcription product. Typically, it may be a region comprising from 4 to 20 bases, from 5 to 16 bases, or from 6 to 12 bases of consecutive nucleosides. As the nucleoside that is recognized by RNase H, for example, a natural deoxyribonucleoside may be used. A modified deoxyribonucleoside, and a suitable nucleoside comprising other bases, are well known in the art. It is also known that a nucleoside having a hydroxy group at the 2' position, such as a ribonucleoside, is not suitable as the above nucleoside. The suitability of a nucleoside for use in the region comprising "at least four consecutive nucleosides" can be readily determined. In an embodiment, the first nucleic acid strand may comprise at least four consecutive deoxyribonucleosides.

In an embodiment, the full length of the first nucleic acid strand is not solely composed of natural ribonucleosides. It is preferable that natural ribonucleosides are contained in not more than half of the full length, or are not contained, in the first nucleic acid strand.

In an embodiment, the second nucleic acid strand may comprise at least four consecutive ribonucleosides that are complementary to the above at least four consecutive nucleosides (*e.g.*, deoxyribonucleosides) in the first nucleic acid strand. This is for the second nucleic acid strand to form a partial DNA-RNA heteroduplex with the first nucleic acid strand so as to be recognized and cleaved by RNaseH. The at least four consecutive ribonucleosides in the second nucleic acid strand are preferably linked by naturally occurring internucleoside linkages, namely phosphodiester linkages.

In the second nucleic acid strand, all the nucleosides may be composed of ribonucleosides and/or modified nucleosides. All the nucleosides in the second nucleic acid strand may be composed deoxyribonucleosides and/or modified nucleosides, or may comprise no ribonucleoside. In an embodiment, all the nucleosides in the second nucleic acid strand may be composed of deoxyribonucleosides and/or modified nucleosides.

The first nucleic acid strand and/or the second nucleic acid strand constituting the double-stranded nucleic acid complex of the present invention may be a gapmer. A "gapmer" herein means a single-stranded nucleic acid consisting, in principle, of a central region (DNA gap region) and wing regions positioned directly at the 5' end and 3' end thereof (respectively referred to as 5' wing region and 3' wing region). The central region in a gapmer comprises at least four consecutive deoxyribonucleosides, and the wing region comprises at least one non-natural nucleoside. Without limitation, the non-natural nucleoside comprised in the wing region usually has a higher binding strength to RNA, and a higher resistance to a nucleolytic enzyme (such as a nuclease) than a natural nucleoside. When a non-natural nucleoside constituting the wing region comprises a bridged nucleoside, or consists of the same, the gapmer is specifically referred to as a "BNA/DNA gapmer". The number of bridged nucleosides comprised in the 5' wing region and the 3' wing region is at least one, and may be, for example, two or three. The bridged nucleosides comprised in the 5' wing region and the 3' wing region may be present consecutively or nonconsecutively in the 5' wing region and the 3' wing region. The bridged nucleoside may further comprise a modified nucleobase (*e.g.*, 5-methylcytosine). When the bridged nucleoside is an LNA nucleoside, the gapmer is referred to as an "LNA/DNA gapmer". When a non-natural nucleoside constituting the 5' wing region and the 3' wing region comprises or consists of a peptide nucleic acid, such a gapmer is specifically referred to as a "peptide nucleic acid gapmer". When a non-natural nucleoside constituting the 5' wing region and the 3' wing region comprises or consists of a morpholino nucleic acid, such a gapmer is specifically referred to as a "morpholino nucleic acid gapmer". The base lengths of the 5' wing region and the 3' wing region may be independently at least 2 base in length, for example from 2 to 10 base in length, from 2 to 7 base in length, or from 3 to 5 base in length. It is

acceptable if the 5' wing region and the 3' wing region comprise at least one kind of non-natural nucleoside, and the 5' wing region and the 3' wing region may further comprise a natural nucleoside.

The first nucleic acid strand and/or the second nucleic acid strand constituting the above gapmer may be constituted in the order from the 5' end by bridged nucleosides of from 2 to 7 base in length or 3 to 5 base in length, ribonucleosides or deoxyribonucleosides of from 4 to 15 base in length or from 8 to 12 base in length, and bridged nucleosides of from 2 to 7 base in length or from 3 to 5 base in length.

In this regard, a nucleic acid strand having a wing region only on either side of the 5' end and the 3' end is called a "hemi-gapmer" in the art. However, herein a hemi-gapmer is also encompassed in a gapmer.

The first nucleic acid strand and/or the second nucleic acid strand constituting the double-stranded nucleic acid complex of the present invention may be a mixmer. The term "mixmer" refers to herein a nucleic acid strand that comprises natural nucleosides and non-natural nucleosides with periodically or randomly alternating segment lengths, and does not comprise four or more consecutive deoxyribonucleosides or ribonucleosides. Among mixmers, a mixmer in which the non-natural nucleoside is a bridged nucleoside, and the natural nucleoside is a deoxyribonucleoside is specifically referred to as a "BNA/DNA mixmer". Among mixmers, a mixmer in which the non-natural nucleoside is a peptide nucleic acid and the natural nucleoside is a deoxyribonucleoside is specifically called a "peptide nucleic acid/DNA mixmer". Among mixmers, a mixmer in which the non-natural nucleoside is a morpholino nucleic acid, and the natural nucleoside is a deoxyribonucleoside is specifically referred to as a "morpholino nucleic acid/DNA mixmer". A mixmer is not restricted to comprise only two kinds of nucleosides. A mixmer may comprise any number of kinds of nucleosides irrespective of a natural or modified nucleoside, or a nucleoside mimic. For example, a mixmer may comprise one or two consecutive deoxyribonucleosides separated by a bridged nucleoside (*e.g.*, LNA nucleoside). A bridged nucleoside may further comprise a modified nucleobase *(e.g.,* 5-methylcytosine).

At least one, at least two, at least three, or at least four nucleosides from the end (5' end, 3' end, or both ends) of the second nucleic acid strand may be modified nucleosides. The modified nucleoside may comprise a modified sugar and/or a modified nucleobase. The modified sugar may be a 2'-modified sugar *(e.g.,* sugar comprising a 2'-O-methyl group). The modified nucleobase can also be 5-methyl cytosine.

The second nucleic acid strand may be constituted in the order from the 5' end by modified nucleosides (*e.g.*, modified nucleosides comprising a 2'-modified sugar) of from 2 to 7 base in length or 3 to 5 base in length, ribonucleosides or deoxyribonucleosides of from 4 to 15 base in length or from 8 to 12 base in length (optionally linked by modified internucleoside linkages), and modified nucleosides *(e.g.,* a modified nucleosides comprising 2'-modified sugar) of from 2 to 7 base in length, or 3 to 5 base in length. In this case, the first nucleic acid strand may be a gapmer.

The first nucleic acid strand and the second nucleic acid strand may comprise, as a whole or in part, a nucleoside mimic or a nucleotide mimic. The nucleotide mimic may be a peptide nucleic acid and/or a morpholino nucleic acid. The first nucleic acid strand may also comprise at least one modified nucleoside. The modified nucleoside may comprise a 2'-modified sugar. This 2'-modified sugar may be a sugar comprising a 2'-O-methyl group. Therefore, an embodiment of the present invention relates to a double-stranded nucleic acid complex in which the first nucleic acid strand comprises a base sequence that can hybridize to all or part of the transcription product of a target gene, and has an antisense effect on the transcription product, the second nucleic acid strand comprises a base sequence that is complementary to the first nucleic acid strand, the first nucleic acid strand is annealed to the second nucleic acid strand, and the first nucleic acid strand comprises a morpholino nucleic acid as a whole (100%) or in part *(e.g.,* 80% or more of the whole).

The first nucleic acid strand and the second nucleic acid strand may comprise any combination of the above modified internucleoside linkage and modified nucleoside.

The first nucleic acid strand and the second nucleic acid strand may be linked via a linker. In this case, the first nucleic acid strand and the second nucleic acid strand can be linked via a linker to form a single-strand. However, even in that case, the functional region has the same configuration as the double-stranded nucleic acid complex, and therefore such a single-stranded nucleic acid is herein also encompassed as an embodiment of the double-stranded nucleic acid complex of the present invention. The linker can be any polymer. Examples thereof include a polynucleotide, polypeptide, and alkylene. Specifically, it can be composed of a natural nucleotide such as DNA, and RNA, or a non-natural nucleotide such as a peptide nucleic acid and a morpholino nucleic acid. When a linker consists of a nucleic acid, the chain length of a linker may be at least one base, or a chain length of from 3 to 10 bases or from 4 to 6 bases. It is preferably 4 base in length. The position of the linker can be either on the 5' side or the 3' side of the first nucleic acid strand. For example, in the case of a configuration in which cholesterol or analog thereof is bound to the 5' side of the second nucleic acid strand, the 5' end of the first nucleic acid strand and the 3' end of the second nucleic acid strand are linked via a linker. In an embodiment, the first nucleic acid strand is a hemi-gapmer with a wing region only on the 3' end side, and the second nucleic acid strand is a nucleic acid strand that does not comprise a sugar-modified nucleoside.

The second nucleic acid strand is bound to cholesterol or analog thereof.

The second nucleic acid strand bound to cholesterol or analog thereof may have the group represented by the following Formula (I).

[Chem 1]

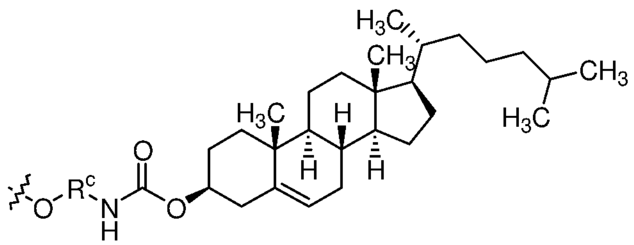

(I)

[wherein $R^c$ represents a C4-C18, preferably C5-C16 alkylene group which may have a substituent (wherein the substituent is a halogen atom, or a C1-C3 alkyl group that may be substituted with a hydroxy group, such as a hydroxymethyl group, and in the alkylene group mutually non-adjacent carbon atoms may be substituted with an oxygen atom)].

$R^c$ may be, but not limited to, $-(CH_2)_3-O-(CH_2)_2-O-(CH_2)_2-O-(CH_2)_2-O-(CH_2)_2-$,$-(CH_2)_3-O-(CH_2)_2-O-(CH_2)_2-O-(CH_2)_2-O-CH_2-CH(CH_2OH)-$, or $-(CH_2)_6-$.

A group represented by the above Formula (I) or (II) can be bound to the 5' end or the 3' end of the second nucleic acid strand via a phosphoester bond.

Cholesterol or analog thereof may be bound to any of the 5' end, the 3' end, or both the ends of the second nucleic acid strand. Further, cholesterol or analog thereof may also be bound to a nucleotide inside the second nucleic acid strand. Without limitation, cholesterol or analog thereof bound to the 5' end of the second nucleic acid strand is particularly suitable.

When the second nucleic acid strand comprises a plurality of cholesterol or analog thereof, they may be the same or different. For example, a case in which cholesterol is bound to the 5' end of the second nucleic acid strand and another cholesterol analog is bound to the 3' end one each corresponds to such a case. With respect to binding positions, cholesterol or analog thereof may be bound to a plurality of positions of the second nucleic acid strand, and/or may be bound as a group to a single position. One cholesterol or analog thereof may be bound to each of the 5' end and 3' end of the second nucleic acid strand.

The bond between the second nucleic acid strand and cholesterol or analog thereof may be a direct bond, or an indirect bond mediated by another substance.

When the second nucleic acid strand and cholesterol or analog thereof are bound directly, it is sufficient if the latter is bound to the second nucleic acid strand via a covalent bond, an ionic bond, a hydrogen bond, or the like. A covalent bond is preferable considering that a more stable bond can be obtained.

When the second nucleic acid strand and cholesterol or analog thereof are bound indirectly, they may be bound via a linking group (herein often referred to as a "linker"). The linker may be either of a cleavable linker and an uncleavable linker.

A "cleavable linker" refers to a linker that can be cleaved under physiological conditions, for example, in a cell or in an animal body *(e.g.,* in a human body). A cleavable linker is selectively cleaved by an endogenous enzyme such as a nuclease. Examples of a cleavable linker comprise, but not limited to, an amide, an ester, one or both esters of a phosphodiester, a phosphoester, a carbamate, and a disulfide bond, as well as a natural DNA linker. As an example, cholesterol or analog thereof may be bound via a disulfide bond.

An "uncleavable linker" refers to a linker that is not cleaved under physiological conditions, for example, in a cell or in an animal body *(e.g.,* in a human body). Examples of an uncleavable linker comprise, but not limited to, a phosphorothioate linkage, modified or unmodified deoxyribonucleosides linked by a phosphorothioate linkage, and a linker consisting of modified or unmodified ribonucleosides. There is no particular restriction on the chain length, when a linker is a nucleic acid such as DNA, or an oligonucleotide, however it may be usually from 2 to 20 base in length, from 3 to 10 base in length, or from 4 to 6 base in length.

Specific examples of the above linker comprise linkers represented by the following Formula II.

[Chem 2]

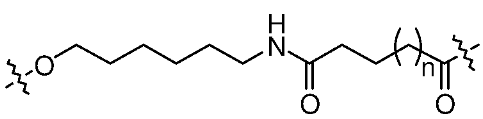

(II)

[wherein n represents 0 or 1.]

The second nucleic acid strand may further comprise at least one functional moiety bound to the polynucleotide constituting the nucleic acid strand. A "functional moiety" refers to a moiety that confers a desired function to a double-stranded nucleic acid complex and/or the nucleic acid strand to which the functional moiety is bound. Examples of the desired function may comprise a labeling function or a purification function. Examples of the moiety that confers a labeling function comprise a compound such as a fluorescent protein and luciferase. Examples of the moiety that confers a purification function comprise a compound such as biotin, avidin, His tag peptide, GST tag peptide, and FLAG tag peptide. The binding position and type of binding of a functional moiety in the second nucleic acid strand are as described above in connection with the binding of cholesterol or analog thereof to the second nucleic acid strand.

In the double-stranded nucleic acid complex of the present invention, the antisense effect in the skeletal muscle or heart muscle on the target transcription product of the first nucleic acid strand can be measured by a method publicly known in the art. For example, after introducing a double-stranded nucleic acid complex into a cell and the like, it can be measured using a publicly known technique such as Northern blotting, quantitative PCR, or Western blotting. By measuring the expression level of a target gene or the level of a target transcription product in skeletal muscle cells or heart muscle cells *(e.g.,* the amount of mRNA, the amount of RNA such as microRNA, the amount of cDNA, and the amount of protein), it can be judged whether or not the target gene expression is suppressed by the double-stranded nucleic acid complex in these sites. As the judgement criteria, without limitation, when the expression level of the target gene or the measurement of the target transcription product is reduced by at least 5%, at least 10%, at least 15%, at least 20%, at least 25%, at least 30%, or at least 40% compared to the measurement of a negative control *(e.g.,* vehicle administration), it may be judged that the double-stranded nucleic acid complex of the present invention has produced an antisense effect in the skeletal muscle or heart muscle.

An exemplary embodiment of the double-stranded nucleic acid complex of the present invention has been described above, however the double-stranded nucleic acid complex of the present invention is not limited to the above exemplary embodiment.

1-4. Method for producing a double-stranded nucleic acid complex

Those skilled in the art can produce the double-stranded nucleic acid complex of the present invention by appropriately selecting a publicly known method. Usually, without limitation, firstly each of the first nucleic acid strand and the second nucleic acid strand that constitute a double-stranded nucleic acid complex is designed and prepared. For example, the first nucleic acid strand is designed based on the information on the base sequence of the target transcription product *(e.g.,* the base sequence of the target gene), and the second nucleic acid strand is designed as a complementary strand thereto. Then, based on the information on the designed base sequences, each nucleic acid strand is synthesized using a commercially available automatic nucleic acid synthesizer, such as that from GE Healthcare, Thermo Fisher Scientific, or Beckman Coulter. Thereafter the prepared oligonucleotides may be purified using a reverse-phase column or the like.

In the case of a double-stranded nucleic acid complex to which a functional moiety is bound, a first nucleic acid strand may be produced according to the above method. Meanwhile, with respect to a second nucleic acid strand to which a functional moiety is bound, it may be produced by performing the aforedescribed synthesis and purification using a nucleic acid species to which a functional moiety has been bound in advance. For example, a second nucleic acid strand may be produced by performing the aforedescribed synthesis and purification using a nucleic acid species to which a cholesterol or analog thereof has been bound in advance. Alternatively, cholesterol or analog thereof may be joined by a publicly known method to a second nucleic acid strand produced by performing the aforedescribed synthesis and purification. After preparation of each nucleic acid strand, a double-stranded nucleic acid complex to which the functional moiety of interest is bound can be produced by performing annealing described below for the first nucleic acid strand and the second nucleic acid strand.

The method for linking a functional moiety to a nucleic acid is well known in the art. The nucleic acids produced by this method are mixed in an appropriate buffer solution to be denatured at about 90°C to 98°C for several minutes *(e.g.,* 5 min), and then the nucleic acids are annealed in a range of about 30°C to 70°C for about 1 to 8 hours to yield a double-stranded nucleic acid complex of the present invention. Further, a nucleic acid strand can be obtained by ordering from various manufacturers (*e.g*., GeneDesign Inc.) by specifying the base sequence and the modification site and type. The aforementioned annealing step can be performed by allowing the nucleic acids to stand at room temperature (about 10°C to about 35°C) for about 5 to 60 minutes. It is possible that the first nucleic acid strand and the second nucleic acid strand may be independently dissolved in a buffer solution (*e.g*., phosphate-buffered saline) or water at about 70°C to 98°C, and the obtained two solutions are mixed, and the mixed liquid is kept at about 70°C to 98°C for several minutes *(e.g.,* 5 minute), and then the same is maintained at about 30°C to 70°C (or 30°C to 50°C) for about 1 to 8 hours to prepare a double-stranded nucleic acid complex of some embodiments of the present invention. It is also possible that the first nucleic acid strand and the second nucleic acid strand are independently dissolved in a buffer solution (*e.g*.,

phosphate-buffered saline) or water at room temperature (about 10°C to about 35°C). The annealing conditions (time and temperature) in preparing a double-stranded nucleic acid complex are not limited to the above conditions. In addition, the conditions suitable for promoting annealing of nucleic acid strands are well known in the art.

1-5. Effects

The double-stranded nucleic acid complex of the present invention can be efficiently delivered to the skeletal muscle or heart muscle of a subject so that the antisense effect on the target gene is produced at the site to suppress the expression of the gene. Therefore, by using this double-stranded nucleic acid complex as an active ingredient, it is possible to treat or prevent a disease such as a muscle disease which may develop or advance in severity due to the expression of the target gene in the skeletal muscle or heart muscle of a subject.

2. Pharmaceutical composition

2-1. Overview

The second aspect of the present invention is a pharmaceutical composition. The pharmaceutical composition of the present invention comprises the double-stranded nucleic acid complex of the first aspect as an active ingredient, and/or a delivery molecule of a drug to the skeletal muscle or heart muscle. The double-stranded nucleic acid complex of the first aspect can regulate the expression level of a target transcription product in the skeletal or heart muscle by an antisense effect. Therefore, by administering the pharmaceutical composition of the present invention to a subject, the double-stranded nucleic acid complex can be delivered to the skeletal muscle or heart muscle of the subject to treat a disease such as a muscle disease that may develop in the sites. The pharmaceutical composition of the present invention may essentially consist of the double-stranded nucleic acid complex of the first aspect. In other words, the pharmaceutical composition of the present invention may further comprise auxiliary components such as a carrier in addition to the double-stranded nucleic acid complex of the first aspect. The pharmaceutical composition of the present invention may consist solely of the double-stranded nucleic acid complex of the first aspect.

2-2. Configuration

The pharmaceutical composition of the present invention can comprise an active ingredient and a carrier as essential ingredients. Each component is described in detail below.

2-2-1. Active ingredient

An active ingredient is an essential constituent in the pharmaceutical composition of the present invention. The pharmaceutical composition of the present invention comprises as an active ingredient at least a double-stranded nucleic acid complex described in the first aspect above. The pharmaceutical composition of the present invention may comprise two or more kinds of the double-stranded nucleic acid complex.

The amount (content) of the double-stranded nucleic acid complex in a pharmaceutical composition varies depending on the kind of double-stranded nucleic acid complex, the site (skeletal muscle or heart muscle) to be delivered, the dosage form of the pharmaceutical composition, the dose of the pharmaceutical composition, and the kind of carrier described below. Therefore, it may be determined as appropriate by taking the respective conditions into consideration. Normally, it may be adjusted so that an effective amount of the double-stranded nucleic acid complex is contained in a single dose of the pharmaceutical composition. An "effective dose" is an amount that is necessary for the double-stranded nucleic acid complex to function as an active ingredient, and has little or no adverse side effects on the living body to which it is applied. This effective amount can vary depending on various conditions such as information on the subject, the administration route, and number of administrations. Ultimately, it may be determined by the judgment of a physician, veterinarian, pharmacist, or the like. "Information on the subject" is various information on an individual of the living body to which the pharmaceutical composition is applied. For example, when the subject is a human, it comprises age, body weight, gender, dietary habit, health status, stage of progression or grade of severity of the disease, drug sensitivity, and presence of a combined drug.

2-2-2. Carrier

The pharmaceutical composition of the present invention may comprise a pharmaceutically acceptable carrier. A "pharmaceutically acceptable carrier" refers to an additive commonly used in the field of pharmaceutical preparation. Examples thereof include a solvent, a vegetable oil, a base, an emulsifier, a suspending agent, a surfactant, a pH

adjuster, a stabilizer, a seasoning, a flavor, an excipient, a vehicle, a preservative, a binder, a diluent, an isotonizing agent, a sedative, a bulking agent, a disintegrating agent, a buffering agent, a coating agent, a lubricant, a colorant, a sweetener, a thickener, a corrective agent, a dissolution aid, and other additives.

The solvent may be any of, for example, water or other pharmaceutically acceptable aqueous medium, and a pharmaceutically acceptable organic solvent. Examples of an aqueous solution comprise a physiological saline, an isotonic solution containing glucose or another additive, a phosphate buffered saline, and a sodium acetate buffer solution. Examples of the additive comprise D-sorbitol, D-mannose, D-mannitol, sodium chloride, and further a nonionic surfactant at a low concentration, and polyoxyethylene sorbitan fatty acid ester.

The above carrier is used to avoid or suppress degradation of the double-stranded nucleic acid complex, which is an active ingredient, *in vivo* by an enzyme and the like, and additionally to facilitate formulation or administration, and to maintain the dosage form and drug efficacy. Therefore, it may be used as appropriate and as needed.

2-2-3. Dosage form

There is no particular restriction on the dosage form of the pharmaceutical composition of the present invention as long as the double-stranded nucleic acid complex described in the first aspect, which is an active ingredient, is delivered to the skeletal muscle or heart muscle, which is the target site, without inactivation by degradation or the like, and the pharmacological effect of the active ingredient (antisense effect on target gene expression) can be produced *in vivo.*

The specific dosage form varies depending on the administration method and/or medication conditions. The administration methods can be broadly classified into parenteral administration and peroral administration, and the dosage form appropriate for the respective administration methods can be selected.

When the administration method is parenteral administration, the preferred dosage form is liquid formulation which can be administered directly to the target site, or administered systemically via the circulatory system. Examples of the liquid formulation comprise an injectable. The injectable can be formulated by mixing in an appropriate combination with the aforedescribed excipient, elixir, emulsifier, suspending agent, surfactant, stabilizer, pH adjuster, etc. in the form of a unit dose required according to the generally approved pharmaceutical practices. In addition, it may be ointment, plaster, cataplasm, transdermal patch, lotion, inhalant, aerosol, eye drop, and suppository.

When the administration method is oral administration, the preferred dosage form comprises solid preparation (comprising tablet, capsule, drop, and lozenge), granule, dusting powder, powder, and liquid formulation (comprising oral liquid preparation, emulsion formulation, and syrup). In the case of a solid preparation, if necessary, it may take a dosage form with a coating as publicly known in the art, such as a sugar-coated tablet, a gelatin-coated tablet, an enteric-coated tablet, a film-coated tablets, a double layer tablet, and a multilayer tablet.

There is no particular restriction on the specific shape and size of each of the above-mentioned dosage forms, as long as the respective dosage forms are within the ranges of dosage forms publicly known in the art. As for the manufacturing method of the pharmaceutical composition of the present invention, it may be formulated according to the common procedure in the art.

2-3. Dosing form and dose

Herein there is no particular restriction on the preferable dosing form of a pharmaceutical composition. For example, it can be oral administration or parenteral administration. Specific examples of the parenteral administration comprise intramuscular administration, intravenous administration, intraarterial administration, intraperitoneal administration, subcutaneous administration (comprising implanted continuous subcutaneous administration), tracheal/bronchial administration, and rectal administration, as well as administration by blood transfusion. Considering that the application target site of the present invention is the skeletal muscle or heart muscle, intramuscular injection administration and intravenous infusion administration at the target site are suitable.

When a pharmaceutical composition is applied by administration or ingestion, the administered amount or ingested amount may be, for example, from 0.00001 mg/kg/day to 10000 mg/kg/day, or from 0.001 mg/kg/day to 100 mg/kg/day for the double stranded nucleic acid complex contained in the pharmaceutical composition. A pharmaceutical composition may be applied by single-dose administration or multiple dose administration. In the case of multiple dose administration, it may be administered daily or at appropriate time intervals (*e.g.*, at intervals of 1 day, 2 days, 3 days, 1 week, 2 weeks, or 1 month), for example, for 2 to 20 times. A single dose of the double-stranded nucleic acid complex described above may be, for example, 0.001 mg/kg or more, 0.005 mg/kg or more, 0.01 mg/kg or more, 0.25 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any dose in the range of from 0.001 mg/kg

to 500 mg/kg *(e.g.,* 0.001 mg/kg, 0.01 mg/kg, 0.1 mg/kg, 1 mg/kg, 5 mg/kg, 10 mg/kg, 50 mg/kg, 100 mg/kg, or 200 mg/kg) may be selected as appropriate.

The double-stranded nucleic acid complex of the present invention may be administered twice a week for total four times at a dose of from 0.01 to 10 mg/kg *(e.g.,* about 6.25 mg/kg). Alternatively, the double-stranded nucleic acid complex may be administered once or twice a week for total two to four times, for example at a frequency of twice a week for total two times, at a dose of from 0.05 to 30 mg/kg *(e.g.,* about 25 mg/kg). By adopting such a dosing regimen (divided administration), the toxicity can be lowered (*e.g.*, avoidance of platelet reduction) compared to a single-dose administration at a higher dose, and the stress to the subject can be reduced.

Even when the pharmaceutical composition is repeatedly administered, its inhibitory effect can be produced additively in a cell. In the case of repeated administration, the efficacy can be improved with certain administration intervals (*e.g.*, half a day or longer)

2-4. Applicable diseases

The disease to which the pharmaceutical composition is applicable are a disease that can develop or become severe as the result of expression of the target gene in the skeletal muscle or heart muscle. Examples thereof include, but not limited to, a muscle disease.

In the present invention, a "muscle disease" is a generic term for a disease that causes muscular weakness due to a muscle cell (comprising a skeletal muscle cell, or a heart muscle cell). Examples thereof include muscular dystrophy, myopathy, inflammatory myopathy (comprising polymyositis and dermatomyositis), Danon disease, myasthenic syndrome, mitochondrial disease, myoglobinuria, glycogen storage disease, periodic paralysis, hereditary cardiomyopathy, hypertrophic cardiomyopathy, dilated cardiomyopathy, and arrhythmia comprising hereditary arrhythmia. A disease that has a primary cause in another organ and can cause secondary dysfunction of a skeletal muscle or heart muscle cell is also included. Examples thereof include neurodegenerative disorder, sarcopenia, and cachexia.

2-5. Drug delivery

The pharmaceutical composition of the present invention can deliver a specific drug to the skeletal muscle or heart muscle by binding the drug to the first nucleic acid strand and/or the second nucleic acid strand, utilizing the fact that the double-stranded nucleic acid complex of the first aspect comprised as an active ingredient can be efficiently delivered to the skeletal muscle or heart muscle. There is no particular restriction on the drug that is delivered to the skeletal muscle or heart muscle, and examples thereof include a peptide, a protein, and a nucleic acid drug as well as other organic compounds, such as an anti-tumor drug, a hormonal drug, an antibiotic, an antiviral drug, and an anti-inflammatory drug. A preferable drug is a small-molecule drug. The small-molecule drug is well understood by those skilled in the art. It refers to typically a drug with a molecular weight less than 1,000 Dalton. The drug may be also a lipophilic drug. Examples of the nucleic acid drug comprise, but not limited to, ASO, antagomiR, splice switching oligonucleotide, aptamer, single-stranded siRNA, microRNA, and pre-microRNA. The position of binding and the type of binding of the drug in the second nucleic acid strand are as described above in connection with the binding of cholesterol or analog thereof to the second nucleic acid strand.

2-6. Effects

The pharmaceutical composition can treat or prevent a muscle disease or the like which can be caused by the expression of a particular gene in the skeletal muscle or heart muscle.

The pharmaceutical composition of the present invention can be efficiently delivered to the skeletal muscle or heart muscle as disclosed in the following Examples, and can effectively suppress the expression of a target gene or the level of a target transcription product at the site. Therefore, a method for reducing the expression level of a target transcription product in the skeletal muscle or heart muscle of a subject comprising administering a pharmaceutical composition comprising the double-stranded nucleic acid complex described above to a subject is provided. The method may be a method for treating a muscle disease in a subject. Further, a method for delivering a drug to the skeletal muscle or heart muscle of a subject comprising administering a pharmaceutical composition comprising the double-stranded nucleic acid complex described above to a subject is also provided.

Examples

<Example 1>

(Purpose)

The *in vivo* inhibitory effect on the expression of mRNA in a tissue by a double-stranded nucleic acid complex agent consisting of an antisense oligonucleotide targeting the SR-B1 gene, and a tocopherol- or cholesterol-conjugated complementary strand is examined.

(Method)

(1) Preparation of nucleic acids

As a target gene, a scavenger receptor B1 (SR-B 1) was selected. The names and base sequences of the first nucleic acid strand and the second nucleic acid strand constituting the double-stranded nucleic acid complex agent used in this Example are shown in Table 1.

[Table 1]

| | Name of oligonucleotide | Sequence (5'-3') | SEQ ID NO |
|---|---|---|---|
| First nucleic acid strand | ASO(mSR-B1) | T*C*a*g*t*c*a*t*g*a*c*t*T*C | 7 |
| Second nucleic acid strand | Toc#1-cRNA(mSR-B1) | Toc-g*a*AGUCAUGACU*g*a | 8 |
| Second nucleic acid strand | Chol#1-cRNA(mSR-B1) | Chol-g*a*AGUCAUGACU*g*a | 8 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Toc: tocopherol; Chol: cholesterol | | | |

The above first nucleic acid strand targets the murine SR-B 1 gene, and is composed of a 14-mer single-stranded LNA/DNA gapmer having a base sequence that is complementary to position 2479 to 2492 of SR-B1 mRNA (GenBank Accession number NM_016741, SEQ ID NO:1), which is a transcription product of the target gene. More specifically, this LNA/DNA gapmer consists of each two LNA nucleosides from the 5' end and the 3' end respectively, and ten DNA nucleosides between them.

The above second nucleic acid strand is composed of a tocopherol-conjugated complementary strand RNA (Toc#1-cRNA(mSR-B1)), to which tocopherol is bound at the 5' end, or a cholesterol-conjugated complementary strand RNA (Chol#1-cRNA(mSR-B1)), to which tocopherol is bound at the 5' end, both of which has a sequence complementary to the first nucleic acid strand.

By annealing the first nucleic acid strand with one of the two second nucleic acid strands, a tocopherol-conjugated heteroduplex oligonucleotide (hereinafter referred to as "Toc-HDO"), or a cholesterol-conjugated heteroduplex oligonucleotide (hereinafter referred to as "Chol-HDO"), which was a double-stranded nucleic acid complex agent of the present invention, was prepared. The first nucleic acid strand was mixed with the second nucleic acid strand in equimolar amounts, the solution was heated at 95°C for 5 min, then cooled to 37°C and retained for 1 hour allowing the nucleic acid strand to anneal, thereby preparing the double-stranded nucleic acid complex agent. The annealed nucleic acids were stored at 4°C or on ice. The double-stranded nucleic acid complex agent after the preparation is referred to as "Toc#1HDO(mR-B1)" or "Chol#1HDO(mSR-B1)".

A conventional single-stranded antisense oligonucleotide (ASO) (control ASO) was used as a reference for comparison with a double-stranded nucleic acid complex agent. This control ASO has the same configuration as the first nucleic acid strand of the double-stranded nucleic acid complex agent. The single-stranded ASO after preparation was designated as "ASO(mSR-B1)".

*(2) In vivo* experiment

As the mice to which a double-stranded nucleic acid complex agent or the like is administered, 6 to 7 week-old male C57BL/6 mice of body weight 20 g were used. In this and following Examples, all experiments using mice were conducted with n=4.

The double-stranded nucleic acid complex agent and the control ASO(mSR-B1) were intravenously injected

through the tail vein into a mouse at a dose of 50 mg/kg respectively in a single-dose administration. In addition, mice injected with PBS only in a single-dose administration were also produced as a negative control group.

(3) Expression analysis

At the time point of 72 hours after the administration, PBS was perfused into the mice, and then the mice were dissected to isolate the heart muscle, quadriceps muscle, diaphragm, and musculi dorsi proprii. Then mRNA was extracted from each tissue using a high-throughput fully automated nucleic acid extraction device MagNA Pure 96 (Roche Life Science) according to the protocol. cDNA was synthesized according to the protocol attached to Transcriptor Universal cDNA Master (Roche Life Science). Quantitative RT-PCR was performed with TaqMan (Roche Life Science). As the primers used in the quantitative RT-PCR, the products designed and produced by Thermo Fisher Scientific based on various gene numbers were used. The PCR conditions (temperature and time) were 95°C for 15 sec, 60°C for 30 sec, and 72°C for 1 sec as 1 cycle, and 40 cycles were repeated. The amplified product thus obtained was quantified by quantitative RT-PCR, and based on the result, the expression level of mRNA (SR-B1)/expression level of mRNA (ACTB: internal standard gene) were calculated respectively to obtain a relative expression level. The mean value and standard error of the relative expression levels were calculated. Further, the results of the individual groups were compared, and evaluated by t-test.

(Results)

The results are shown in Figure 3. Figure 3 shows the inhibitory effects of the double-stranded nucleic acid complexes of the present invention targeting the SR-B1 gene to which tocopherol or cholesterol is bound (Toc#1HDO(mSR-B1), and Chol#1HDO(mSR-B1), respectively) on the expression of the target SR-B1 gene in the heart muscle (Heart), quadriceps muscle (Quadriceps), diaphragm (Diaphragm), and musculi dorsi proprii (Back). The error bars indicate the respective standard errors.

Both the Toc#1HDO(mSR-B1) and Chol#1HDO(mSR-B1) showed significant inhibitory effects on the expression of the target gene in various skeletal muscles and the heart muscle compared to the single-stranded control ASO(mSR-B1).

<Example 2>

(Purpose)

The *in vivo* inhibitory effect on the expression of mRNA in a tissue by a single-dose administration of a double-stranded nucleic acid complex agent targeting the malat1 gene in which the second nucleic acid strand consists of a tocopherol- or cholesterol-conjugated complementary strand was examined.

(Method)

(1) Preparation of nucleic acids

The metastasis associated lung adenocarcinoma transcription product (malat1) was selected as a target gene. The names and base sequences of the first nucleic acid strand and the second nucleic acid strand constituting the double-stranded nucleic acid complex agent used in this Example are shown in Table 2.

[Table 2]

| | Name of oligonucleotide | Sequence (5'-3') | SEQ ID NO |
|---|---|---|---|
| First nucleic acid strand | ASO(mMalat1) | C*T*A*g*t*t*c*a*c*t*g*a*a*T*G*C | 9 |
| Second nucleic acid strand | Toc#1-cRNA(mMalat1) | Toc-g*c*a*UUCAGUGAAC*u*a*g | 10 |
| Second nucleic acid strand | Chol#1-cRNA(mMalat1) | Chol-g*c*a*UUCAGUGAAC*u*a*g | 10 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Toc: tocopherol; Chol: cholesterol | | | |

The above first nucleic acid strand targets the murine malat1 gene, and is composed of a 16-mer single-stranded

LNA/DNA gapmer having a base sequence that is complementary to position 1316 to 1331 targeting malat1 noncoding RNA (GenBank Accession number NR_002847, SEQ ID NO:3), which is a transcription product of the target gene. More specifically, this LNA/DNA gapmer consists of each three LNA nucleosides from the 5' end and the 3' end respectively, and ten DNA nucleosides between them.

Meanwhile, the second nucleic acid strand is composed of a tocopherol-conjugated complementary strand RNA (Toc#1-cRNA(mMalat1)), to which tocopherol is bound at the 5' end, or a cholesterol-conjugated complementary strand RNA (Chol#1-cRNA(mMalat1)), to which cholesterol is bound at the 5' end, both of which have a sequence complementary to the first nucleic acid strand.

The above first nucleic acid strand was mixed with either of two kinds of the second nucleic acid strands in equimolar amounts, the solution was heated at 95°C for 5 min, then cooled to 37°C and retained for 1 hour allowing the two nucleic acid strands to anneal, thereby preparing the double-stranded nucleic acid complex agent described above. The annealed nucleic acids were stored at 4°C or on ice. The double-stranded nucleic acid complex agent after the preparation is referred to as "Toc#1HDO(mMalat1)" or "Chol#1HDO(mMalat1)".

A conventional single-stranded antisense oligonucleotide (ASO) (control ASO) was used as a reference for comparison with a double-stranded nucleic acid complex agent. This control ASO has the same configuration as the first nucleic acid strand of the double-stranded nucleic acid complex agent. The single-stranded ASO after preparation was designated as "ASO(mMalat1)".

*(2) In vivo* experiment

As the mice to which a double-stranded nucleic acid complex agent or the like is administered, 6 to 7 week-old male C57BL/6 mice of body weight 20 g were used.

The double-stranded nucleic acid complex agent and the control ASO were intravenously injected through the tail vein into each mouse at a dose of 50 mg/kg in a single-dose administration. In addition, mice injected with PBS only in a single-dose administration were also produced as a negative control group.

(3) Expression analysis

At the time point of 72 hours after the administration, PBS was perfused into the mice, and then the mice were dissected to isolate the heart muscle, quadriceps muscle, diaphragm, and musculi dorsi proprii. Then mRNA was extracted from each tissue using a high-throughput fully automated nucleic acid extraction device MagNA Pure 96 (Roche Life Science) according to the protocol. cDNA was synthesized according to the protocol attached to Transcriptor Universal cDNA Master (Roche Life Science). Quantitative RT-PCR was performed with TaqMan (Roche Life Science). As the primers used in the quantitative RT-PCR, the products designed and produced by Thermo Fisher Scientific based on various gene numbers were used. The PCR conditions (temperature and time) were 95°C for 15 sec, 60°C for 30 sec, and 72°C for 1 sec as 1 cycle, and 40 cycles were repeated. The amplified product thus obtained was quantified by quantitative RT-PCR, and based on the result, the expression level of mRNA (malat1)/the expression level of mRNA (ACTB; internal standard gene) were calculated respectively to obtain a relative expression level. The mean value and standard error of the relative expression levels were calculated. Further, the results of the individual groups were compared, and evaluated by t-test.

(Results)

The results are shown in Figure 4. Figure 4 shows the inhibitory effects of the double-stranded nucleic acid complexes to which tocopherol or cholesterol is bound (Toc#1HDO(mMalat1), and Chol#1HDO(mMalat1), respectively) on the expression of the target Malat1 gene in the heart muscle (Heart), quadriceps muscle (Quadriceps), diaphragm (Diaphragm), and musculi dorsi proprii (Back). The error bars indicate the respective standard errors.

Both the Toc#1HDO(mMalat1) and Chol#1HDO(mMalat1) showed significant inhibitory effects on the expression of the target gene in various skeletal muscles and the heart muscle compared to the single-stranded control ASO(mMalat).

<Example 3>

(Purpose)

The *in vivo* inhibitory effect on the expression of mRNA in a tissue by a single-dose administration of a double-stranded nucleic acid complex agent consisting of an antisense oligonucleotide targeting the DMPK gene and a tocopherol- or cholesterol-conjugated complementary strand was examined as in Examples 1 and 2.

(Method)

(1) Preparation of nucleic acids

The DMPK (dystrophia myotonica-protein kinase) gene was selected as a target gene. The DMPK gene encoding a myotonin-protein kinase is known to be the responsible gene for myotonic dystrophy, which is the most frequent form of muscular dystrophy in adults. It is thought that an abnormal elongation of the CTG repeat sequence present in the 3' untranslated region of the DMPK gene is a cause of the disease.
The names and base sequences of the first nucleic acid strand and the second nucleic acid strand constituting the double-stranded nucleic acid complex agent used in this Example are shown in Table 3.

[Table 3]

| | Name of oligonucleotide | Sequence (5'-3') | SEQ ID NO |
|---|---|---|---|
| First nucleic acid strand | ASO(mDMPK) | A*C*A*a*t*a*a*a*t*a*c*c*g*A*G*G | 11 |
| Second nucleic acid strand | Toc#1-cRNA(mDMPK) | Toc-c*c*u*CGGUAUUUAU*u*g*u | 12 |
| Second nucleic acid strand | Chol#1-cRNA(mDMPK) | Chol-c*c*u*CGGUAUUUAU*u*g*u | 12 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Toc: tocopherol; Chol: cholesterol | | | |

The above first nucleic acid strand targets the murine DMPK gene, and is composed of a 16-mer single-stranded LNA/DNA gapmer having a base sequence that is complementary to position 2682 to 2697 targeting DMPK mRNA (GenBank Accession number NM_032418, SEQ ID NO:5), which is a transcription product of the target gene. More specifically, this LNA/DNA gapmer consists of each three LNA nucleosides from the 5' end and the 3' end respectively, and ten DNA nucleosides between them.
The above second nucleic acid strand is composed of a tocopherol-conjugated complementary strand RNA (Toc#1-cRNA(mDMPK)), to which tocopherol is bound at the 5' end, or a cholesterol-conjugated complementary strand RNA (Chol#1-cRNA(mDMPK)), to which cholesterol is bound at the 5' end, both of which have a sequence complementary to the first nucleic acid strand.
By annealing the above first nucleic acid strand with either of the second nucleic acid strands, Toc-HDO, or cholesterol-conjugated heteroduplex oligonucleotide Chol-HDO, which was a double-stranded nucleic acid complex agent of the present invention, was prepared. Specifically, the first nucleic acid strand and the second nucleic acid strand were mixed in equimolar amounts, the solution was heated at 95°C for 5 min, then cooled to 37°C and retained for 1 hour allowing the two nucleic acid strands to anneal, thereby preparing the double-stranded nucleic acid complex agent. The annealed nucleic acids were stored at room temperature, 4°C, or on ice. The double-stranded nucleic acid complex agent after the preparation is referred to as "Toc#1HDO(mDMPK)" or "Chol#1HDO(mDMPK)".
A conventional single-stranded antisense oligonucleotide (ASO) (control ASO) was used as a reference for comparison with a double-stranded nucleic acid complex agent. This control ASO has the same configuration as the first nucleic acid strand of the double-stranded nucleic acid complex agent. The single-stranded ASO after preparation was designated as "ASO(mDMPK)".

(2) *In vivo* experiment

As the mice to which a double-stranded nucleic acid complex agent or the like was administered, 6 to 7 week-old male C57BL/6 mice of body weight 20 g were used.
The double-stranded nucleic acid complex agent and the control ASO were intravenously injected through the tail vein into each mouse at a dose of 12.5 mg/kg, 25 mg/kg, or 50 mg/kg in a single-dose administration. In addition, mice injected with PBS only in a single-dose administration were also produced as a negative control group.

(3) Expression analysis

At the time point of 72 hours after the administration, PBS was perfused into the mice, and then the mice were dissected to isolate the heart muscle (Heart), quadriceps muscle (Quadriceps), diaphragm (Diaphragm), musculi dorsi proprii (Back), tibialis anterior muscle (TA), gastrocnemius muscle (GC), and triceps brachii muscle (TB). Then mRNA was extracted from each tissue using a high-throughput fully automated nucleic acid extraction device MagNA Pure 96

(Roche Life Science) according to the protocol. cDNA was synthesized according to the protocol attached to Transcriptor Universal cDNA Master (Roche Life Science). Quantitative RT-PCR was performed with TaqMan (Roche Life Science). As the primers used in the quantitative RT-PCR, the products designed and produced by Thermo Fisher Scientific based on various gene numbers were used. The PCR conditions (temperature and time) were 95°C for 15 sec, 60°C for 30 sec, and 72°C for 1 sec as 1 cycle, and 40 cycles were repeated. The amplified product thus obtained was quantified by quantitative RT-PCR, and based on the result, the expression level of mRNA (DMPK)/the expression level of mRNA (ACTB; internal standard gene) were calculated respectively to obtain a relative expression level. The mean value and standard error of the relative expression levels were calculated. Further, the results of the individual groups were compared, and evaluated by t-test.

(Results)

The results are shown in Figures 5 to 7. Figures 5, 6, and 7 respectively show the inhibitory effects of the double-stranded nucleic acid complexes to which tocopherol or cholesterol is bound (Toc#1HDO(mDMPK), and Chol#1HDO(mDMPK), respectively) administered at 12.5 mg/kg, 25 mg/kg, and 50 mg/kg on the expression of the target DMPK gene in the gastrocnemius muscle (GC), tibialis anterior muscle (TA), triceps brachii muscle (TB), quadriceps muscle (Quadriceps), diaphragm (Diaphragm), musculi dorsi proprii (Back), and heart muscle (Heart). The error bars indicate the respective standard errors.

Both the Toc#1HDO(mDMPK) and Chol#1HDO(mDMPK) showed significant inhibitory effects on the expression of the target gene in various skeletal muscles and the heart muscle compared to the single-stranded control ASO(mDMPK). The effects of Chol#1HDO(mDMPK) were particularly remarkable in a dose-dependent manner.

<Example 4: DNA only>

(Purpose)

The *in vivo* inhibitory effect on the expression of a target gene in the heart muscle and skeletal muscle by a single-dose administration of a double-stranded nucleic acid complex agent to which tocopherol or cholesterol was bound in which the second nucleic acid strand is composed of DNA was examined.

(Method)

(1) Preparation of nucleic acids

α-tocopherol and cholesterol are bound to the second nucleic acid strand in the basic structure of the double-stranded nucleic acid complex agent in this Example. It differs from Example 2 in that the second nucleic acid strand is composed entirely of DNA. The names and base sequences of the first nucleic acid strand and the second nucleic acid strand constituting the double-stranded nucleic acid complex agent used in this Example are shown in Table 4.

[Table 4]

| | Name of oligonucleotide | Sequence (5'-3') | SEQ ID NO |
|---|---|---|---|
| First nucleic acid strand | ASO(mMalatl) | C*T*A*g*t*t*c*a*c*t*g*a*a*T*G*C | 9 |
| Second nucleic acid strand | Toc#1-cDNA(mMalat1) | Toc-g*c*a*ttcagtgaac*t*a*g | 13 |
| Second nucleic acid strand | Chol#1-cDNA(mMalat1) | Chol-g*c*a*ttcagtgaac*t*a*g | 13 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Toc: tocopherol; Chol: cholesterol | | | |

As the above first nucleic acid strand, the first nucleic acid strand prepared in Example 2 was used.

The second nucleic acid strand has a sequence complementary to the first nucleic acid strand and cholesterol bound to the 5' end thereof, as in the cholesterol-conjugated complementary strand RNA (Chol#1-cRNA(mMalatl)) of the second nucleic acid strand prepared in Example 2, but is entirely composed of DNA unlike the second nucleic acid strand in Example 2.

The preparation of the double-stranded nucleic acid complex agent was in accordance with the method described in Example 2. A double-stranded nucleic acid complex agent prepared using Toc#1-cDNA(mMalat1) as the

second nucleic acid strand is called "Toc#1DNA/DNA", and a double-stranded nucleic acid complex agent prepared using Chol#1-cDNA(mMalat1) is called "Chol#1DNA/DNA". Meanwhile, as the comparison reference for the double-stranded nucleic acid complex agent, a single-stranded antisense oligonucleotide (ASO) (ASO(mMalat1)) was used.

(2) *In vivo* experiment

The basic procedure was in accordance with the method described in Example 2. The double-stranded nucleic acid complex agent was administered to mice in a single dose at 50 mg/kg.

(3) Expression analysis

At 72 hours after the final administration, PBS was perfused into the mice, and then the mice were dissected to isolate separately each of the heart muscle, quadriceps muscle, and the diaphragm. RNA extraction from each of the obtained tissues, cDNA synthesis, quantitative RT-PCR, and evaluation of the expression level of malat1 mRNA were performed in accordance with Example 2.

(Results)

The results are shown in Figure 8. Figure 8 shows the inhibitory effects of the double-stranded nucleic acid complexes to which cholesterol is bound (Chol#1DNA/DNA) on the expression of the target malat1 gene in the heart muscle (Heart), quadriceps muscle (Quadriceps), and diaphragm (Diaphragm). The error bars indicate the respective standard errors.

Even when cholesterol was bound to the second nucleic acid strand composed solely of DNA, a significant inhibitory effect on the expression of the target gene in various skeletal muscles and the heart muscle was confirmed compared to the single-stranded control ASO(mMalat), or tocopherol.

<Example 5>

(Purpose)

The *in vivo* inhibitory effect of the double-stranded nucleic acid complex composed of a cholesterol-conjugated complementary strand composed solely of DNA as in Example 4 having various modification patterns of internucleoside linkages administered in a single dose on the expression of a target gene in the heart muscle and skeletal muscle was examined.

(Method)

(1) Preparation of nucleic acids

As a target gene, malat1 was selected. The names and base sequences of the first nucleic acid strand and the second nucleic acid strand constituting the double-stranded nucleic acid complex agent used in this Example are shown in Table 5.

[Table 5]

| | Name of oligonucleotide | Sequence (5'-3') | SEQ ID NO |
|---|---|---|---|
| First nucleic acid strand | ASO(mMalatl) | C*T*A*g*t*t*c*a*c*t*g*a*a*T*G*C | 9 |
| Second nucleic acid strand | Chol#1-cDNA(mMalat1) | Chol-g*c*a*ttcagtgaac*t*a*g | 29 |
| Second nucleic acid strand | Chol#1-cDNA(mMalat1)(PS) | Chol-g*c*a*t*t*c*a*g*t*g*a*a*c*t*a*g | 13 |
| Second nucleic acid strand | Chol#1-cDNA(mMalat1)(P0) | Chol-gcattcagtgaacuag | 13 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

In Chol#1-cDNA(mMalat1) (PS), phosphorothioate linkages are present between all the nucleosides, and in Chol#1-cDNA(mMalat1) (PO), phosphodiester linkages are present between all the nucleosides.

The preparation of the double-stranded nucleic acid complex agent was in accordance with the method described in Example 2. A double-stranded nucleic acid complex agent prepared using Chol#1-cDNA(mMalat1) as the second nucleic acid strand is called "Chol#1DNA/DNA", a double-stranded nucleic acid complex agent prepared using Chol#1-cDNA(mMalat1) (PS) is called "Chol#1DNA/DNA-PS", and a double-stranded nucleic acid complex agent prepared using Chol#1-cDNA(mMalat1) (PO) is called "Chol#1DNA/DNA-PO".

(2) *In vivo* experiment

The basic procedure was in accordance with the method described in Example 2. The double-stranded nucleic acid complex agent was administered to mice in a single dose at 50 mg/kg.

(3) Expression analysis

At the time point of 72 hours after the administration, PBS was perfused into the mice, and then the mice were dissected to isolate separately each of the heart muscle, diaphragm, and musculi dorsi proprii. RNA extraction from each of the obtained tissues, cDNA synthesis, quantitative RT-PCR, and evaluation of the expression level of malat1 mRNA were performed in accordance with Example 2.

(Results)

The results are shown in Figure 9. In a case where the second nucleic acid strand is composed solely of DNA, even when phosphorothioate linkages are present entirely between all the nucleosides, or phosphodiester linkages are present entirely between all the nucleosides, it was confirmed from Figure 9 that a remarkable inhibitory effect on the expression of the target gene was obtained in various skeletal muscles and the heart muscle as in Example 4.

<Example 6>

(Purpose)

The *in vivo* inhibitory effect of the double-stranded nucleic acid complex in which the second nucleic acid strand is composed of a cholesterol-conjugated complementary strand having various modification patterns of internucleoside linkages administered in a single dose in the heart muscle and skeletal muscle was examined.

(Method)

(1) Preparation of nucleic acids

As the target gene, malat1 was selected. The names and base sequences of the first nucleic acid strand and the second nucleic acid strand constituting the double-stranded nucleic acid complex agent used in this Example are shown in Table 6.

[Table 6]

| | Name of oligonucleotide | Sequence (5'-3') | SEQ ID NO |
|---|---|---|---|
| First nucleic acid strand | ASO(mMalatl) | C*T*A*g*t*t*c*a*c*t*g*a*a*T*G*C | 9 |
| Second nucleic acid strand | Chol#1-cRNA(mMalat1)(PO) | Chol-gcaUUCAGUGAACuag | 10 |
| Second nucleic acid strand | Chol#1-RNA(mMalat1)(5'PS) | Chol-g*c*a*UUCAGUGAACuag | 10 |
| Second nucleic acid strand | Chol#1-cRNA(mMalat1)(3'PS) | Chol-gcaUUCAGUGAAC*u*a*g | 10 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

Internucleoside linkages of Chol#1-cRNA(mMalat1)(PO) is composed solely of phosphorothioate linkages with no modification. Meanwhile, Chol#1-cRNA(mMalat1)(5'PS) and Chol#1-cRNA(mMalat1)(3'PS) have a configuration in which respectively 3 internucleoside linkages from the 5' end to which cholesterol is bound and 3' end, respectively, are phosphorothioate linkages (PS).

The preparation of the double-stranded nucleic acid complex agent was in accordance with the method described in Example 2. A double-stranded nucleic acid complex agent prepared using ASO(mMalatl) as the first nucleic acid strand and Chol#1-cRNA(mMalat1)(PO) as the second nucleic acid strand is called "Chol #1HDO(PO)", a double-stranded nucleic acid complex agent prepared using Chol#1-cRNA(mMalat1)(5'PS) is called "Chol#1HDO(5'PS)", and a double-stranded nucleic acid complex agent prepared using Chol#1-cRNA(mMalat1)(3'PS) is called "Chol#1HDO(3'PS)".

(2) *In vivo* experiment

The basic procedure was in accordance with the method described in Example 2. The double-stranded nucleic acid complex agent was administered to mice in a single dose at 50 mg/kg. Mice to which only PBS was administered were also produced as a negative control group.

(3) Expression analysis

At the time point of 72 hours after the administration, PBS was perfused into the mice, and then the mice were dissected to isolate separately each of the heart muscle, quadriceps muscle, diaphragm, and musculi dorsi proprii. RNA extraction from each of the obtained tissues, cDNA synthesis, quantitative RT-PCR, and evaluation of the expression level of malat1 mRNA were performed in accordance with Example 2.

(Results)

The results are shown in Figure 10. Figure 10 shows the inhibitory effects of the cholesterol-conjugated double-stranded nucleic acid complexes Chol#1HDO(PO), Chol#1HDO(5'PS), and Chol#1HDO(3'PS) on the expression of the target Malat1 gene in the heart muscle (Heart), quadriceps muscle (Quadriceps), diaphragm (Diaphragm), and musculi dorsi proprii (Back). The error bars indicate the respective standard errors.

All the double-stranded nucleic acid complexes significantly suppressed the expression of malat1 non-coding RNA. In particular, when there were modified internucleoside linkages from the 3' end such as in Chol#1HDO(3'PS) the inhibitory activity on the expression tended to increase.

<Example 7>

(Purpose)

The *in vivo* inhibitory effect on the expression of mRNA in a tissue by multiple doses of a double-stranded nucleic acid complex agent consisting of an antisense oligonucleotide targeting the malat1 gene and a tocopherol- or cholesterol-conjugated complementary strand was examined.

(Method)

(1) Preparation of nucleic acids

As double-stranded nucleic acid complex agents, Toc#1HDO(mMalat1) and Chol#1HDO(mMalat1) prepared in Example 2 were used, and ASO(mMalatl) was used as a single-stranded control ASO.

(2) *In vivo* experiment

As the mice to which the double-stranded nucleic acid complex agent or the like was administered, 6 to 7 week-old male C57BL/6 mice of body weight 20 g were used.

The double-stranded nucleic acid complex agent and the control ASO were intravenously injected through the tail vein into each mouse at 50 mg/kg per each dose. The administration was given for a total of four doses over four weeks. In addition, mice injected with PBS only in a single dose were also produced as a negative control group.

(3) Expression analysis

At the time point of 72 hours after the final administration, PBS was perfused into the mice, and then the mice were dissected to isolate the heart muscle, quadriceps muscle, diaphragm, and musculi dorsi proprii. Then mRNA was extracted from each tissue using a high-throughput fully automated nucleic acid extraction device MagNA Pure 96 (Roche

Life Science) according to the protocol. cDNA was synthesized according to the protocol attached to Transcriptor Universal cDNA Master (Roche Life Science). Quantitative RT-PCR was performed with TaqMan (Roche Life Science). As the primers used in the quantitative RT-PCR, the products designed and produced by Thermo Fisher Scientific based on various gene numbers were used. The PCR conditions (temperature and time) were 95°C for 15 sec, 60°C for 30 sec, and 72°C for 1 sec as 1 cycle, and 40 cycles were repeated. The amplified product thus obtained was quantified by quantitative RT-PCR, and based on the result, the expression level of mRNA (malat1)/the expression level of mRNA (ACTB; internal standard gene) were calculated respectively to obtain a relative expression level. The mean value and standard error of the relative expression levels were calculated. Further, the results of the individual groups were compared, and evaluated by t-test.

(Results)

The results are shown in Figure 11. Figure 11 shows the inhibitory effects of the tocopherol- or cholesterol-conjugated double-stranded nucleic acid complexes (Toc#1HDO(mMalat1), and Chol#1HDO(mMalat1), respectively) on the expression of the target Malat1 gene in the heart muscle (Heart), quadriceps muscle (Quadriceps), diaphragm (Diaphragm), and musculi dorsi proprii (Back). The error bars indicate the respective standard errors.

It was found that multiple doses of both Toc#1HDO(mMalat1) and Chol#1HDO(mMalat1) further enhanced the inhibitory effects on the expression of the target gene (malat1) in various skeletal muscles and the heart muscle.

<Example 8>

(Purpose)

The *in vivo* inhibitory effect on the expression of mRNA in a tissue by multiple doses of a double-stranded nucleic acid complex agent consisting of an antisense oligonucleotide targeting the DMPK gene and a cholesterol-conjugated complementary strand was examined.

(Method)

(1) Preparation of nucleic acids

As the double-stranded nucleic acid complex agent, Chol#1HDO(mDMPK) prepared in Example 3 was used.

(2) *In vivo* experiment

As the mice to which a double-stranded nucleic acid complex agent or the like was administered, 6 to 7 week-old male C57BL/6 mice of body weight 20 g were used.

The double-stranded nucleic acid complex agent was intravenously injected through the tail vein into each mouse at 50 mg/kg per each dose. The dose was given twice a week for a total of four doses. In addition, mice injected with PBS alone by a single-dose administration were also produced as a negative control group.

(3) Expression analysis

At the time point of 72 hours after the final administration, PBS was perfused into the mice, and then the mice were dissected to isolate separately each of the heart muscle, quadriceps muscle, diaphragm, and musculi dorsi proprii. Then RNA extraction from each of the obtained tissues, cDNA synthesis, quantitative RT-PCR, and evaluation of the expression level of DMPK mRNA were performed in accordance with Example 8.

(Results)

The results are shown in Figure 12. Figure 12 shows the inhibitory effect of the cholesterol-conjugated double-stranded nucleic acid complex (Chol#1HDO(mDMPK)) on the expression of the target DMPK gene in the heart muscle (Heart), quadriceps muscle (Quadriceps), diaphragm (Diaphragm), and musculi dorsi proprii (Back). The error bars indicate the respective standard errors.

It was found that multiple doses of Chol#1HDO(mDMPK) further enhanced the inhibitory effects on the expression of the target gene (DMPK) in various skeletal muscles and the heart muscle compared to the negative control (PBS only).

<Example 9>

(Purpose)

An experiment for evaluating the *in vivo* inhibitory effect over a long period of time on the expression of mRNA in a tissue in the heart muscle and skeletal muscle by a single-dose administration of a double-stranded nucleic acid complex agent was investigated.

(Method)

(1) Preparation of nucleic acids

As the double-stranded nucleic acid complex agent, Chol#1HDO(mMalat1) prepared in Example 2 was used.

(2) *In vivo* experiment

The double-stranded nucleic acid complex agent was administered in a single dose to mice in the same manner as in Example 2.

(3) Expression analysis

On day 3, day 7, day 14, day 28, day 56, and day 168 from the administration, PBS was perfused into the mice, and then the mice were dissected to isolate the heart muscle, quadriceps muscle, diaphragm, musculi dorsi proprii, liver, kidney, colon, and lung. Then RNA extraction from each of the obtained tissues, cDNA synthesis, quantitative RT-PCR, and evaluation of the expression level of malat1 non-coding RNA were performed according to the method described in Example 2.

(Results)

The results are shown in Figures 13 and 14.

Figure 13 shows graphs for the inhibitory effect of a cholesterol-conjugated double-stranded nucleic acid complex on the expression of a target gene (malat1) in the heart muscle and skeletal muscle. The vertical axis represents the relative expression level of malat1 non-coding RNA, and the horizontal axis represents time (days) after the administration. The error bars indicate the respective standard errors. Figure 13a shows results in the heart muscle (Heart), Figure 13b in the musculi dorsi proprii (Back), Figure 13c in the quadriceps muscle (Quadriceps), and Figure 13d in the diaphragm (Diaphragm).

Figure 14 shows the relative expression levels of the malat1 non-coding RNA in each tissue at 8 weeks (56 days) after the administration in comparison to the negative control (PBS only).

It was found from Figures 13 and 14 that Chol#1HDO(mMalat1) significantly suppressed the expression of the malat1 non-coding RNA in the heart muscle, quadriceps muscle, diaphragm, and musculi dorsi proprii over a long period of time compared to the negative control. In addition, it was also found that the effect persisted in the skeletal muscle even after 8 weeks of the administration.

<Example 10>

(Purpose)

The *in vivo* inhibitory effect by single-dose administration on the expression of mRNA in a tissue in the heart muscle and skeletal muscle when a double-stranded nucleic acid complex was administered at various doses was examined.

(Method)

(1) Preparation of nucleic acids

The double-stranded nucleic acid complex agent Chol#1HDO(mMalat1) prepared in Example 2 was used.

(2) *In vivo* experiment

The double-stranded nucleic acid complex agent Chol#1HDO (mMalat1) was injected intravenously into mice through the tail vein in a single-dose administration at 12.5 mg/kg, 25.0 mg/kg, 50 mg/kg, or 75 mg/kg.

(3) Expression analysis

At 72 hours after the administration, PBS was perfused into the mice, and then the mice were dissected to isolate separately each of the heart muscle, quadriceps muscle, diaphragm, and musculi dorsi proprii.

RNA extraction from each of the obtained tissues, cDNA synthesis, quantitative RT-PCR, and evaluation of the expression level of malat1 mRNA were performed in accordance with Example 2.

(Results)

The results are shown in Figure 15. It was found that Chol#1HDO(mMalat1) could dose-dependently suppress the expression of the malat1 non-coding RNA in any of the heart muscle and the skeletal muscle.

<Example 11>

(Purpose)

The *in vivo* inhibitory effect by single-dose administration of a double-stranded nucleic acid complex agent in which cholesterol and a saturated fatty acid group are bound to the second nucleic acid strand on the expression of mRNA in a tissue in the heart muscle and skeletal muscle was examined.

(Method)

(1) Preparation of nucleic acids

As the target gene, malat1 was selected as in Example 2. As the first nucleic acid strand constituting the double-stranded nucleic acid complex agent used in this Example, the first nucleic acid strand described in Example 2, namely the 16-mer single-stranded LNA/DNA gapmer ASO(mMalatl) targeting the malat1 non-coding RNA, which was the transcription product of the murine malat1 gene, was used. Meanwhile, the second nucleic acid strand was a strand which had a sequence complementary to the first nucleic acid strand, and to which cholesterol was bound at the 5' end or 3' end, having a configuration that a linker (C6) consisting of a C6 saturated fatty acid group (hexyl group) is present between cholesterol and an end of the second nucleic acid strand. The names of the respective second nucleic acid strands are shown in Table 7.

[Table 7]

| | Name of oligonucleotide | Sequence (5'-3') | SEQ ID NO |
|---|---|---|---|
| First nucleic acid strand | ASO(mMalatl) | C*T*A*g*t*t*c*a*c*t*g*a*a*T*G*C | 9 |
| Second nucleic acid strand | 5'Chol(C6)-cRNA (mMalat1) | Chol (C6)-g*c*a*UUCAGUGAAC*u*a*g | 10 |
| Second nucleic acid strand | 3'Chol(C6)-cRNA (mMalat1) | g*c*a*UUCAGUGAAC*u*a*g-Chol (C6) | 10 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

A double-stranded nucleic acid complex agent of the present invention was prepared by annealing the first nucleic acid strand above with either 5'Chol(C6)-cRNA(mMalat1) or 3'Chol(C6)-cRNA(mMalat1) of the second nucleic acid strand. The specific preparation method was as in Example 2. The prepared double-stranded nucleic acid complex agents are referred to as 5'Chol(C6)HDO and 3'Chol(C6)HDO.

(2) *In vivo* experiment

The basic procedure was in accordance with the method described in Example 2. The double-stranded nucleic acid complex agent was administered to the mice in a single dose at 50 mg/kg.

(3) Expression analysis

At the time point of 72 hours after the administration, PBS was perfused into the mice, and then the mice were dissected to isolate the heart muscle (Heart), quadriceps muscle (Quadriceps), diaphragm (Diaphragm), and musculi dorsi proprii (Back). RNA extraction from each of the obtained tissues, cDNA synthesis, quantitative RT-PCR, and evaluation of the expression level of malat1 were performed in accordance with the methods described in Example 2.

(Results)

The results are shown in Figure 16. Even when a C6 linker was present between an end of the second nucleic acid strand and cholesterol, the *in vivo* inhibitory effect of the double-stranded nucleic acid complex agent by single-dose administration on the expression of mRNA in a tissue in the heart muscle and skeletal muscle was confirmed. The effect was stronger when it was bound to the 5' end.

<Example 12>

(Purpose)

The *in vivo* inhibitory effects on the mRNA expression in a tissue by double-stranded nucleic acid complex agents having different lengths with respect to the target gene were examined.

(Method)

(1) Preparation of nucleic acids

As the target gene, malat1 was selected as in Example 2. As the first nucleic acid strand composing the double-stranded nucleic acid complex agent used in this Example, the first nucleic acid strands described in Example 2, namely the 13-mer and 16-mer single-stranded LNA/DNA gapmer ASO(mMalatl) targeting the malat1 non-coding RNA, which was the transcription product of the murine malat1 gene, were used. The second nucleic acid strands were a strand complementary to each of the first nucleic acid strands, and had a configuration in which cholesterol was bound to the 5' end. The names and sequences of the first nucleic acid strands and the second nucleic acid strands used in this Example are shown in Table 8.

[Table 8]

| | Name of oligonucleotide | Sequence (5'-3') | SEQ ID NO |
|---|---|---|---|
| First nucleic acid strand | ASO(mMalat1)16mer | C*T*A*g*t*t*c*a*c*t*g*a*a*T*G*C | 9 |
| Second nucleic acid strand | Chol#1-cRNA(mMalat1) 16mer | Chol-g*c*a*UUCAGUGAAC*u*a*g | 30 |
| First nucleic acid strand | ASO(mMalat1)13mer | G*T*T*c*a*c*t*g*a*a*t*G*C | 14 |
| Second nucleic acid strand | Chol#1-cRNA(mMalat1) 13mer | Chol-g*c*a*UUCAGUGA*a*c | 15 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

The prepared 16mer and 13mer double-stranded nucleic acid complex agents are referred to as "16mer Chol-HDO" and "13mer Chol-HDO", respectively.

(2) *In vivo* experiment

The basic procedure was in accordance with the method described in Example 2. The double-stranded nucleic acid complex agent was administered to the mice in a single dose at 50 mg/kg.

(3) Expression analysis

At the time point of 72 hours after the administration, PBS was perfused into the mice, and then the mice were dissected to isolate the heart muscle (Heart), quadriceps muscle (Quadriceps), diaphragm (Diaphragm), and musculi dorsi proprii (Back). RNA extraction from each of the obtained tissues, cDNA synthesis, quantitative RT-PCR, and evaluation of the expression level of malat1 were performed in accordance with the methods described in Example 2.

(Results)

The results are shown in Figure 17. Figure 17 shows a graph for the inhibitory effects of double-stranded nucleic acid complex agents having different lengths with respect to the target gene, on expression of the target gene (malat1) in the heart and skeletal muscles throughout the body. It shows the results in the heart muscle, quadriceps muscle, diaphragm, and musculi dorsi proprii. The error bars indicate the standard errors.

Significant inhibitory effects on the expression were confirmed for both 13mer Chol-HDO and 16mer Chol-HDO compared to the negative control PBS. Especially, 13mer Chol-HDO exhibited remarkable effects.

<Example 13>

(Purpose)

The long-term *in vivo* inhibitory effect on the expression by a double-stranded nucleic acid complex administered subcutaneously in a single dose was examined.

(Method)

(1) Preparation of nucleic acids

The double-stranded nucleic acid complex agent Chol#1 HDO (malat1) prepared in Example 2 was used.

(2) *In vivo* experiment

The double-stranded nucleic acid complex agent was administered subcutaneously in a single dose to mice at 50 mg/kg in this Example although the basic procedures were in accordance with the method described in Example 7.

(3) Expression analysis

At the time points of day 7, day 14, and day 28 after the subcutaneous administration, PBS was perfused into the mice, and then the mice were dissected to isolate the heart muscle (Heart), quadriceps muscle (Quadriceps), and diaphragm (Diaphragm). RNA extraction from each of the obtained tissues, cDNA synthesis, quantitative RT-PCR, and evaluation of the expression level of malat1 were performed in accordance with the method described in Example 2.

(Results)

The results are shown in Figure 18. Figure 18 show graphs for the inhibitory effects on the expression of the target gene (malat1) in the heart muscle, quadriceps muscle, and diaphragm when the double-stranded nucleic acid complex agent is subcutaneously administered. The error bars indicate the standard errors.

It was demonstrated that the double-stranded nucleic acid complex agent of the present invention could maintain the inhibitory effect on the expression of the target gene over a long period of time not only by intravenous injection but also by subcutaneous administration.

<Example 14>

(Purpose)

The toxicity of the cholesterol-conjugated double-stranded nucleic acid complex of the present invention and cholesterol-conjugated ASO administered to the living body was examined.

(Method)

(1) Preparation of nucleic acids

The name and base sequence of the first nucleic acid strands constituting the single-stranded nucleic acid complexes used in this Example are shown in Table 9.

[Table 9]

| | Name of oligonucleotide | Sequence (5'-3') | SEQ ID NO |
|---|---|---|---|
| First nucleic acid strand | ASO(mMalat1) | C*T*A*g*t*t*c*a*c*t*g*a*a*T*G*C | 9 |
| First nucleic acid strand | 5'-Chol-DNA-ASO (mMalat1) | Chol-cttcC*T*A*g*t*t*c*a*c*t*g*a*a*T*G*C | 16 |
| First nucleic acid strand | 3'-Chol-ASO(mMalat1) | C*T*A*g*t*t*c*a*c*t*g*a*a*T*G*C-Chol | 9 |
| First nucleic acid strand | 3'-Chol-DNA-ASO (mMalat1) | C*T*A*g*t*t*c*a*c*t*g*a*a*T*G*Ccttc-Chol | 17 |
| Second nucleic acid strand | Chol#1-cRNA(mMalat1) | chol-g*c*a*UUCAGUGAAC*u*a*g | 10 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

The above first nucleic acid strand has a configuration in which cholesterol is bound to the 5' end or 3' end of the ASO(mMalat1) described in the Example above and targeting the murine malat1 gene, via a DNA linker (ccttc) or without the linker.

(2) *In vivo* experiment

The basic procedure was in accordance with the method described in Example 2. A double-stranded nucleic acid complex agent was administered to mice subcutaneously or intravenously in a single dose at 50 mg/kg.

(3) Expression analysis

Blood samples were taken from the individual mice 72 hours after the administration, and the measurement of blood counts was outsourced to LSI Medience.

(Results)

The results are shown in Figures 19 and 20. Figure 19 shows a graph for the inhibitory effects of the single-stranded nucleic acid complex agent described above, the double-stranded nucleic acid complex agent Chol#1HDO(mMalat1) as a positive control, and PBS as a negative control on the expression of the malat1 gene in the heart muscle (Heart), quadriceps muscle (Quadriceps), diaphragm (Diaphragm), and musculi dorsi proprii (Back). The s.c. stands for subcutaneous administration. The error bars indicate the respective standard errors. For both Chol-HDO and Chol-HDO s.c., a remarkable inhibitory effect on the expression of the target gene (malat1 gene) was confirmed compared to the negative control of PBS in any of the heart muscle, quadriceps muscle, diaphragm, and musculi dorsi proprii. Further, even compared to the single-stranded nucleic acid complex (3'-Chol-DNA-ASO(mMalat1)), the double-stranded nucleic acid complex of the present invention (Chol-HDO(mMalatl)) significantly suppressed the expression of the target gene. In particular, when the single-stranded nucleic acid complex was administered subcutaneously (3'-Chol-DNA-ASO(mMalat1) s.c.), the inhibition of the expression of the target gene was not confirmed. However, when the

double-stranded nucleic acid complex of the present invention was administered subcutaneously (Chol-HDO(mMalatl) s.c.), a strong inhibitory effect was observed.

Figure 20 shows a graph for the platelet count in the blood after the administration of each nucleic acid complex, in which s.c. stands for subcutaneous administration, and i.v. stands for intravenous administration. With respect to the double-stranded nucleic acid complex of the present invention (5'-Chol-HDO(mMalat1)i.v., and 5'-Chol-HDO(mMalat1)s.c.), no decrease in the platelet count was observed compared to a single-stranded nucleic acid complex to which cholesterol was bound at an end (5'-Chol-DNA-ASO(mMalat1), and 3'-Chol-DNA-ASO(mMalat1)). This result suggests that the double-stranded nucleic acid complex of the present invention is less toxic to the living body compared to the single-stranded nucleic acid complex.

<Example 15>

(Purpose)

The objective is to evaluate the exon skipping effect and dystrophin expression in the muscles throughout the body by multiple dose administration of a double-stranded nucleic acid complex consisting of an antisense oligonucleotide (morpholino oligomer) for which the boundary region of exon 23/intron 23 of mdx mice (Duchenne muscular dystrophy model mice) is the target for exon skipping, and a tocopherol-conjugated complementary strand.

An experiment for evaluating the *in vivo* effect for induction of exon skipping and the expression of a dystrophin protein in a tissue by the double-stranded nucleic acid agent consisting of an antisense oligonucleotide targeting exon 23/intron 23 of the murine dystrophin gene, and a tocopherol-conjugated complementary strand was conducted.

(Method)

(1) Preparation of nucleic acids

The double-stranded nucleic acid complex agent was compared to the conventional single-stranded antisense oligonucleotide (ASO) serving as a control. The control (ASO) was a 25 mer single-stranded morpholino targeting the exon 23/intron 23 of the pre-mRNA of the murine dystrophin gene. This 25-mer ASO is composed entirely of morpholino. This morpholino has a base sequence complementary to position 83803536 to 83803512 of the murine dystrophin pre-mRNA (GenBank Accession number: NC_000086.7). A tocopherol-conjugated heteroduplex oligonucleotide (Toc-HDO), which was a double-stranded nucleic acid agent, was prepared by annealing the morpholino (first strand) with tocopherol-conjugated Toc#1-cRNA (mDystrophin). The first strand and the second strand were mixed in equimolar amounts, and the solution was heated at 95°C for 5 min, then cooled to 37°C and held for 1 hour. In this way the nucleic acid strands were annealed to form the above double-stranded nucleic acid agent. The annealed nucleic acid was stored at 4°C or on ice. The prepared double-stranded nucleic acid agent is referred to as Toc#1HDO.

The names and base sequences of the first strand and second strand used in this Example are shown in Table 10.

[Table 10]

| | Name of oligonucleotide | Sequence (5'-3') | SEQ ID NO |
|---|---|---|---|
| First nucleic acid strand | PMO(mDystrophin) | *ggccaaacctcggcttacctgaaat* | 18 |
| Second nucleic acid strand | Toc#1-cRNA (mDystrophin) | Toc-a*u*u*UCAGGUAAGCCGAGGUUUG*g*c*c | 19 |
| Italic lowercase letter: morpholino; Uppercase letter: RNA; Underlined lowercase letter: 2'-O-methyl RNA; *: phosphorothioate linkage (PS linkage); Toc: tocopherol | | | |

(2) *In vivo* experiment

The mice were 6 to 7 week-old male mdx mice of body weight 20 g. All experiments using mice were performed with n=4. Toc#1HDO was intravenously injected into mice at a dose of 100 mg/kg through a vein. The administration was carried out once a week for a total of five times. In addition, mice injected with PBS only or PMO instead of Toc#1HDO were also produced as negative control groups.

(3) Expression analysis

Two weeks after the final administration, PBS was perfused into the mdx mice, and then the mice were dissected to isolate the heart muscle, quadriceps muscle, and diaphragm. Then, mRNA was extracted from each tissue with ISOGEN. For 200 ng of the extracted total RNA, One-Step RT-PCR was performed using a Qiagen One Step RT-PCR Kit (Qiagen). A reaction solution was prepared according to the protocol attached to the kit. As a thermal cycler, LifeECO (manufactured by Bioer Technology) was used. According to the applied RT-PCR program, a reverse transcription reaction was performed at 42°C for 30 min, then thermal denaturation was performed at 95°C for 15 min, then the cycle of [94°C for 30 sec; 60°C for 30 sec; and 72°C for 60 sec] was repeated to conduct 35 cycles of PCR amplification reaction, and the final elongation reaction was performed at 72 °C for 7 min.

The base sequences of the forward (Fw) primer and reverse (Rv) primer used for the RT-PCR were as follows.

Fw primer: 5'-ATCCAGCAGTCAGAAAGCAAA-3' (SEQ ID NO:20)
Rv primer: 5'-CAGCCATCCATTTCTGTAAGG-3' (SEQ ID NO:21)

For 1 μL of the reaction product of the above RT-PCR, an analysis was performed using an Agilent DNA1000 kit and the Bioanalyzer 2100 (manufactured by Agilent Technologies). The electrophoresis diagram of the Bioanalyzer 2100 is shown in Figure 21. The polynucleotide amount "A" of the band where exon 23 was skipped (arrow) and the polynucleotide amount "B" of the band where exon 23 was not skipped (arrowhead) were measured.

In mdx mice, there is an abnormal stop codon in exon 23. Therefore, in the mRNA (B) in which exon 23 is not skipped, the subsequent exons are not translated, and normal dystrophin is not expressed. On the other hand, in the mRNA (A), in which exon 23 is skipped, although the mRNA becomes shorter because exon 23 is not included, normal dystrophin is expressed. Based on the measurements of "A" and "B", the skipping efficiency was determined according to the following formula.

$$\text{Skipping efficiency (\%)} = A/(A + B) \times 100$$

A muscle sample was sliced on a cryostat (Leica CM3050 S) into 25 μM × 40 pieces and then dissolved in 150 μL of a buffer (125 mM Tris-HCl pH 6.4, 10% glycerol, 4% SDS, 4 M urea, 10% mercaptoethanol, 0.005% BPB, $H_2O$).

The solution was then sonicated, heated at 100°C × 3 min, centrifuged at 1000 g × 5 min, and the supernatant was recovered.

Proteins were electrophoresed using a 4-15% gradient 10-well (Mini-PROTEAN® TGX Precast Gels). After transferred to a membrane, a rabbit anti-dystrophin antibody (Abcam ab15277-rabbit) was used as a primary antibody and an HRP-conjugated goat anti-rabbit IgG antibody (The Jackson Laboratory) was used as a secondary antibody to perform Western blotting. Finally, the luminescence was evaluated with a SuperSignal West Dura Extended Duration Substrate (Thermo Fisher) on a ChemiDoc Imaging System (Bio-Rad Laboratories, Inc.).

Subsequently, a 10 μM-thick thin slice was prepared by sectioning the muscle sample on a cryostat (Leica CM3050 S) and placed on a cover slip (MAS-02, Matsunami Glass Ind., Ltd.). The glass slide was dipped in acetone cooled at -30°C for 10 min to be cooled down. TBS was poured to soak the sample for adjusting to TBS. Then after blocking with 5% goat serum, the sample was treated with the primary antibody solution of the rabbit anti-dystrophin antibody (Abcam ab15277-rabbit) (1/400)/5% goat serum/0.25% Tween 20/TBS, and incubated overnight. The primary antibody solution was washed off three times with 0.25% Tween 20/TBS, and then the sample was incubated for 1 hour with the secondary antibody solution of the goat anti-rabbit IgG antibody (Invitrogen; Alexa Fluor 568) (1/1000)/5% goat serum/0.25% Tween 20/TBS. The secondary antibody solution was washed off three times with 0.25% Tween 20/TBS, and the specimen was encapsulated in VECTASHIELD, and then observed under a microscope BZ-X700 (Keyence Corporation), and the immunostaining diagram was photographed.

(Results)

The results of the skipping efficiency are shown in Figure 22, the Western blottings are shown in Figure 23, and the immunostainings are shown in Figure 24. As seen in Figure 22, almost no exon skipping was observed with the single-stranded nucleic acid complex agent (PMO) in (a) the heart (Heart), but exon skipping of 27% or more was observed with the double-stranded nucleic acid complex agent (Toc-HDO) of the present invention. Also in other skeletal muscles as shown in Figures 22(b) to (f), with the double-stranded nucleic acid complex agent (Toc-HDO) of the present invention, exon skipping two to four times as much as that occurred with the single-stranded nucleic acid complex agent (PMO) was observed. The Western blotting in Figure 23 also showed that more dystrophin was expressed with the double-stranded nucleic acid complex agent (Toc-HDO) than with the single-stranded nucleic acid complex agent (PMO)

in the (a) heart and (b) quadriceps muscle. In addition, in the immunostainings in Figure 24 for the heart (a) and (b), and the quadriceps muscle (c) and (d), it was observed that more dystrophin was expressed with the double-stranded nucleic acid complex agent (Toc-HDO) (b), (d) than with the single-stranded nucleic acid complex agent (PMO) (a), (c) as in Figure 23.

<Example 16>

(Purpose)

The objective is to evaluate the exon skipping effect and dystrophin expression in the muscles throughout the body by single dose administration of a double-stranded nucleic acid complex (Chol-HDO) consisting of an antisense oligonucleotide (morpholino oligomer) for which the boundary region of exon 23/intron 23 of mdx mice is the target for exon skipping, and a cholesterol-conjugated complementary strand. The basic evaluation method is the same as in Example 15.

(Method)

(1) Preparation of nucleic acids

The first strand of the double-stranded nucleic acid complex agent used was that prepared in Example 15. By annealing the first strand with cholesterol conjugated Chol#1-cRNA(mDystrophin), a cholesterol-conjugated heteroduplex oligonucleotide (Chol-HDO) was prepared as the double-stranded nucleic acid agent. The first strand and the second strand were mixed in equimolar amounts, and the solution was heated at 95°C for 5 min, then cooled to 37°C and held for 1 hour to anneal the nucleic acid strands thereby preparing the aforedescribed double-stranded nucleic acid agent. The annealed nucleic acid was stored at 4°C or on ice. The prepared double-stranded nucleic acid agent is referred to as Chol#1HDO.

The names and base sequences of the first nucleic acid strand and the second nucleic acid strand used in this Example are shown in Table 11.

[Table 11]

| | Name of oligonucleotide | Sequence (5'-3') | SEQ ID NO |
|---|---|---|---|
| First nucleic acid strand | PMO(mDystrophin) | *ggccaaacctcggcttacctgaaat* | 18 |
| Second nucleic acid strand | Chol#1-cRNA (mDystrophin) | Chol-a*u*u*UCAGGUAAGCCGAGGUUUG*g*c*c | 19 |
| Italic lowercase letter: morpholino; Uppercase letter: RNA; Underlined lowercase letter: 2'-O-methyl RNA; *: phosphorothioate linkage (PS linkage); Chol: cholesterol | | | |

(2) *In vivo* experiment

As for mice, 6 to 7 week-old male mdx mice of body weight 20 g were used. All experiments were performed with n=1. Chol#1HDO was intravenously injected into the mouse at a dose of 100 mg/kg via the orbital vein. The number of doses was a single dose. Mice injected with PBS, PMO only, or Toc-HDO instead of Chol#1HDO were produced as negative control groups as references for comparison.

(3) Expression analysis

Expression analysis was performed in accordance with the method described in Example 15.

(Results)

The results are shown in Figure 25. There was no difference in the effect between Chol-HDO and Toc-HDO in the heart (a), but in the skeletal muscles (b) to (e), Chol-HDO exhibited a higher exon skipping effect than Toc-PMO.

<Example 17>

(Purpose)

The objective is to evaluate the exon skipping effect and dystrophin expression in the muscles throughout the body by single dose subcutaneous administration of a double-stranded nucleic acid complex consisting of an antisense oligonucleotide (mixmer oligomer) for which the boundary region of exon 23/intron 23 of mdx mice is the target for exon skipping, and a tocopherol-conjugated complementary strand. The basic evaluation method is the same as in Example 15.

(Method)

(1) Preparation of nucleic acids

The double-stranded nucleic acid complex agent was compared to the conventional single-stranded antisense oligonucleotide (ASO) serving as a control. The control (ASO) was a 13 mer single-stranded mixmer targeting the exon 23/intron 23 of the pre-mRNA of the murine dystrophin gene. This ASO is composed of LNA and DNA, and has a base sequence complementary to the murine dystrophin pre-mRNA (GenBank Accession number: NC_000086.7). A tocopherol-conjugated heteroduplex oligonucleotide (Toc-HDO) which was a double-stranded nucleic acid agent, was prepared by annealing the above mixmer as the first strand with tocopherol-conjugated Toc#1-cRNA (mDystrophin). The first strand and the second strand were mixed in equimolar amounts, and the solution was heated at 95°C for 5 min, then cooled to 37°C and held for 1 hour to anneal the nucleic acid strands thereby preparing the above double-stranded nucleic acid agent. The annealed nucleic acid was stored at 4°C or on ice. The prepared double-stranded nucleic acid agent is referred to as Toc#2HDO.

The names and base sequences of the first nucleic acid strand and second nucleic acid strand used in this Example are shown in Table 12.

[Table 12]

| | Name of oligonucleotide | Sequence (5'-3') | SEQ ID NO |
|---|---|---|---|
| First nucleic acid strand | Mixmer(mDystrophin) | a*C*c*T*c*G*g*C*t*T*a*C*c | 22 |
| Second nucleic acid strand | Toc#1-cRNA(mDystrophin) | Toc-g*g*t*AAGCCGA*g*g*t | 23 |

Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Toc: tocopherol

(2) *In vivo* experiment

As for mice, 6 to 7 week-old male mdx mice of body weight 20 g were used. All experiments were performed with n=2. Toc#2HDO was subcutaneously injected into the mice at a dose of 100 mg/kg. The number of administration was single. Mice injected with PBS only, or a single-stranded mixmer instead of Toc#2HDO were also produced as negative control groups.

(3) Expression analysis

Expression analysis was performed in accordance with the method described in Example 17.

(Results)

The results are shown in Figure 26. It was found that Toc#2HDO (Toc-Mixmer), which was a double-stranded mixmer, exhibited a higher exon skipping effect than a single-stranded mixmer (Mixmer) not only in the heart (a), but also in the skeletal muscles (b) to (e).

<Example 18>

(Purpose)

The *in vivo* inhibitory effect on the mRNA expression in a tissue was examined by administering in a single

dose the double-stranded nucleic acid complex agent (Chol#1HDO(mMalat1)) targeting the malat1 gene and consisting of a double-stranded nucleic acid complex in which cholesterol was bound to the second nucleic acid strand, or a nucleic acid molecule obtained by self-annealing a single-stranded nucleic acid in which the first nucleic acid strand and a cholesterol-conjugated complementary strand are linked by an RNA linker.

(Method)

(1) Preparation of nucleic acids

As the target gene malat1 was selected. As the double-stranded nucleic acid complex agent, Chol#1HDO(mMalat1) prepared in Example 2 was used. The name and base sequence of the single-stranded nucleic acid in which the first nucleic acid strand (ASO) and the complementary strand (cholesterol-conjugated cRNA) Chol#1-cRNA(mMalat1) are linked by an RNA linker are shown in Table 13. Chol#1sHDO(mMalat1) (or CholsHDO) was prepared by self-annealing the single-stranded nucleic acid as illustrated in Figure 1c. Specifically, a Chol#1-sHDO(mMalat1) solution was heated at 95°C for 5 min, then cooled to 37°C and held for 1 hour, thereby preparing the nucleic acid strand by self-annealing. The annealed nucleic acid was stored at room temperature, 4°C, or on ice. The prepared nucleic acid is referred to as "Chol#1sHDO(mMalat1)".

[Table 13]

| | Name of oligonucleotide | Sequence (5'-3') | SEQ ID NO |
|---|---|---|---|
| First nucleic acid strand | ASO(mMalat1) | C*T*A*g*t*t*c*a*c*t*g*a*a*T*G*C | 9 |
| Second nucleic acid strand | Chol#1-cRNA (mMalat1) | Chol-g*c*a*UUCAGUGAAC*u*a*g | 10 |
| Single-stranded nucleic acid strand | Chol#1-sHDO (mMalat1) | Chol-g*c*a*UUCAGUGAAC*u*a*gUUCAAGAGA C*T*A*g*t*t*c*a*c*t*g*a*a*T*G*C | 24 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

(2) *In vivo* experiment

The basic procedure was in accordance with the method described in Example 2. The prepared nucleic acid was administered to mice in a single dose at 50 mg/kg in terms of ASO.

(3) Expression analysis

At the time point of 72 hours from the administration, PBS was perfused into the mice, and then the mice were dissected to isolate separately each of the heart muscle, quadriceps muscle, diaphragm, and musculi dorsi proprii. RNA extraction from each of the obtained tissues, cDNA synthesis, quantitative RT-PCR, and evaluation of the expression level of malat1 mRNA were performed in accordance with Example 2.

(Results)

The results are shown in Figure 27. It was confirmed from Figure 27 that a remarkable inhibitory effect on the expression of the target gene was obtained in various skeletal muscles and the heart muscle comparable to the double-stranded nucleic acid complex agent consisting of a cholesterol-conjugated complementary strand, even in a case of a single-stranded nucleic acid in which the first nucleic acid strand and the complementary strand (cholesterol-conjugated) were linked by an RNA linker.

<Example 19>

(Purpose)

The *in vivo* inhibitory effect on the mRNA expression in a tissue was examined by administering in a single

dose the double-stranded nucleic acid complex agent (Chol#1HDO(mMalat1)) targeting the malat1 gene and consisting of a double-stranded nucleic acid complex in which cholesterol was bound to the second nucleic acid strand, or a nucleic acid (3'Chol(TEG)HDO(mMalat1)) having a configuration in which the second nucleic acid strand had a sequence complementary to the first nucleic acid strand, and cholesterol was bound at the 3' end, and further a linker (TEG) consisting of tetraethylene glycol was present between the cholesterol and the end of the second nucleic acid strand.

(1) Preparation of nucleic acids

As the target gene, malat1 was selected as in Example 2. The double-stranded nucleic acid complex agent (Chol#1HDO(mMalat1)) used in Example 2, and as the first nucleic acid strand, the first nucleic acid strand described in Example 2, namely the 16-mer single-stranded LNA/DNA gapmer ASO(mMalat1) targeting the malat1 non-coding RNA, which was the transcription product of the murine malat1 gene, were used. Meanwhile, the double-stranded nucleic acid complex agent of the present invention was prepared by annealing the above first nucleic acid strand with the second nucleic acid strand 3'Chol(TEG)-cRNA(mMalat1). The specific preparation method was in accordance with Example 2. The prepared double-stranded nucleic acid complex agent is referred to as "3'Chol(TEG)HDO". The respective names and base sequences of the first and second nucleic acid strands are as shown in Table 14.

[Table 14]

| | Name of oligonucleotide | Sequence (5'-3') | SEQ ID NO |
|---|---|---|---|
| First nucleic acid strand | ASO(mMalat1)16mer | C*T*A*g*t*t*c*a*c*t*g*a*a*T*G*C | 9 |
| Second nucleic acid strand | Chol#1-cRNA(mMalat1)16mer | Chol-g*c*a*UUCAGUGAAC*u*a*g | 10 |
| Second nucleic acid strand | Chol(TEG)#1-cRNA(mMalat1) 16mer | g*c*a*UUCAGUGAAC*u*a*g-Chol(TEG) | 10 |
| Underlined uppercase letter: LNA (C stands for 5-methylcytosine LNA); Lowercase letter: DNA; Uppercase letter: RNA; Underlined lowercase letter: 2'-O-methyl RNA; *: phosphorothioate linkage (PS linkage); Chol: cholesterol | | | |

(2) *In vivo* experiment

The basic procedure was in accordance with the method described in Example 2. The double-stranded nucleic acid complex agent was administered to mice in a single dose at 50 mg/kg.

(3) Expression analysis

At the time point of 72 hours after the administration, PBS was perfused into the mice, and then the mice were dissected to isolate the heart muscle (Heart), quadriceps muscle (Quadriceps), diaphragm (Diaphragm), and musculi dorsi proprii (Back). RNA extraction from each of the obtained tissues, cDNA synthesis, quantitative RT-PCR, and evaluation of the expression level of malat1 were performed in accordance with the methods described in Example 2.

(Results)

The results are shown in Figure 28. The effect is compromised when it is bound to the 3' end side, therefore binding to the 5' end side is important.

<Example 20>

(Purpose)

The objective is to evaluate the effect on the motor ability of multiple doses administration of the double-stranded nucleic acid complex (Chol-HDO or Toc-HDO) consisting of an antisense oligonucleotide (morpholino oligomer) targeting the exon 23/intron 23 boundary region in mdx mice for exon skipping and a cholesterol-conjugated complementary strand or a tocopherol-conjugated complementary strand.

(Method)

(1) Preparation of nucleic acids

As the first strand of the double-stranded nucleic acid complex agent used, the first strand prepared in Example 15 was used. By annealing the first strand with cholesterol-conjugated Chol#1-cRNA(mDystrophin), or tocopherol-conjugated Toc#1-cRNA(mDystrophin), a cholesterol-conjugated heteroduplex oligonucleotide (Chol-HDO) and a tocopherol-conjugated heteroduplex oligonucleotide (Toc-HDO) were prepared as the double-stranded nucleic acid agent. The first strand and the second strand were mixed in equimolar amounts, and the solution was heated at 95°C for 5 min, then cooled to 37°C and held for 1 hour to anneal the nucleic acid strands thereby preparing the above double-stranded nucleic acid agent. The annealed nucleic acid was stored at 4°C or on ice. The prepared double-stranded nucleic acid agents are referred to as Chol#1HDO and Toc#1HDO.

The names and base sequences of the first nucleic acid strand and the second nucleic acid strand used in this Example are shown in Table 15.

[Table 15]

| | Name of oligonucleotide | Sequence (5'-3') | SEQ ID NO |
|---|---|---|---|
| First nucleic acid strand | PMO(mDystrophin) | *ggccaaacctcggcttacctgaaat* | 18 |
| Second nucleic acid strand | Chol#1-cRNA (mDystrophin) | Chol-a*u*u*UCAGGUAAGCCGAGGUUUG*g*c*c | 19 |
| Second nucleic acid strand | Toc#1-cRNA (mDystrophin) | Toc-a*u*u*UCAGGUAAGCCGAGGUUUG*g*c*c | 19 |
| Italic lowercase letter: morpholino; Uppercase letter: RNA; Underlined lowercase letter: 2'-O-methyl RNA; *: phosphorothioate linkage (PS linkage); Chol: cholesterol; Toc: tocopherol | | | |

(2) *In vivo* experiment

Chol#1HDO or Toc#1HDO was intravenously injected into mice through a vein at a dose of 100 mg/kg. The administration was carried out once a week for a total of five doses. In addition, mice injected with PBS only or PMO instead of Chol#1HDO and Toc#1HDO were also produced as a negative control group, and B10 (normal mice) were designated as a positive control.

(3) Exercise tolerance test

One week or more after the fifth and final administration, an exercise tolerance test was performed. The exercise tolerance test was performed on a treadmill for both rats and mice (belt-type forced running device) (MK-680S, Muromachi Kikai Co., Ltd.) under conditions with electrical stimulation and without inclination. The speed was 5 m/min for 5 min from the test start, and then increased by 1 m/min every minute, and the running duration was measured.

(Results)

The results are shown in Figure 29. For the mdx mice (n=3) administered with the single-stranded nucleic acid complex agent (PMO), the running duration was slightly increased compared to the negative control mdx mice (mdx, n=6) administered with PBS only. In contrast, with respect to the mdx mice administered with the double-stranded nucleic acid complex agent (Toc-HDO, n=4) or the double-stranded nucleic acid complex agent (Chol-HDO, n=6), the running duration was greatly increased. Especially in the case of the double-stranded nucleic acid complex agent (Chol-HDO), the motor ability was restored to a level comparable to that of the positive control B 10 (n=7).

<Example 21>

(Purpose)

The objective is to evaluate the effect on the grip power and motor ability by multiple doses administration of the double-stranded nucleic acid complex (Chol-HDO or Toc-HDO) consisting of an antisense oligonucleotide (morpholino oligomer) targeting the exon 23/intron 23 boundary region in mdx mice for exon skipping and a cholesterol-conjugated complementary strand or a tocopherol-conjugated complementary strand.

(Method)

(1) Preparation of nucleic acids

The preparation method for the nucleic acid complex agent used in this Example was in accordance with the method described in Example 20. The names and sequences of the first nucleic acid strand and the second nucleic acid strand used in this Example are also as shown in Table 15.

(2) *In vivo* experiment

The mice used in this Example and the administration method of the nucleic acid complex agent were in accordance with Example 20.

(3) Grip power measurement test

One week or more after the fifth and final administration, a grip power measurement test was performed. For the grip power measurement test, a grip power measuring device for mouse (MK-380CM, Muromachi Kikai Co., Ltd.), a stainless steel net (Muromachi Kikai Co., Ltd., MK-380CM-F/MM) and a digital force gauge (DS2-50N, IMADA Co., Ltd.) were used. The tail of a mouse holding the wire net with its forelimbs was pulled, and the tension when the mouse released the wire net was measured. The measurements were repeated three times and the mean value was calculated.

(4) Wire hanging test

One week or more after the fifth and final administration, a wire hanging test was performed. The wire hanging test was performed by allowing a mouse to cling to a wire net, followed by overturning the wire net, and measuring the time period (hang time) until the mouse fell down from the net. The product of the mouse's body mass (g) and the hang time (s) was calculated and defined as the holding impulse (s*g) [Holding impulse (s*g) = Body mass (g) × Hang time (s)]. The mean value of the two measurements was calculated.

(Results)

The results are shown in Figure 30. With respect to the mdx mice (n=9) administered with the single-stranded nucleic acid complex agent (PMO) the grip power and the holding impulse were slightly increased compared to the negative control mdx mice (mdx, n=7) administered with PBS only. In contrast, with respect to the mdx mice administered with the double-stranded nucleic acid complex agent (Toc-HDO, n=9) or the double-stranded nucleic acid complex agent (Chol-HDO, n=6) the grip power and the holding impulse were shown to have greatly increased. In Figure 30, B10 (n=6 for grip power and n=5 for holding impulse) was used as a positive control.

<Example 22>

(Purpose)

In the blood of a mdx mouse, the concentrations of a creatine kinase (CK), an aspartate aminotransferase (AST), and an alanine aminotransferase (ALT) that are derived from muscles are elevated. Therefore, the objective is to evaluate the levels of CK, AST, and ALT in the serum after multiple doses administration of the double-stranded nucleic acid complex (Chol-HDO or Toc-HDO) consisting of an antisense oligonucleotide (morpholino oligomer) targeting the exon 23/intron 23 boundary region in mdx mice for exon skipping and a cholesterol-conjugated complementary strand or a tocopherol-conjugated complementary strand.

(Method)

(1) Preparation of nucleic acids

The preparation method for the nucleic acid complex agent used in this Example was in accordance with the method described in Example 20. The names and sequences of the first nucleic acid strand and the second nucleic acid

strand used in this Example are also as shown in Table 15.

(2) *In vivo* experiment

The mice used in this Example and the administration method of the nucleic acid complex agent were in accordance with Example 20.

(3) Serum analysis

One week or more after the fifth and final administration, the blood was taken from the mouse and the serum was separated. The obtained serum was sent to SRL Inc. for the measurements of CK, AST, and ALT.

(Results)

The results are shown in Figure 31. The mdx mice (n=7) to which a single-stranded nucleic acid complex agent (PMO) was administered showed a slight decrease in each of the CK, AST, and ALT levels compared to the negative control mdx mice (mdx, n=11) to which only PBS was administered. In contrast, the mdx mice administered with the double-stranded nucleic acid complex agent (Toc-HDO, n=7), or the double-stranded nucleic acid complex agent (Chol-HDO, n=5 or 6) showed a significant decrease in each of CK, AST, and ALT. Especially, in the case of Chol-HDO it was shown that those levels were decreased to the comparable levels as the positive control B10 (n=8).

<Example 23>

(Purpose)

The objective is to evaluate the effect on the heart muscle function by multiple doses administration of the double-stranded nucleic acid complex (Chol-HDO or Toc-HDO) consisting of an antisense oligonucleotide (morpholino oligomer) targeting the exon 23/intron 23 boundary region in mdx mice for exon skipping and a cholesterol-conjugated complementary strand or a tocopherol-conjugated complementary strand.

(Method)

(1) Preparation of nucleic acids

The preparation method for the nucleic acid complex agent used in this Example was in accordance with the method described in Example 20. The names and sequences of the first nucleic acid strand and the second nucleic acid strand used in this Example are also as shown in Table 15.

(2) *In vivo* experiment

The mice used in this Example and the administration method of the nucleic acid complex agent were in accordance with Example 20.

(3) Electrocardiogram measurement

One week or more after the fifth and final administration, an electrocardiogram measurement was performed. The electrocardiogram measurement was performed under isoflurane anesthesia using a PowerLab 2/26 PL2602 with 2 analog input channels, and a High Performance Differential Bio Amplifier ML132. The QT time was corrected with the RR interval to calculate a corrected QT time (QTc).

(Results)

The results are shown in Figure 32. The negative control mdx mice (mdx, n=5) to which only PBS was administered had a prolonged QTc compared to the normal mice (B10, n=5). In the mdx mice to which the single-stranded nucleic acid complex agent was administered (PMO, n=5), the QTc prolongation was almost unimproved. On the other hand, in the mdx mice to which the double-stranded nucleic acid complex agent (Toc-HDO, n=5) or the double-stranded nucleic acid complex agent (Chol-HDO, n=5) was administered, the QTc prolongation was shown to be significantly improved.

<Example 24>

(Purpose)

The objective is to evaluate the expression of the dystrophin protein in the heart muscle and quadriceps muscle by multiple doses administration of the double-stranded nucleic acid complex (Chol-HDO or Toc-HDO) consisting of an antisense oligonucleotide (morpholino oligomer) targeting the exon 23/intron 23 boundary region in mdx mice for exon skipping, and a cholesterol-conjugated complementary strand or a tocopherol-conjugated complementary strand.

(Method)

(1) Preparation of nucleic acids

The preparation method for the nucleic acid complex agent used in this Example was in accordance with the method described in Example 20. The names and sequences of the first nucleic acid strand and the second nucleic acid strand used in this Example are also as shown in Table 15.

(2) *In vivo* experiment

The mice used in this Example and the administration method of the nucleic acid complex agent were in accordance with Example 20.

(3) Expression analysis

One week or more after the fifth and final administration, mice were dissected and expression analyses by Western blotting and immunostaining were performed. The method of expression analysis was in accordance with the method described in Example 15. However, in this Example, also Western blotting of vinculin was performed together as a control. An anti-vinculin antibody (anti-Vinculin (hVIN-1) antibody; Novus Biologicals, NB600-1293) was used at 1/1000.

(Results)

The results are shown in Figures 33 to 36. Compared to the mdx mice to which the single-stranded nucleic acid complex agent (PMO) was administered, in the mdx mice to which the double-stranded nucleic acid complex agent (Toc-HDO) or the double-stranded nucleic acid complex agent (Chol-HDO) was administered, a higher level of expression of dystrophin was observed in the heart muscle (Figure 33) and the quadriceps muscle (Figure 34). Further, in the immunostaining in Figures 35 and 36, a higher level of expression of dystrophin was observed in the heart muscle (Figure 35) and quadriceps muscle (Figure 36) for the double-stranded nucleic acid complex agent (Toc-HDO) or the double-stranded nucleic acid complex agent (Chol-HDO) compared to the single-stranded nucleic acid complex agent (PMO).

## Claims

1. A double-stranded nucleic acid complex for suppressing or increasing the expression level of a transcription product or a translation product of a target gene, or inhibiting the function of a transcription product or a translation product of a target gene in the skeletal muscle or heart muscle of a subject, the complex comprising a first nucleic acid strand and a second nucleic acid strand, wherein:

   said first nucleic acid strand comprises a base sequence that is capable of hybridizing to all or part of said transcription product of the target gene, and has an antisense effect on said transcription product,
   said second nucleic acid strand comprises a base sequence complementary to said first nucleic acid strand, and is bound to cholesterol or analog thereof, and
   said first nucleic acid strand is annealed to said second nucleic acid strand.

2. The double-stranded nucleic acid complex according to claim 1, wherein said first nucleic acid strand comprises at least four consecutive deoxyribonucleosides.

3. The double-stranded nucleic acid complex according to claim 2, wherein said first nucleic acid strand is a gapmer.

4. The double-stranded nucleic acid complex according to claim 1 or 2, wherein said first nucleic acid strand is a mixmer.

5. The double-stranded nucleic acid complex according to any one of claims 1 to 4, wherein said second nucleic acid strand comprises at least four consecutive ribonucleosides complementary to at least four consecutive deoxyribonucleosides in said first nucleic acid strand.

6. The double-stranded nucleic acid complex according to any one of claims 1 to 5, wherein said second nucleic acid strand does not comprise a natural ribonucleoside.

7. The double-stranded nucleic acid complex according to any one of claims 1 to 6, wherein the nucleic acid portion of said second nucleic acid strand consists of deoxyribonucleosides and/or sugar-modified nucleosides linked by modified or unmodified internucleoside linkages.

8. The double-stranded nucleic acid complex according to any one of claims 1 to 7, wherein said second nucleic acid strand is bound to cholesterol or analog thereof.

9. The double-stranded nucleic acid complex according to any one of claims 1 to 8, wherein said cholesterol or analog thereof is bound to 5' end and/or 3' end of said second nucleic acid strand.

10. The double-stranded nucleic acid complex according to any one of claims 1 to 9, wherein said second nucleic acid

strand is bound to a ligand via a cleavable or uncleavable linker.

**11.** The double-stranded nucleic acid complex according to any one of claims 1 to 10, wherein said first nucleic acid strand is bound to said second nucleic acid strand via said linker.

**12.** The double-stranded nucleic acid complex according to claim 10 or 11, wherein said linker consists of nucleic acids.

**13.** A pharmaceutical composition comprising the double-stranded nucleic acid complex according to any one of claims 1 to 12 as an active ingredient.

**14.** The pharmaceutical composition according to claim 13 for treating skeletal muscle dysfunction, or cardiac dysfunction of a subject.

**15.** The pharmaceutical composition according to claim 13 or 14, wherein said skeletal muscle dysfunction or cardiac dysfunction is a disease selected from the group consisting of muscular dystrophy, myopathy, inflammatory myopathy, polymyositis, dermatomyositis, Danon disease, myasthenic syndrome, mitochondrial disease, myoglobinuria, glycogen storage disease, periodic paralysis, hereditary cardiomyopathy, hypertrophic cardiomyopathy, dilated cardiomyopathy, hereditary arrhythmia, neurodegenerative disorder, sarcopenia, and cachexia.

**16.** The pharmaceutical composition according to any one of claims 13 to 15 wherein the pharmaceutical composition is administered by intravenous, intramuscular, or subcutaneous administration.

**17.** The pharmaceutical composition according to any one of claims 13 to 16, wherein a single dose of said double-stranded nucleic acid complex is 0.1 mg/kg or more.

**18.** The pharmaceutical composition according to any one of claims 13 to 17, wherein a single dose of said double-stranded nucleic acid complex is from 0.01 mg/kg to 200 mg/kg.

**19.** The pharmaceutical composition according to any one of claims 13 to 18, wherein the transcription product of the target gene is an RNA selected from the group consisting of mRNA, microRNA, pre-mRNA, long non-coding RNA, and natural antisense RNA.

**20.** The pharmaceutical composition according to any one of claims 13 to 19, wherein the first nucleic acid strand is an RNA selected from the group consisting of steric blocking, splicing switch, exon skipping, and exon inclusion.

**21.** The pharmaceutical composition according to any one of claims 13 to 20, wherein the base sequence of the first nucleic acid strand in said double-stranded nucleic acid complex is represented by SEQ ID NO: 24.

**22.** A double-stranded nucleic acid complex for inducing RNA editing, exon skipping, or exon inclusion of a target gene, or causing steric blocking of a target RNA in the skeletal muscle or heart muscle of a subject, the complex comprising a first nucleic acid strand and a second nucleic acid strand, wherein:

said first nucleic acid strand comprises a base sequence that is capable of hybridizing to all or part of the transcription product of said target gene, and has an antisense effect on said transcription product,
said second nucleic acid strand comprises a base sequence complementary to said first nucleic acid strand, and
said first nucleic acid strand is annealed to said second nucleic acid strand.

**23.** The double-stranded nucleic acid complex according to claim 22, wherein said first nucleic acid strand comprises at least one morpholino nucleic acid or nucleic acid modified at the 2'-position of the ribose.

**24.** The double-stranded nucleic acid complex according to claim 22 or 23, wherein 50% or more of bases in said first nucleic acid strand are morpholino nucleic acids or nucleic acids modified at the 2'-position of the ribose.

**25.** The double-stranded nucleic acid complex according to any one of claims 22 to 24, wherein said first nucleic acid strand is a mixmer.

**26.** The double-stranded nucleic acid complex according to any one of claims 22 to 25, wherein 100% of bases in said first nucleic acid strand are morpholino nucleic acids or nucleic acids modified at the 2'-position of the ribose.

**27.** The double-stranded nucleic acid complex according to any one of claims 22 to 26, wherein said second nucleic acid strand does not comprise a natural ribonucleoside.

**28.** The double-stranded nucleic acid complex according to any one of claims 22 to 27, wherein the nucleic acid portion of said second nucleic acid strand consists of deoxyribonucleosides and/or sugar-modified nucleosides linked by modified or unmodified internucleoside linkages.

**29.** The double-stranded nucleic acid complex according to any one of claims 22 to 28, wherein said second nucleic acid strand is bound to a functional moiety.

**30.** The double-stranded nucleic acid complex according to any one of claims 22 to 28, wherein said functional moiety is selected from the group consisting of cholesterol or analog thereof, tocopherol or analog thereof, phosphatidylethanolamine or analog thereof, a substituted or unsubstituted C1-C30 alkyl group, a substituted or unsubstituted C2-C30 alkenyl group, and a substituted or unsubstituted C1-C30 alkoxy group.

**31.** The double-stranded nucleic acid complex according to claim 30, wherein said functional moiety is cholesterol or analog thereof.

**32.** The double-stranded nucleic acid complex according to any one of claims 22 to 31, wherein said cholesterol or analog thereof is bound to 5' end and/or 3' end of said second nucleic acid strand.

**33.** The double-stranded nucleic acid complex according to any one of claims 22 to 32, wherein said second nucleic acid strand is bound to a ligand via a cleavable or uncleavable linker.

**34.** A pharmaceutical composition comprising the double-stranded nucleic acid complex according to any one of claims 22 to 33 as an active ingredient.

**35.** The pharmaceutical composition according to claim 34 for treating muscular dystrophy of a subject.

**36.** The pharmaceutical composition according to claim 35, wherein said muscular dystrophy is myotonic dystrophy or Duchenne muscular dystrophy.

**37.** The pharmaceutical composition according to any one of claims 34 to 36, wherein the pharmaceutical composition is administered by intravenous or subcutaneous administration.

**38.** The pharmaceutical composition according to any one of claims 34 to 37, wherein a single dose of said double-stranded nucleic acid complex is 0.1 mg/kg or more.

**39.** The pharmaceutical composition according to any one of claims 34 to 38, wherein a single dose of said double-stranded nucleic acid complex is from 0.01 mg/kg to 200 mg/kg.

**40.** The pharmaceutical composition according to any one of claims 34 to 39, wherein the base sequence of the first nucleic acid strand in said double-stranded nucleic acid complex is represented by any one of SEQ ID NOs: 25 to 28.

Fig. 1

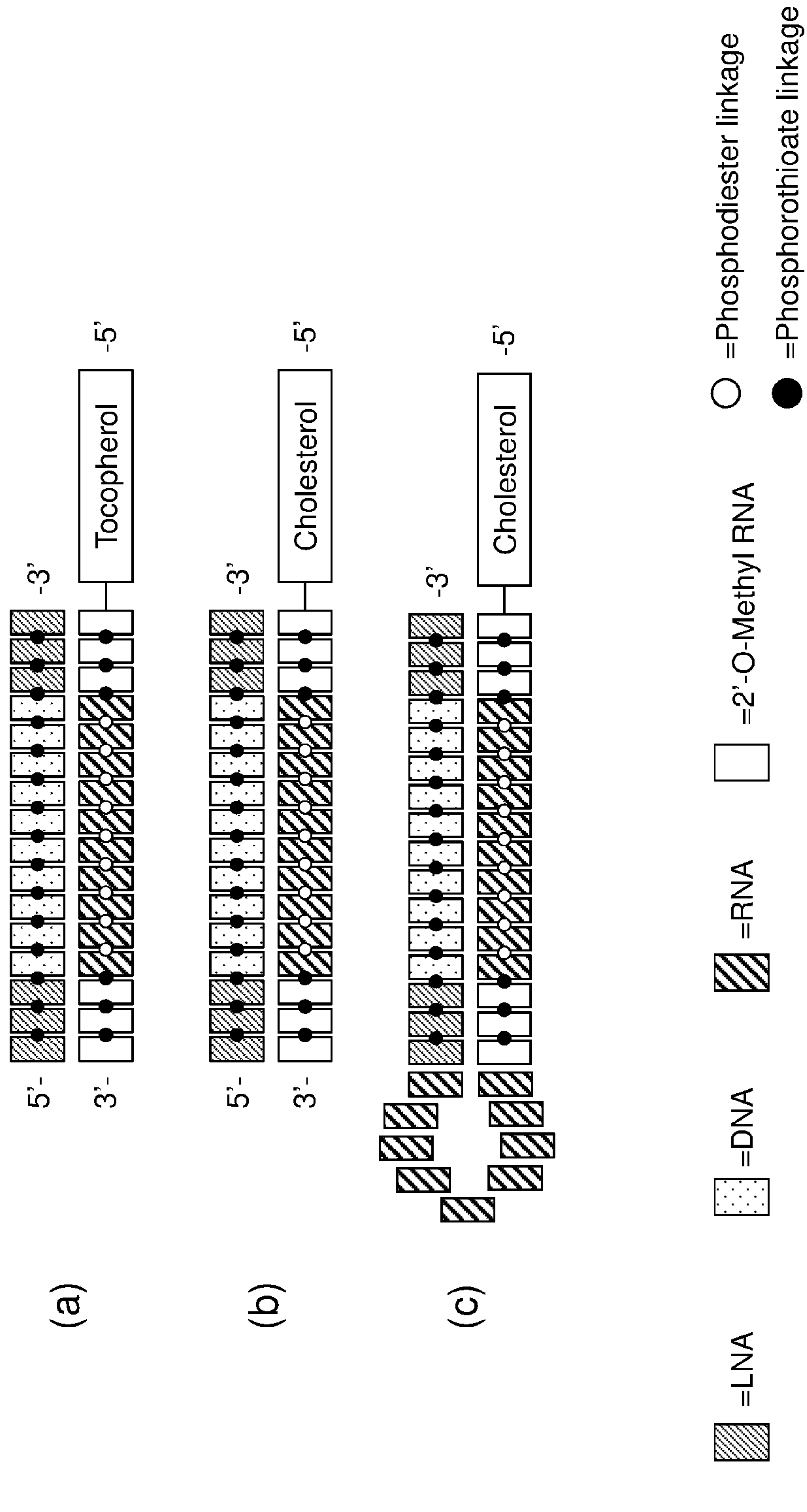

(a)
5'-
-3'
3'-
-5'
Tocopherol
(b)
5'-
-3'
3'-
-5'
Cholesterol
(c)
-3'
-5'
Cholesterol
=LNA
=DNA
=RNA
=2'-O-Methyl RNA
=Phosphodiester linkage
=Phosphorothioate linkage

Fig. 2

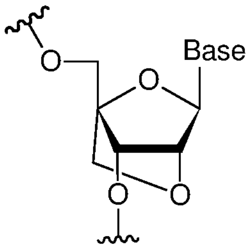

LNA

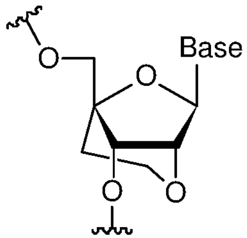

ENA

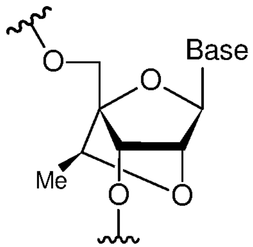

cEt

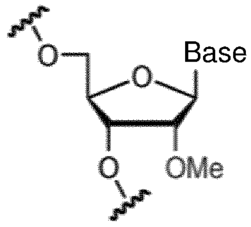

**2'-*O*-Me-RNA**

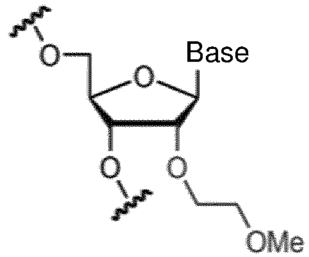

**2'-*O*-MOE-RNA**

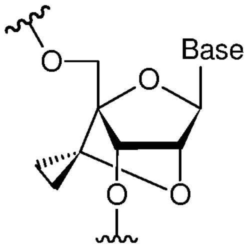

2'-O,4'C-Spirocyclopropylene-
bridged nucleic acid
(scpBNA)

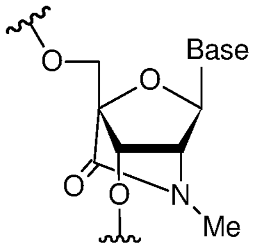

Amido-bridged nucleic acid
(AmNA)

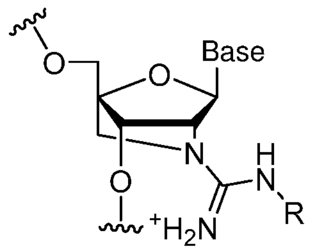

Guanidine-bridged nucleic acid
(GuNA)
(GuNA[N-H] when R=H;
GuNA[N-Me] when R=Me)

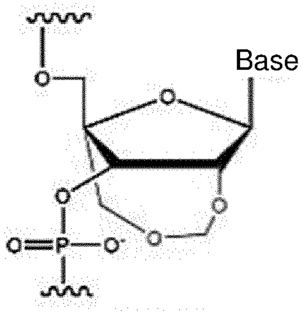

BNA$^{COC}$

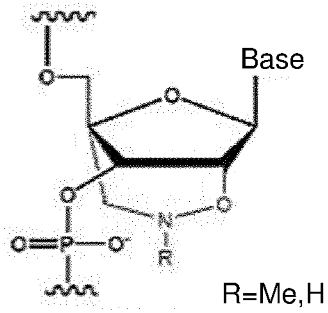

R=Me,H

BNA$^{NC}$

Fig. 3

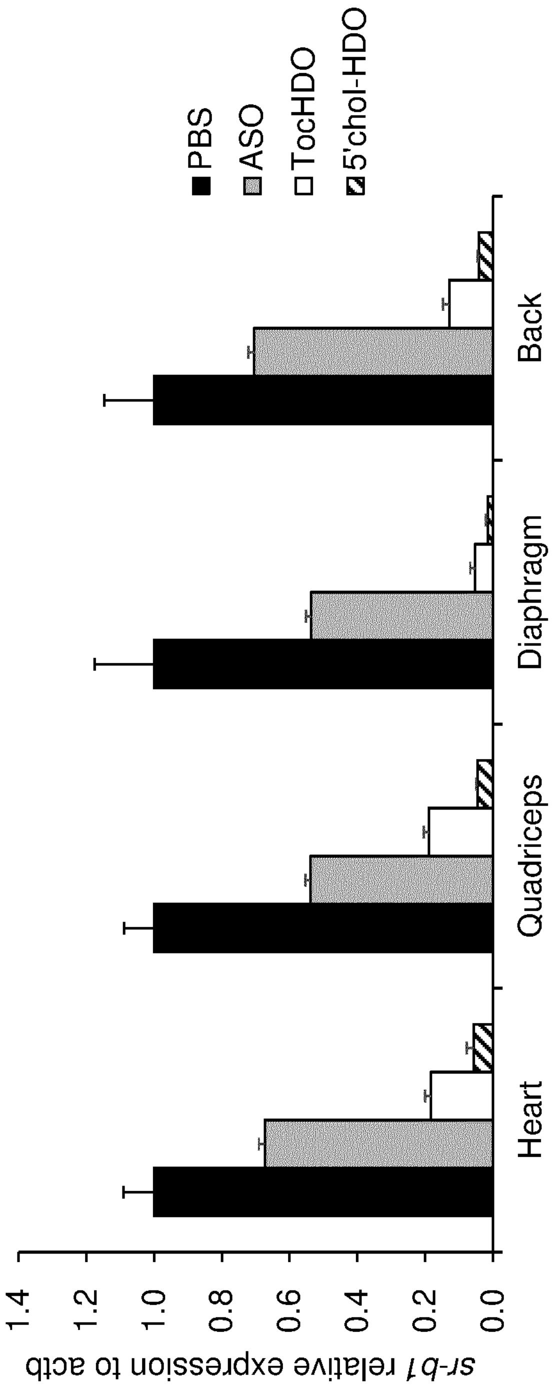

sr-b1 relative expression to actb
1.4
1.2
1.0
0.8
0.6
0.4
0.2
0.0
Heart
Quadriceps
Diaphragm
Back
PBS
ASO
TocHDO
5'chol-HDO

Fig. 4

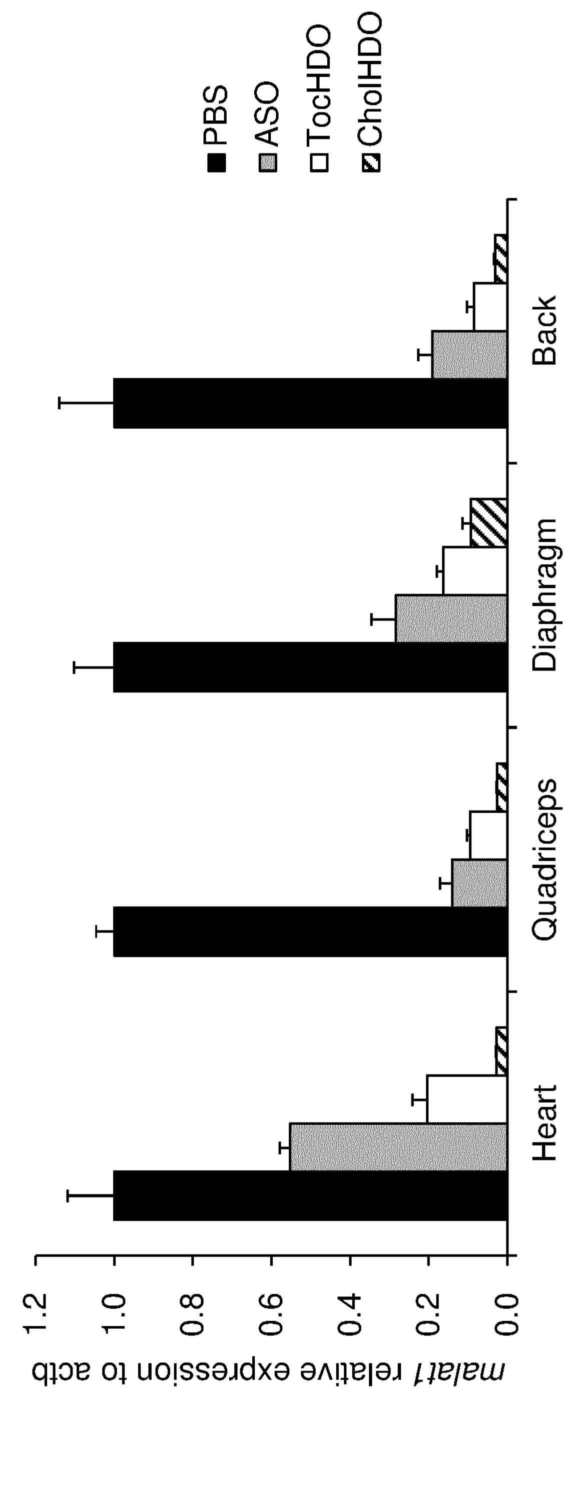
malat1 relative expression to actb
0.0
0.2
0.4
0.6
0.8
1.0
1.2
Heart
Quadriceps
Diaphragm
Back
PBS
ASO
TocHDO
CholHDO

Fig. 5

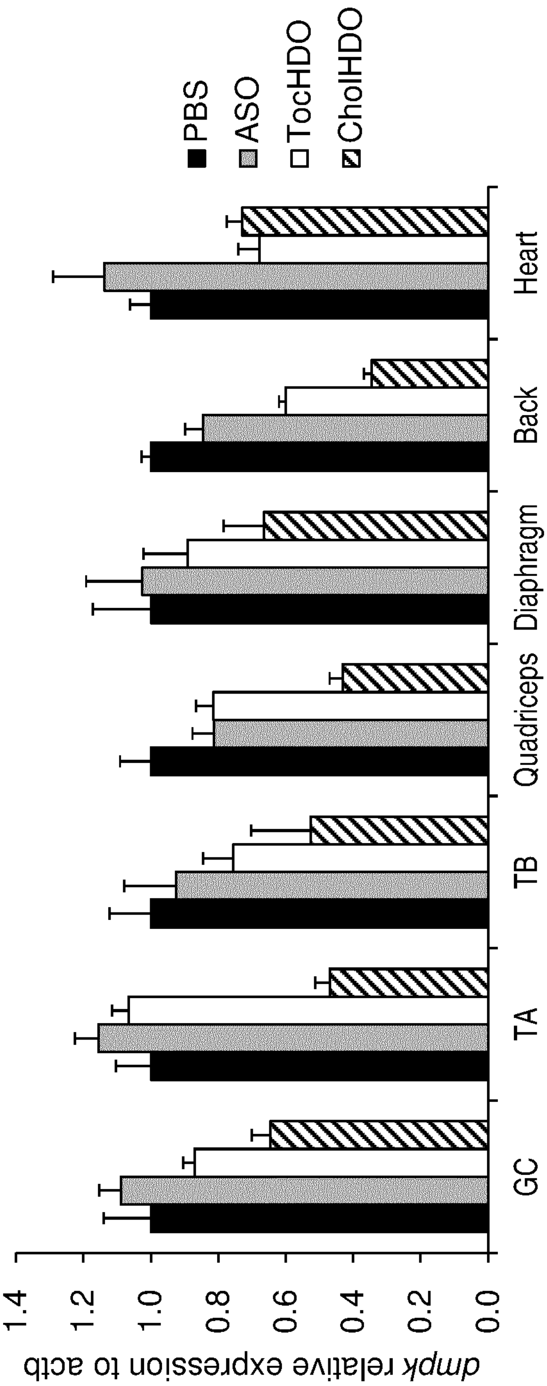
PBS
ASO
TocHDO
CholHDO
*dmpk* relative expression to actb
0.0
0.2
0.4
0.6
0.8
1.0
1.2
1.4
GC
TA
TB
Quadriceps
Diaphragm
Back
Heart

Fig. 6

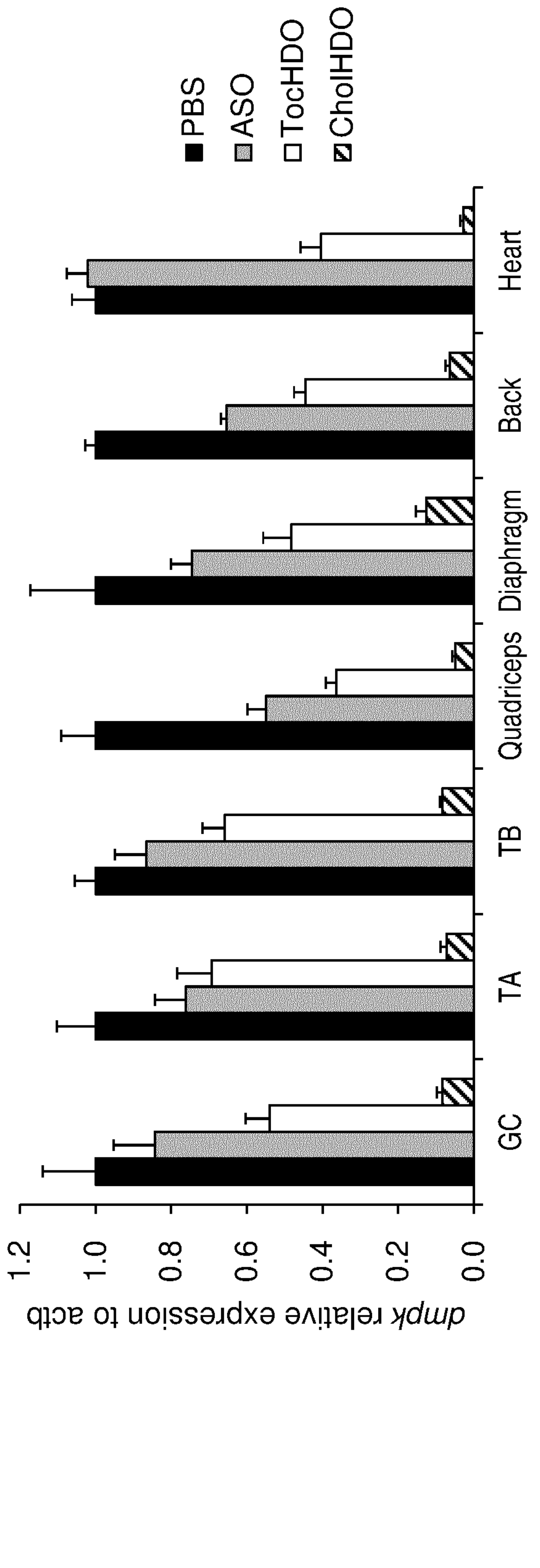

PBS
ASO
TocHDO
CholHDO
dmpk relative expression to actb
0.0
0.2
0.4
0.6
0.8
1.0
1.2
GC
TA
TB
Quadriceps
Diaphragm
Back
Heart

Fig. 7

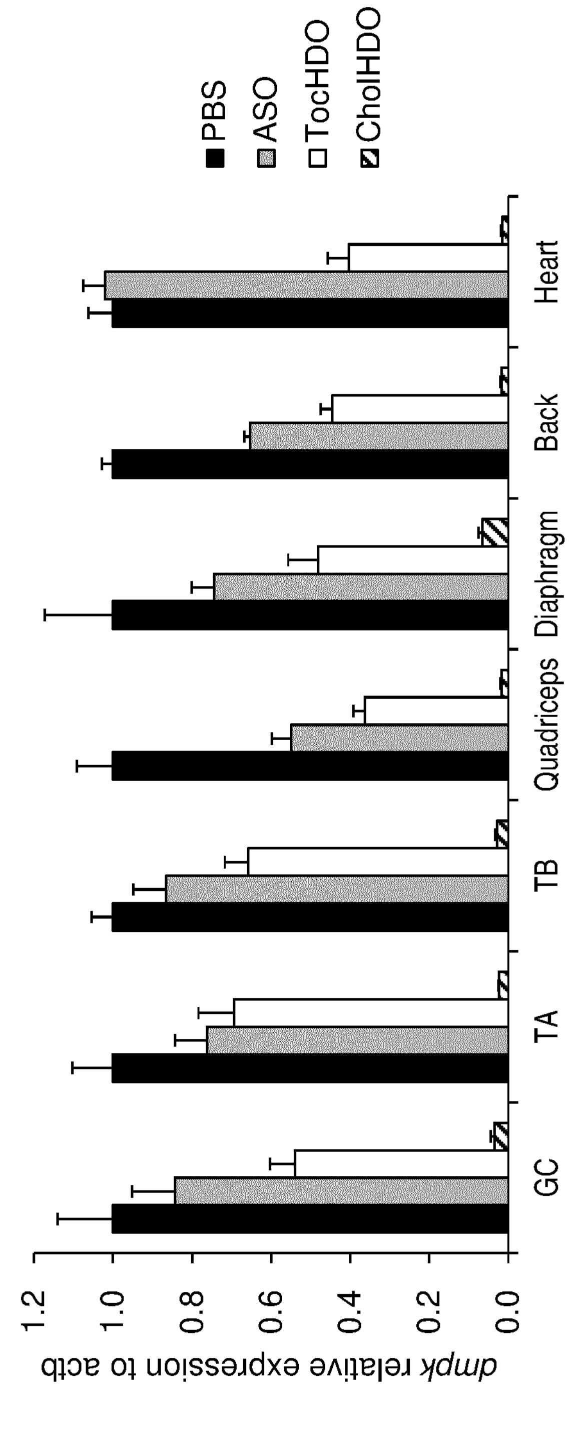

*dmpk* relative expression to actb
0.0
0.2
0.4
0.6
0.8
1.0
1.2
GC
TA
TB
Quadriceps
Diaphragm
Back
Heart
PBS
ASO
TocHDO
CholHDO

Fig. 8

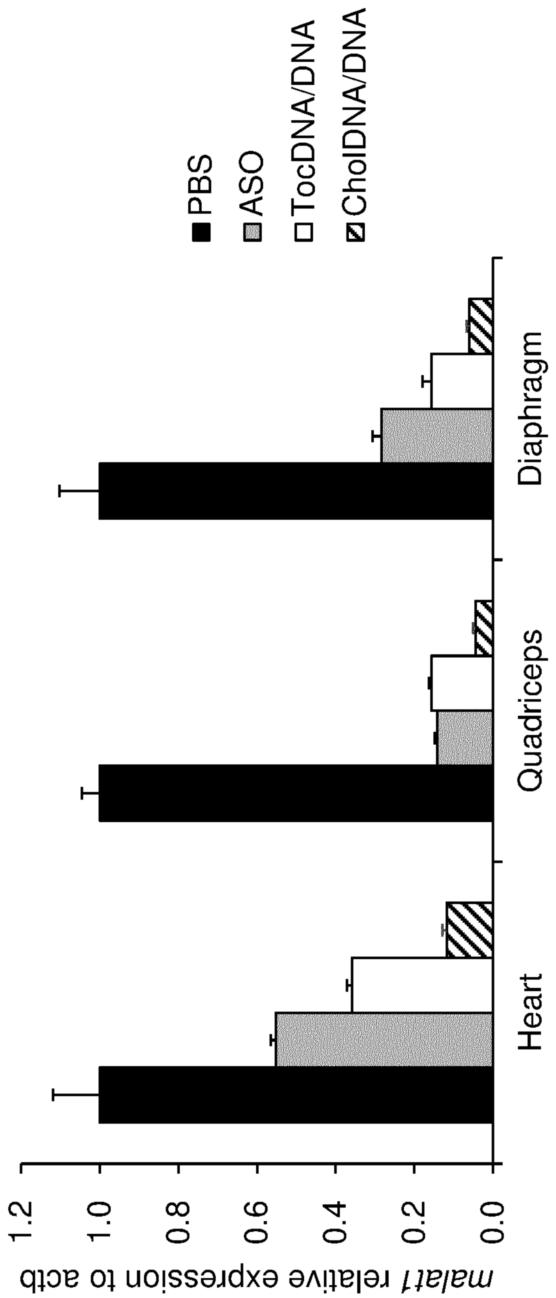
*malat1* relative expression to actb
1.2
1.0
0.8
0.6
0.4
0.2
0.0
Heart
Quadriceps
Diaphragm
PBS
ASO
TocDNA/DNA
CholDNA/DNA

Fig. 9

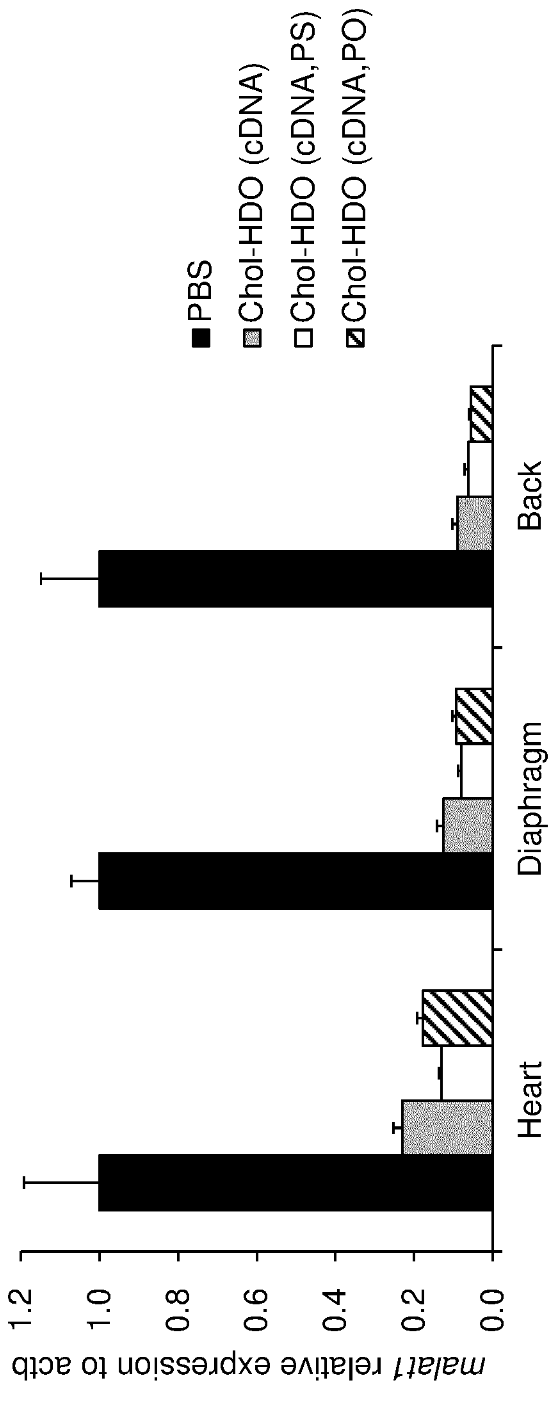
malat1 relative expression to actb
1.2
1.0
0.8
0.6
0.4
0.2
0.0
Heart
Diaphragm
Back
PBS
Chol-HDO (cDNA)
Chol-HDO (cDNA,PS)
Chol-HDO (cDNA,PO)

Fig. 10

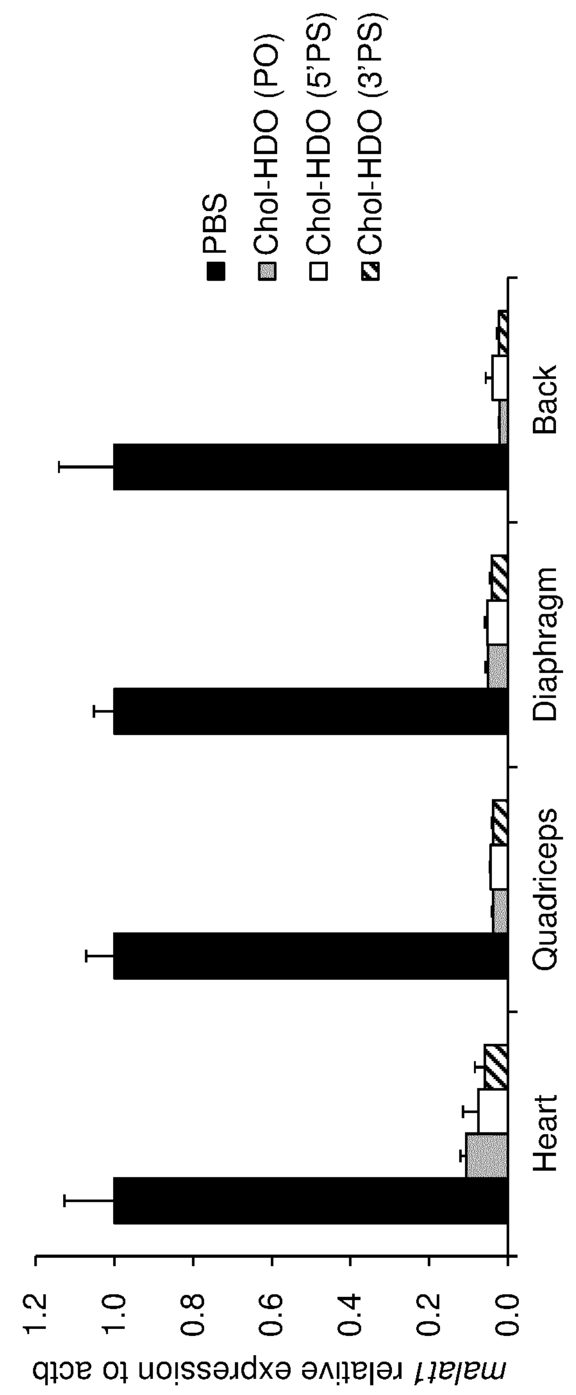
malat1 relative expression to actb
0.0
0.2
0.4
0.6
0.8
1.0
1.2
Heart
Quadriceps
Diaphragm
Back
PBS
Chol-HDO (PO)
Chol-HDO (5'PS)
Chol-HDO (3'PS)

Fig. 11

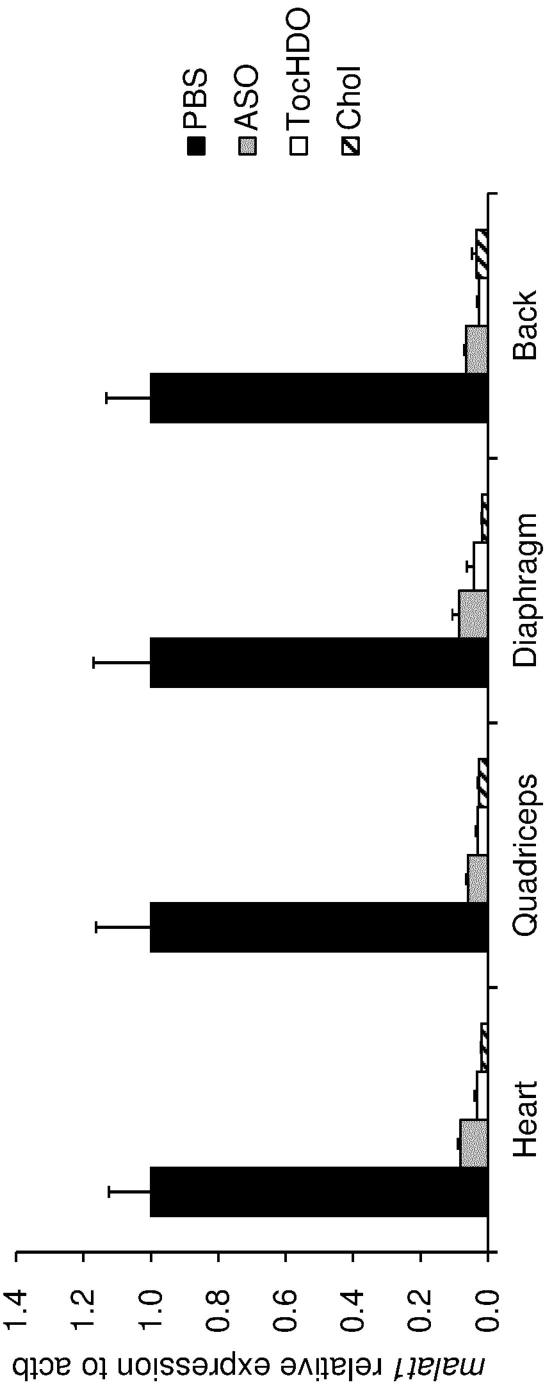

PBS
ASO
TocHDO
Chol
*malat1* relative expression to actb
1.4
1.2
1.0
0.8
0.6
0.4
0.2
0.0
Heart
Quadriceps
Diaphragm
Back

Fig. 12

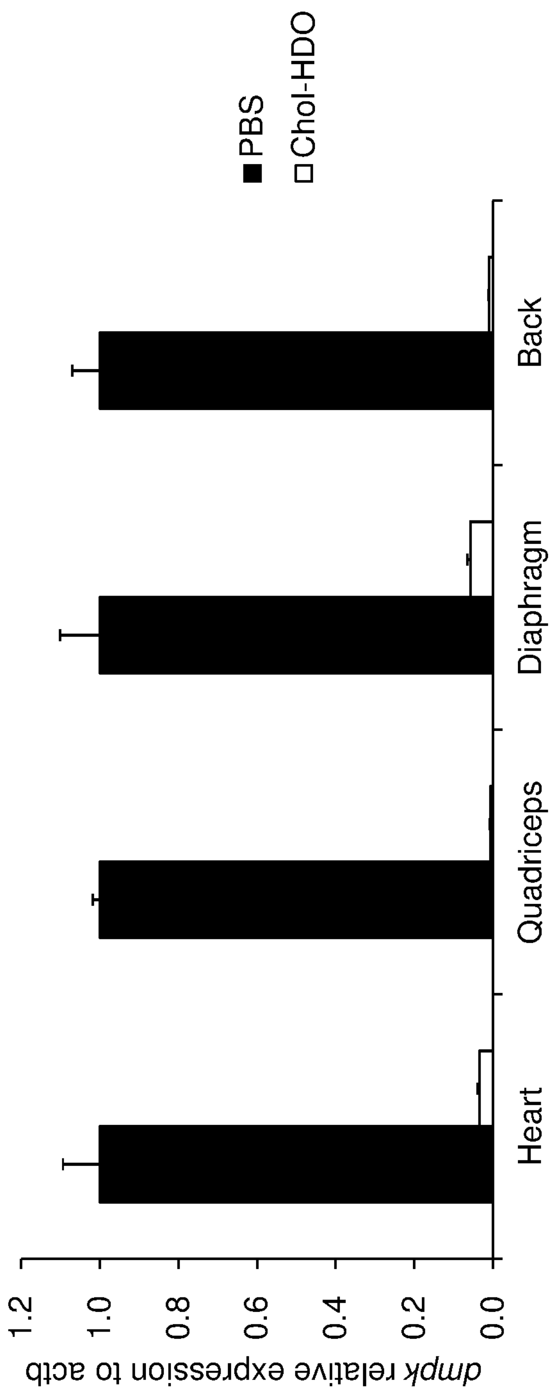
PBS
Chol-HDO
dmpk relative expression to actb
1.2
1.0
0.8
0.6
0.4
0.2
0.0
Heart
Quadriceps
Diaphragm
Back

Fig. 13

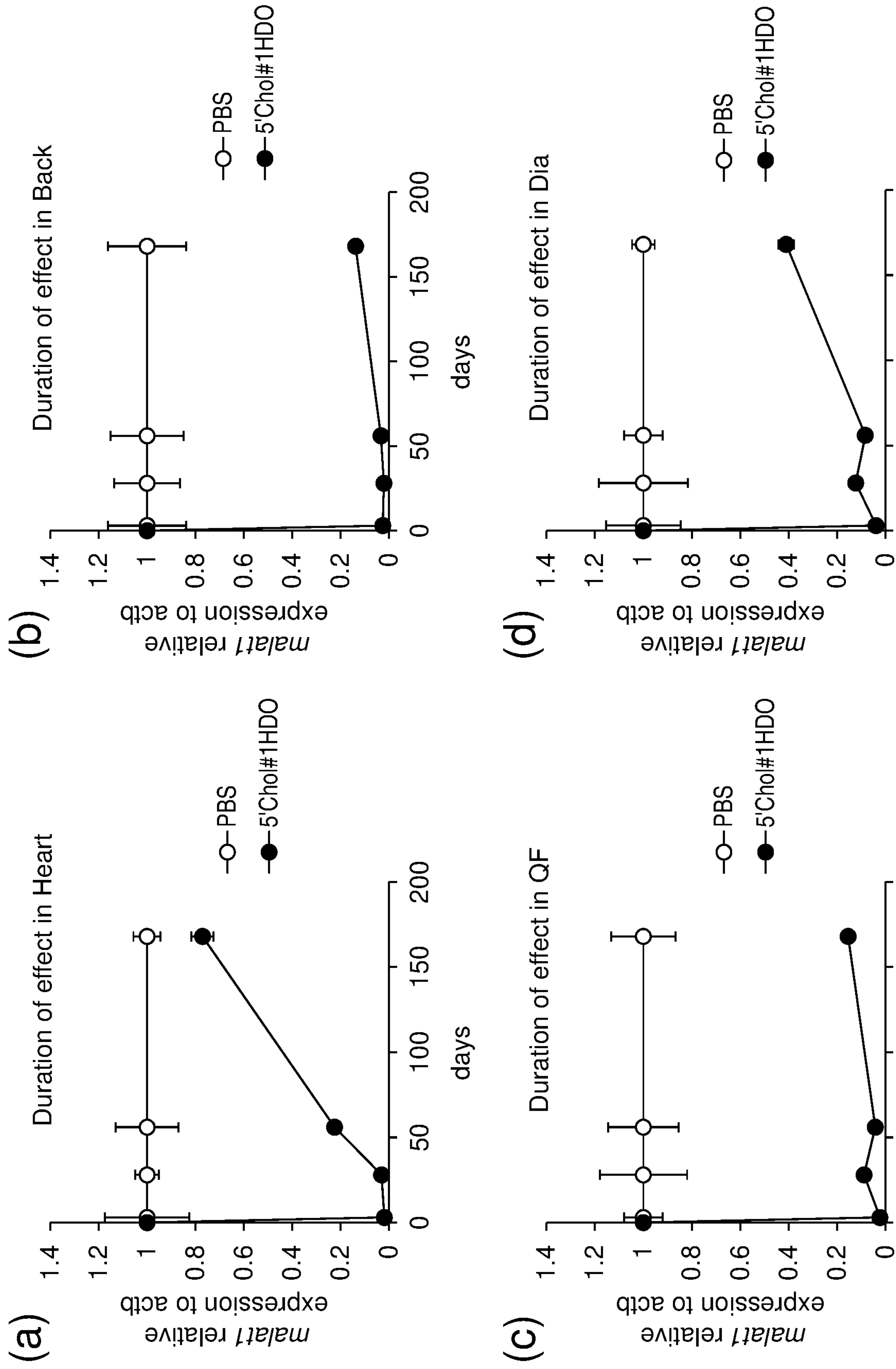
(a)
Duration of effect in Heart
malat1 relative expression to actb
days
0
50
100
150
200
0
0.2
0.4
0.6
0.8
1
1.2
1.4
PBS
5'Chol#1HDO
(b)
Duration of effect in Back
malat1 relative expression to actb
days
PBS
5'Chol#1HDO
(c)
Duration of effect in QF
malat1 relative expression to actb
days
PBS
5'Chol#1HDO
(d)
Duration of effect in Dia
malat1 relative expression to actb
days
PBS
5'Chol#1HDO

Fig. 14

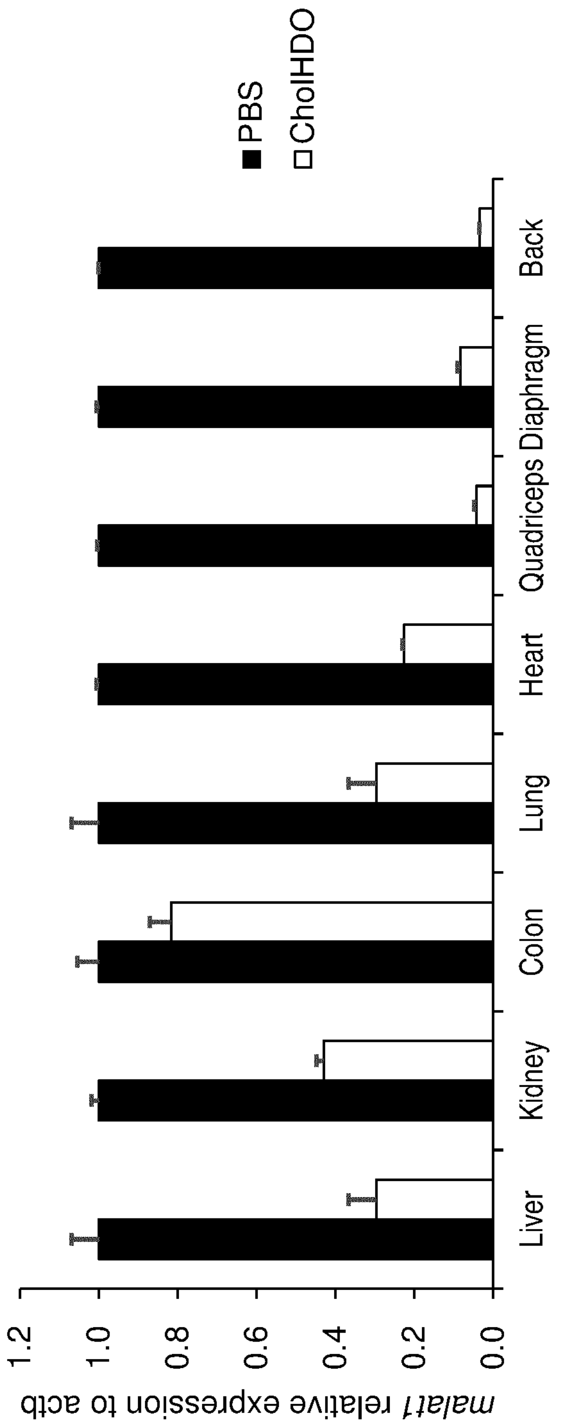

malat1 relative expression to actb
0.0
0.2
0.4
0.6
0.8
1.0
1.2
Liver
Kidney
Colon
Lung
Heart
Quadriceps
Diaphragm
Back
PBS
CholHDO

Fig. 15

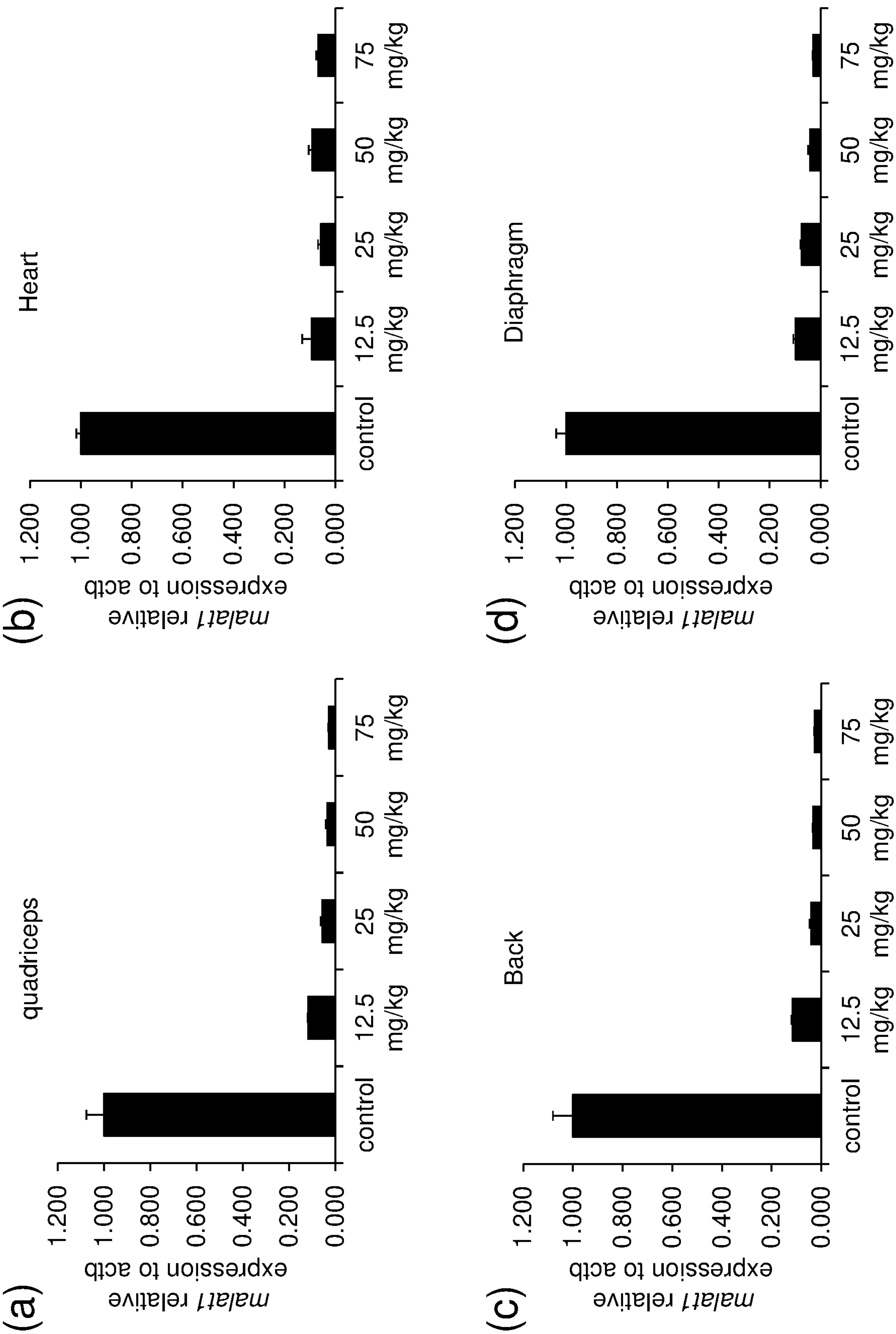
(a)
quadriceps
malat1 relative expression to actb
1.200
1.000
0.800
0.600
0.400
0.200
0.000
control
12.5 mg/kg
25 mg/kg
50 mg/kg
75 mg/kg
(b)
Heart
malat1 relative expression to actb
(c)
Back
malat1 relative expression to actb
(d)
Diaphragm
malat1 relative expression to actb

Fig. 16

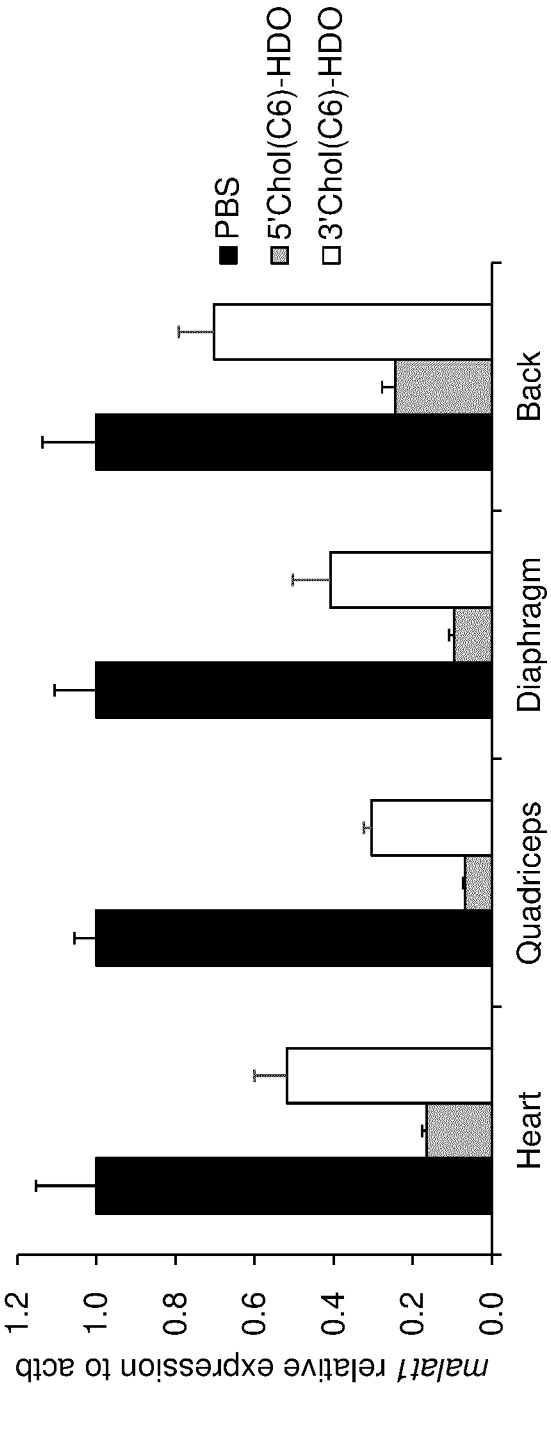

malat1 relative expression to actb
1.2
1.0
0.8
0.6
0.4
0.2
0.0
Heart
Quadriceps
Diaphragm
Back
PBS
5'Chol(C6)-HDO
3'Chol(C6)-HDO

Fig. 17

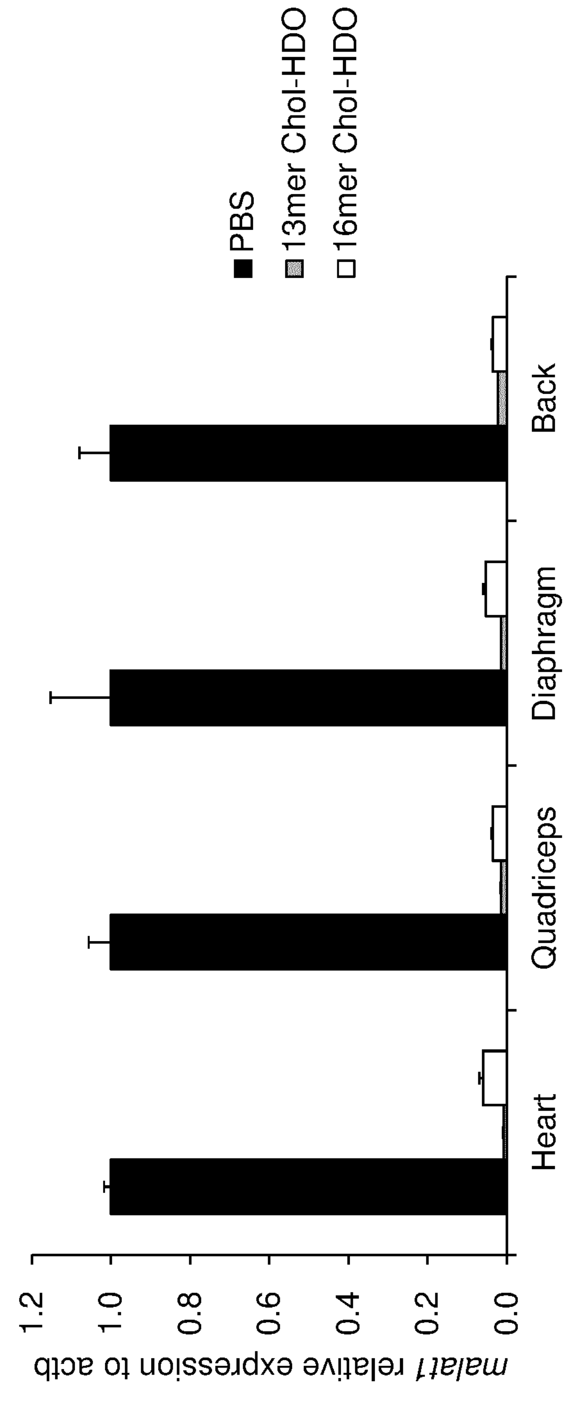
PBS
13mer Chol-HDO
16mer Chol-HDO
*malat1* relative expression to actb
1.2
1.0
0.8
0.6
0.4
0.2
0.0
Heart
Quadriceps
Diaphragm
Back

Fig. 18

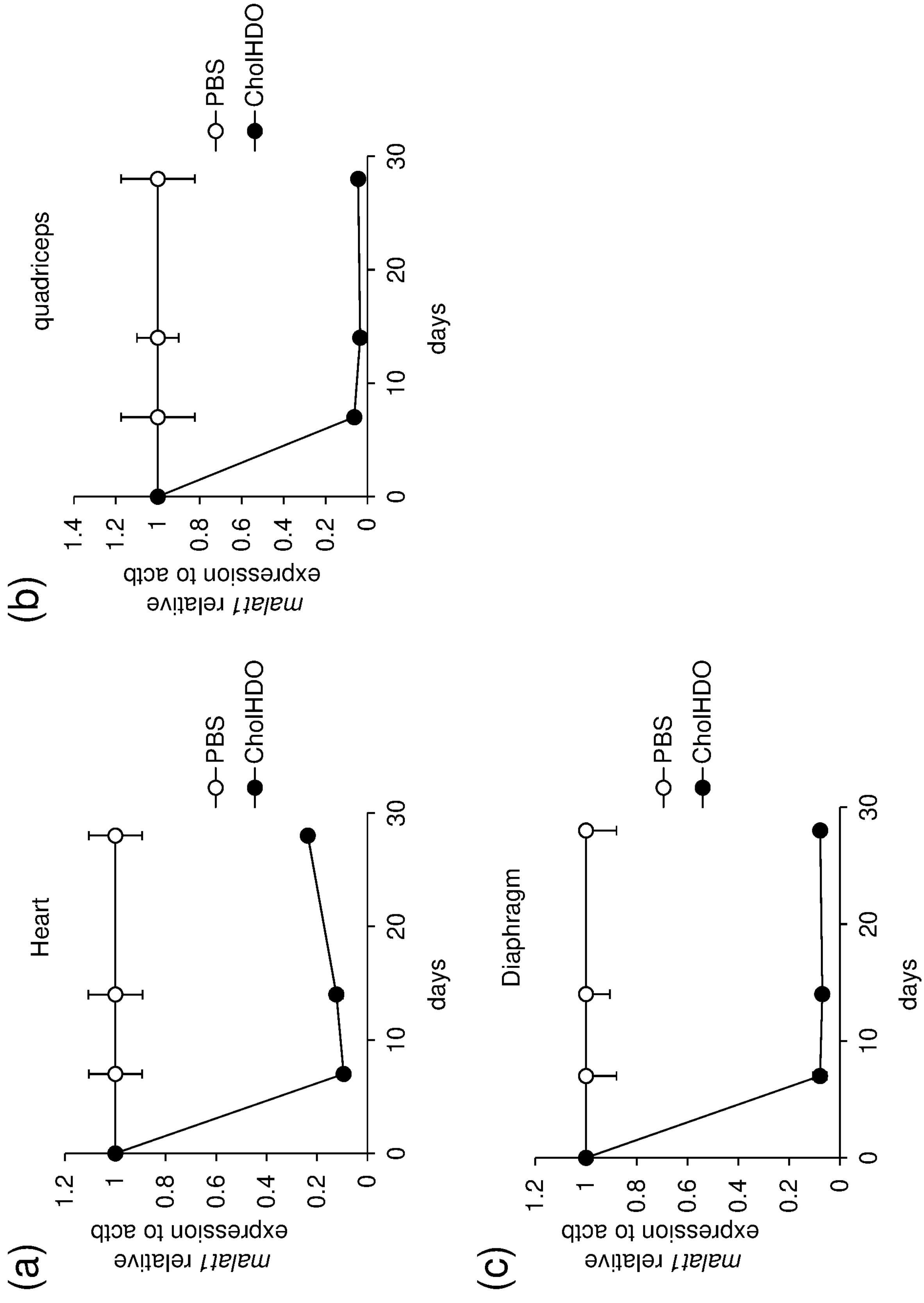
(a)
Heart
malat1 relative expression to actb
days
PBS
CholHDO
(b)
quadriceps
malat1 relative expression to actb
days
PBS
CholHDO
(c)
Diaphragm
malat1 relative expression to actb
days
PBS
CholHDO

Fig. 19

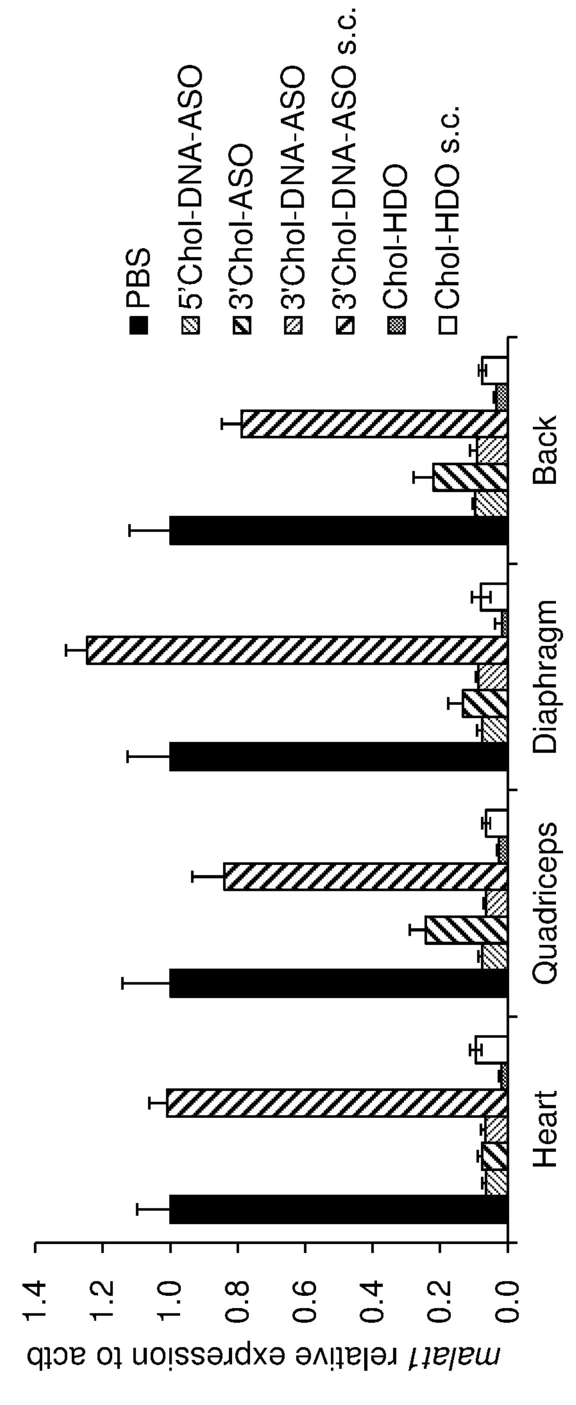
malat1 relative expression to actb
0.0
0.2
0.4
0.6
0.8
1.0
1.2
1.4
Heart
Quadriceps
Diaphragm
Back
PBS
5'Chol-DNA-ASO
3'Chol-ASO
3'Chol-DNA-ASO
3'Chol-DNA-ASO s.c.
Chol-HDO
Chol-HDO s.c.

Fig. 20

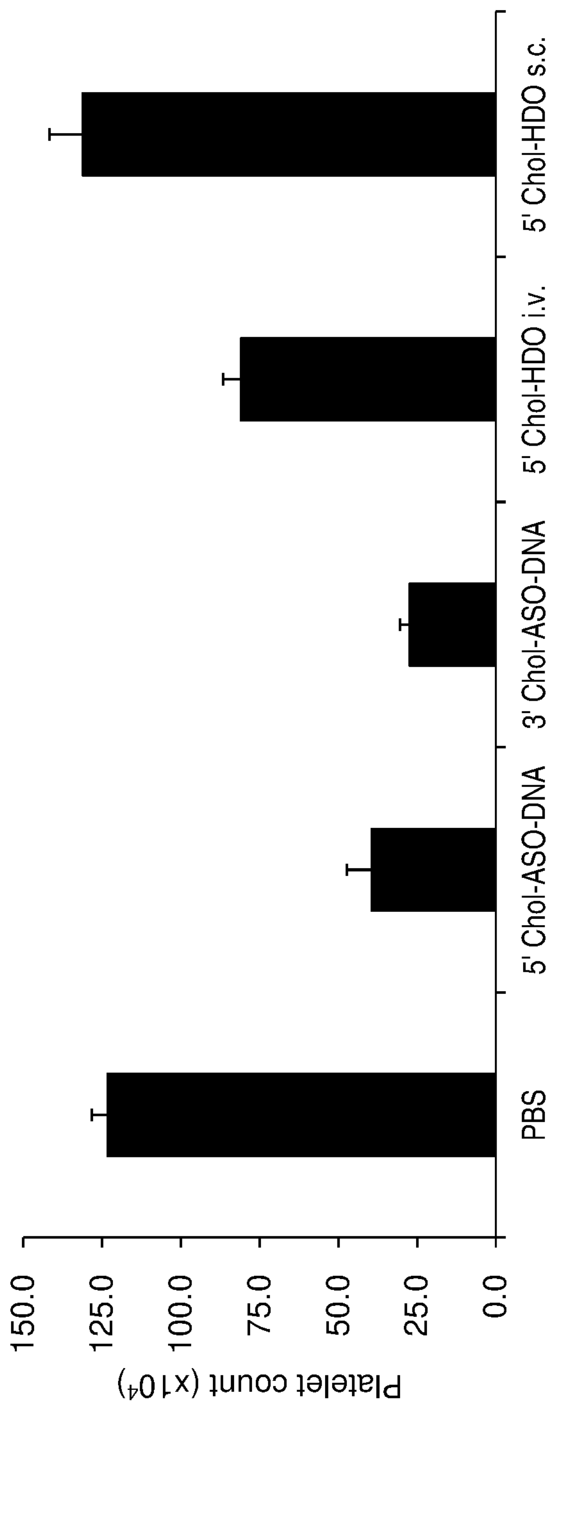
Platelet count (x10⁴)
150.0
125.0
100.0
75.0
50.0
25.0
0.0
PBS
5' Chol-ASO-DNA
3' Chol-ASO-DNA
5' Chol-HDO i.v.
5' Chol-HDO s.c.

Fig. 21

(a)

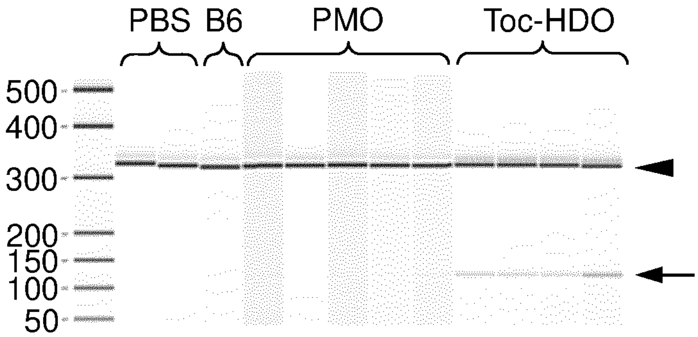
PBS
B6
PMO
Toc-HDO
500
400
300
200
150
100
50

(b)

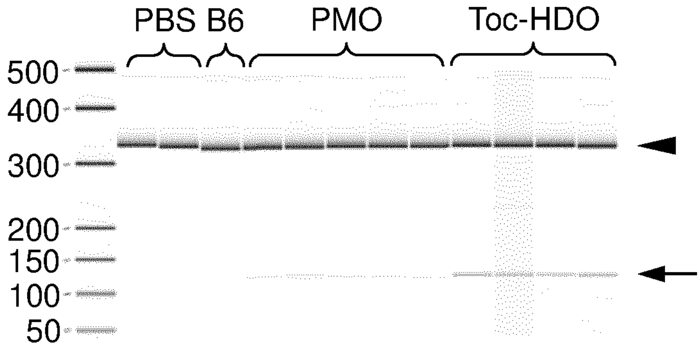
PBS
B6
PMO
Toc-HDO
500
400
300
200
150
100
50

Fig. 22

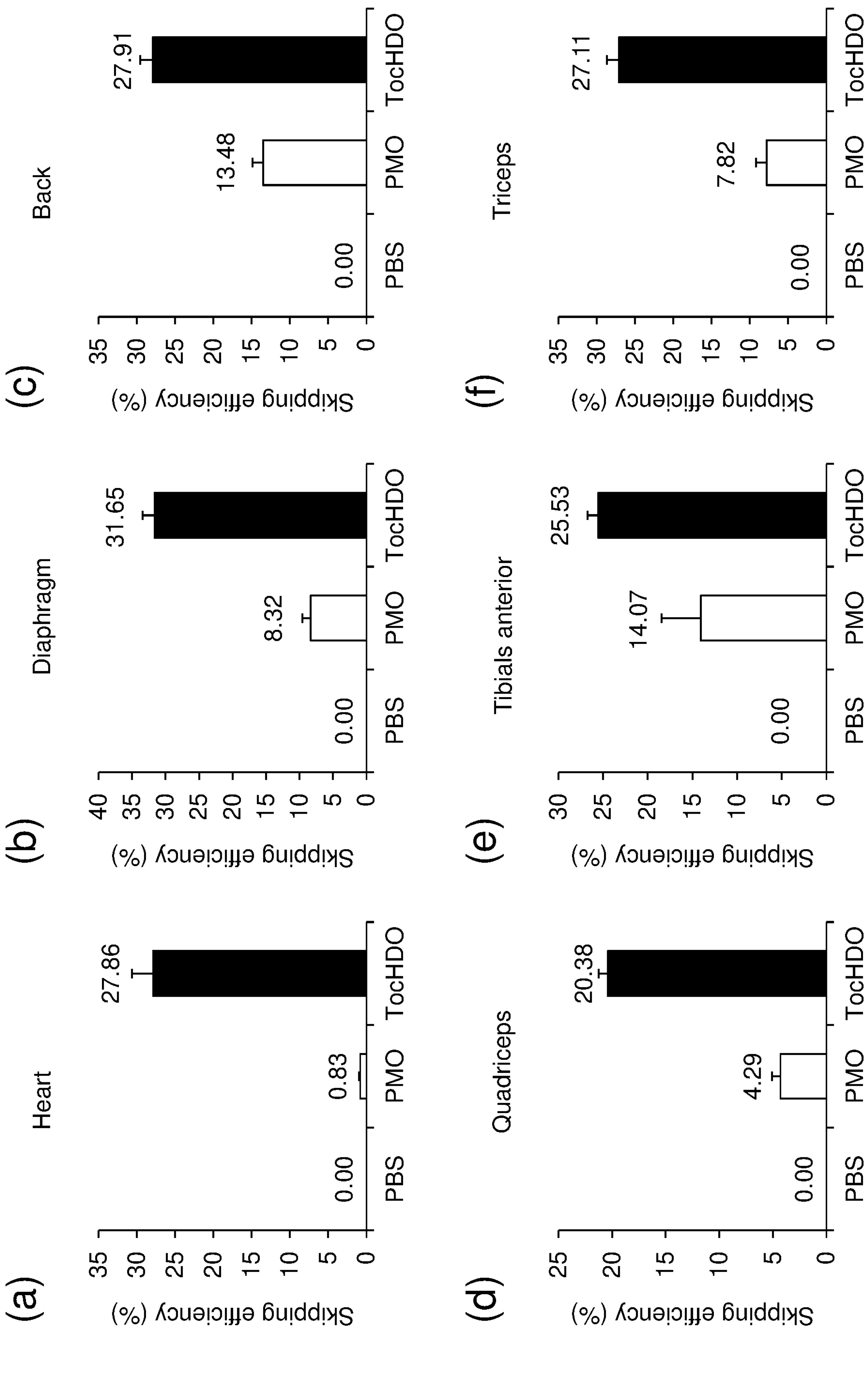
(a)
Heart
Skipping efficiency (%)
PBS
PMO
TocHDO
0.00
0.83
27.86
(b)
Diaphragm
Skipping efficiency (%)
PBS
PMO
TocHDO
0.00
8.32
31.65
(c)
Back
Skipping efficiency (%)
PBS
PMO
TocHDO
0.00
13.48
27.91
(d)
Quadriceps
Skipping efficiency (%)
PBS
PMO
TocHDO
0.00
4.29
20.38
(e)
Tibials anterior
Skipping efficiency (%)
PBS
PMO
TocHDO
0.00
14.07
25.53
(f)
Triceps
Skipping efficiency (%)
PBS
PMO
TocHDO
0.00
7.82
27.11

Fig. 23

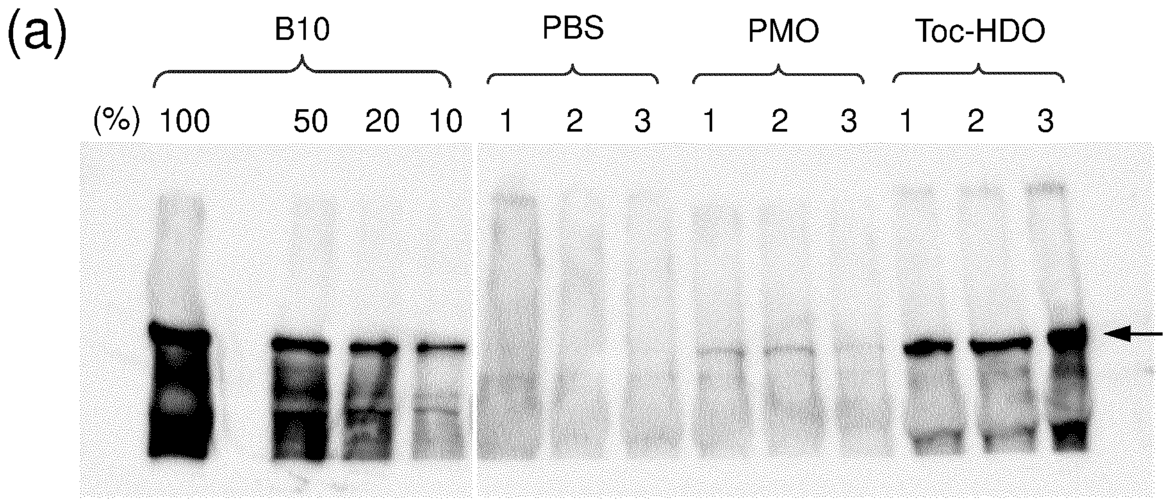
(a)
B10
PBS
PMO
Toc-HDO
(%) 100 50 20 10 1 2 3 1 2 3 1 2 3

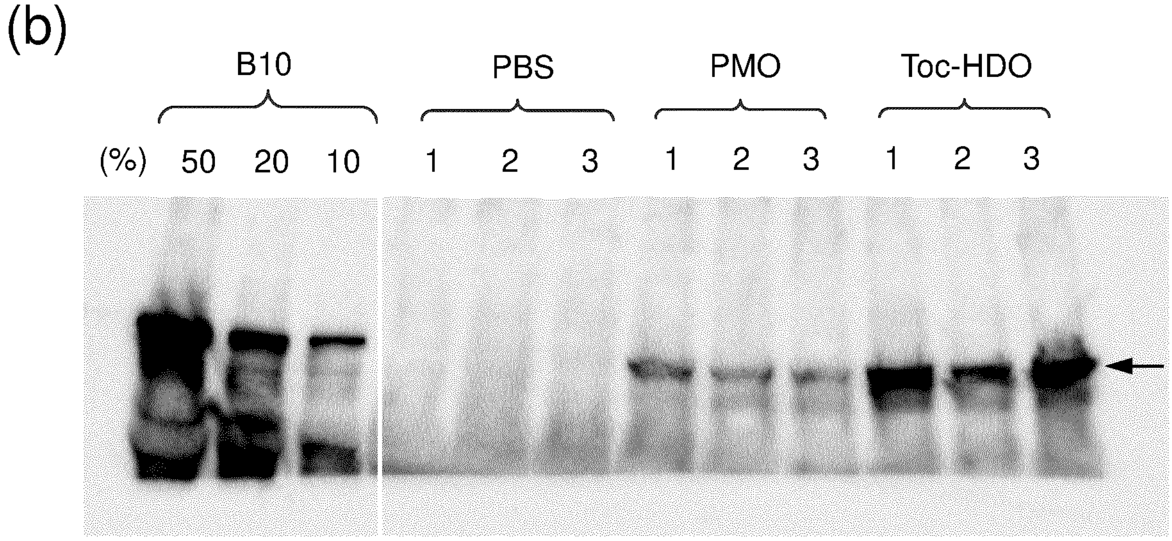
(b)
B10
PBS
PMO
Toc-HDO
(%) 50 20 10 1 2 3 1 2 3 1 2 3

Fig. 24

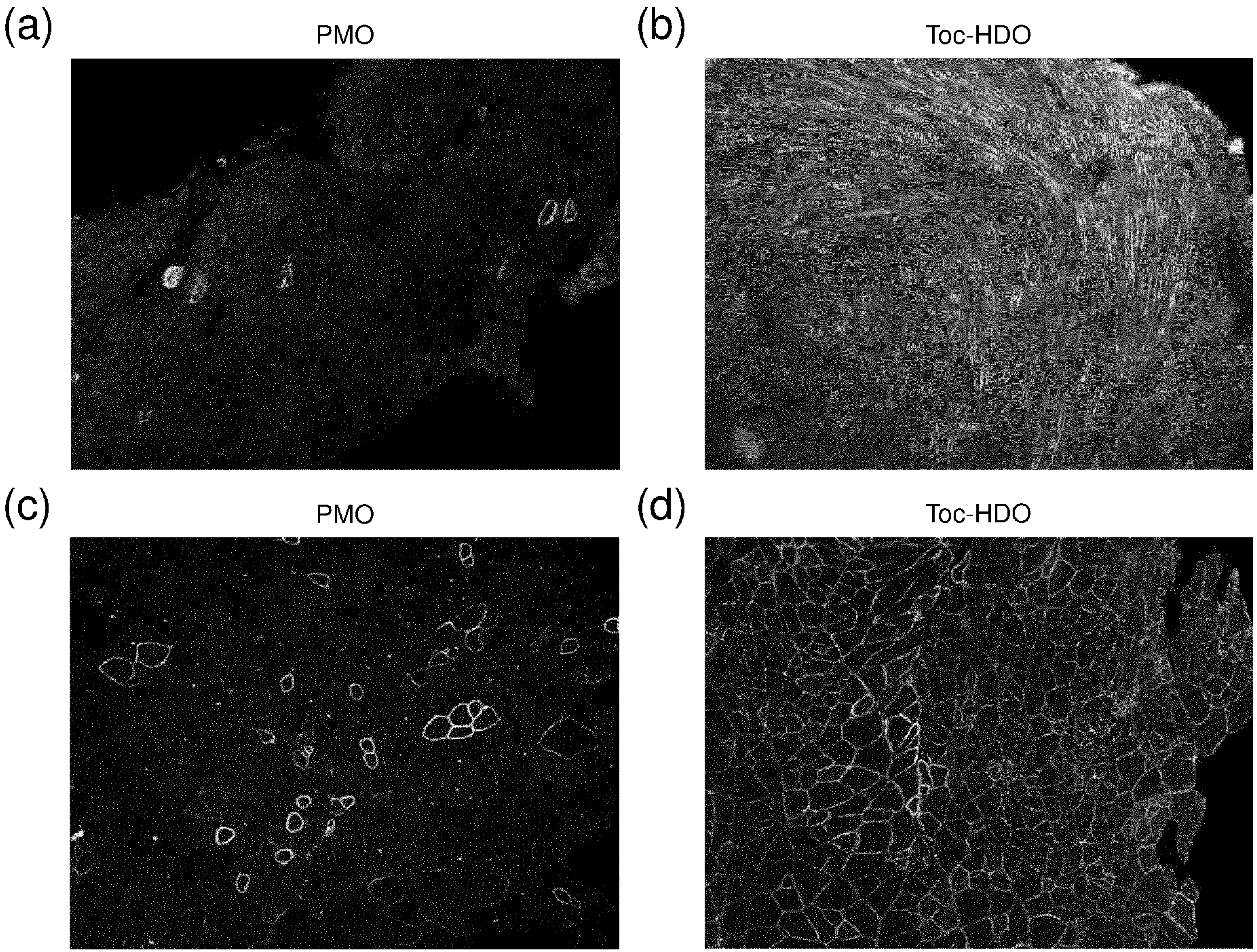

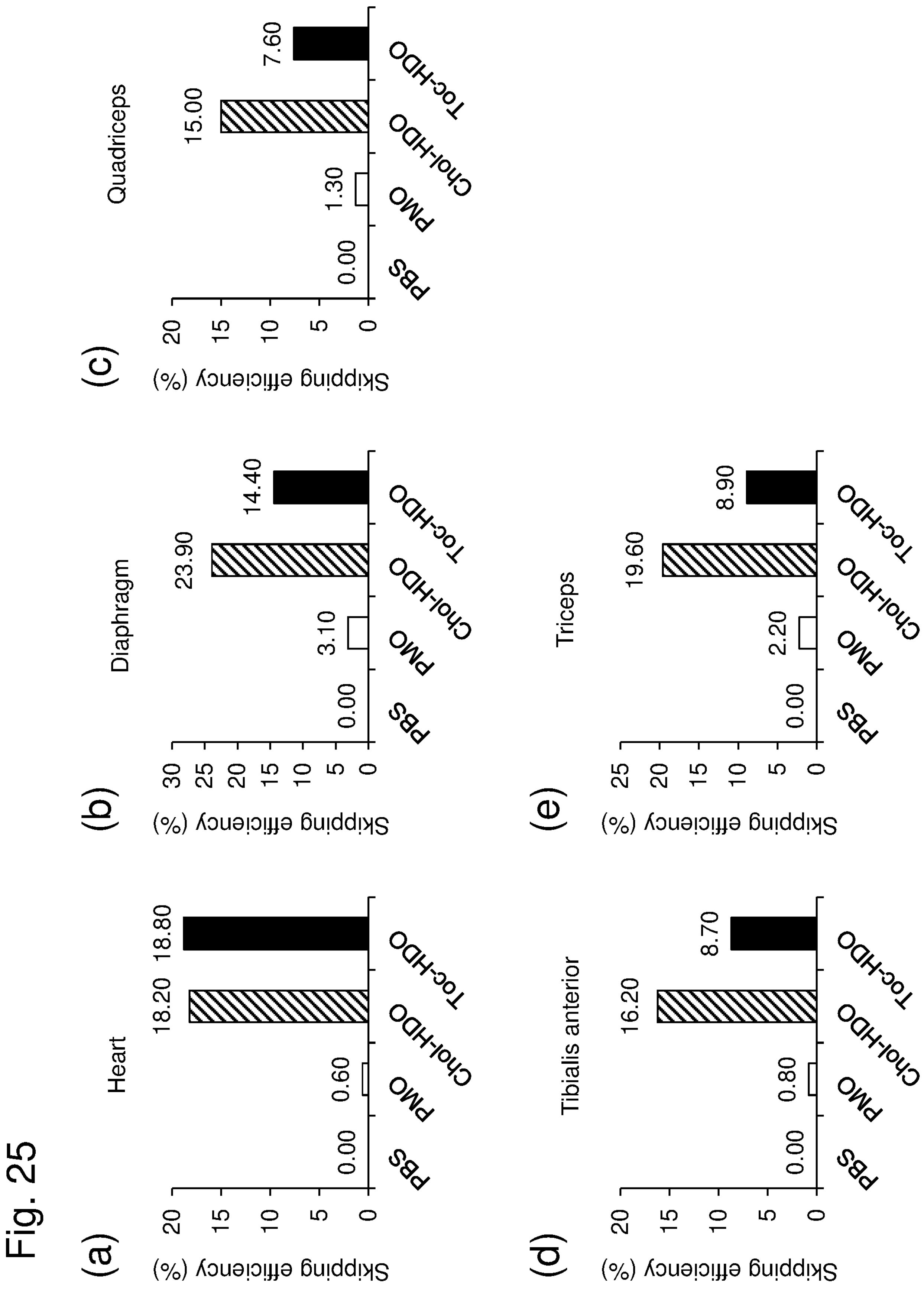
Fig. 25
(a)
Heart
Skipping efficiency (%)
0.00
0.60
18.20
18.80
PBS
PMO
Chol-HDO
Toc-HDO
(b)
Diaphragm
Skipping efficiency (%)
0.00
3.10
23.90
14.40
PBS
PMO
Chol-HDO
Toc-HDO
(c)
Quadriceps
Skipping efficiency (%)
0.00
1.30
15.00
7.60
PBS
PMO
Chol-HDO
Toc-HDO
(d)
Tibialis anterior
Skipping efficiency (%)
0.00
0.80
16.20
8.70
PBS
PMO
Chol-HDO
Toc-HDO
(e)
Triceps
Skipping efficiency (%)
0.00
2.20
19.60
8.90
PBS
PMO
Chol-HDO
Toc-HDO

Fig. 26

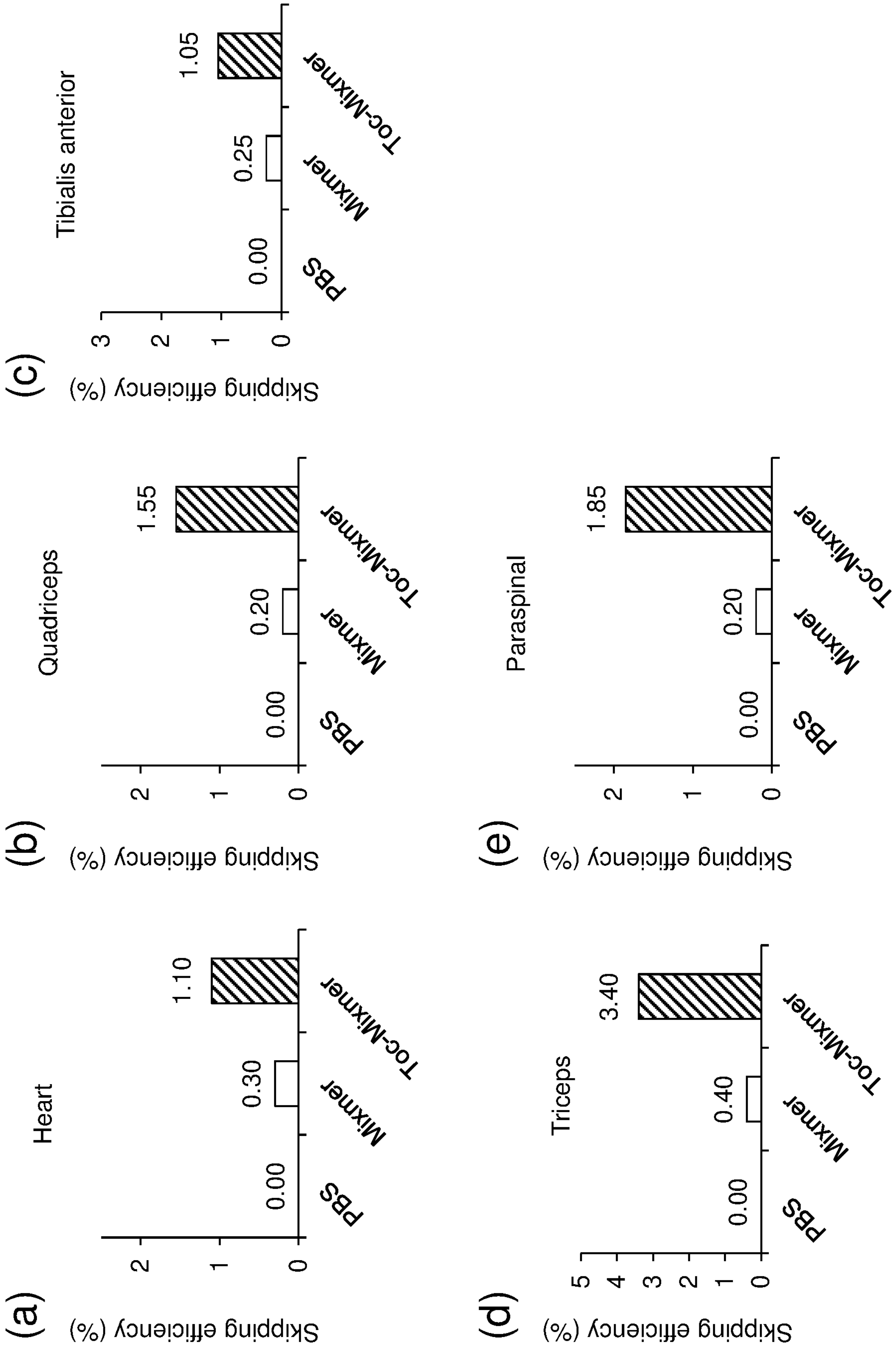
(a)
Heart
Skipping efficiency (%)
0
1
2
0.00
0.30
1.10
PBS
Mixmer
Toc-Mixmer
(b)
Quadriceps
Skipping efficiency (%)
0
1
2
0.00
0.20
1.55
PBS
Mixmer
Toc-Mixmer
(c)
Tibialis anterior
Skipping efficiency (%)
0
1
2
3
0.00
0.25
1.05
PBS
Mixmer
Toc-Mixmer
(d)
Triceps
Skipping efficiency (%)
0
1
2
3
4
5
0.00
0.40
3.40
PBS
Mixmer
Toc-Mixmer
(e)
Paraspinal
Skipping efficiency (%)
0
1
2
0.00
0.20
1.85
PBS
Mixmer
Toc-Mixmer

Fig. 27

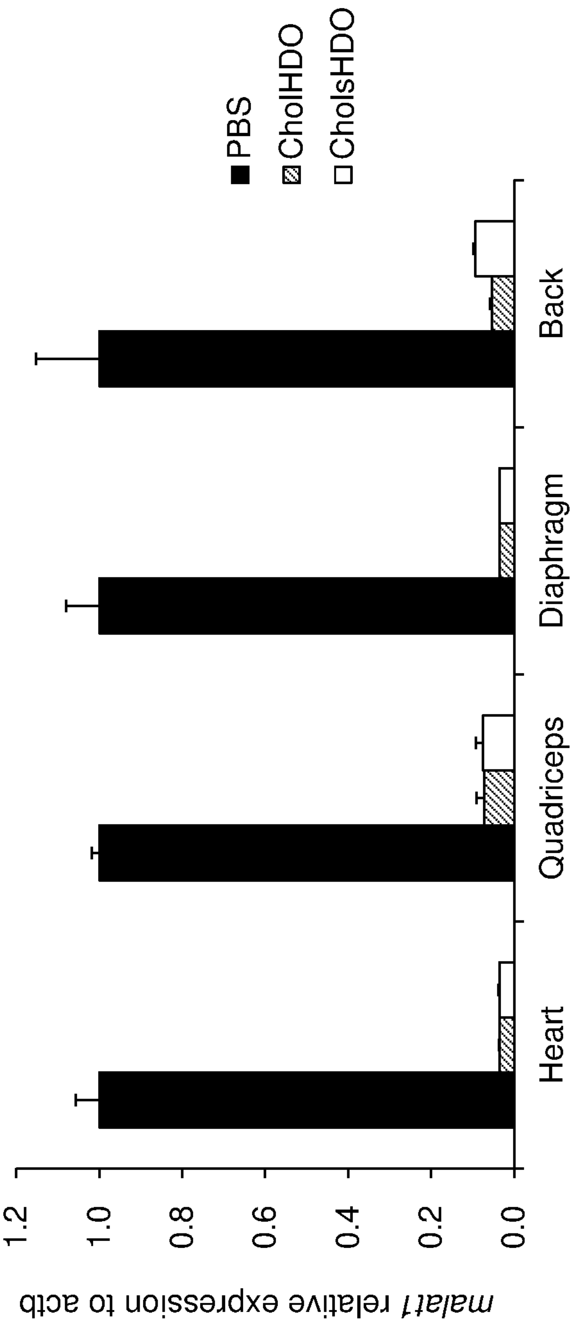
PBS
CholHDO
CholsHDO
1.2
1.0
0.8
0.6
0.4
0.2
0.0
malat1 relative expression to actb
Heart
Quadriceps
Diaphragm
Back

Fig. 28

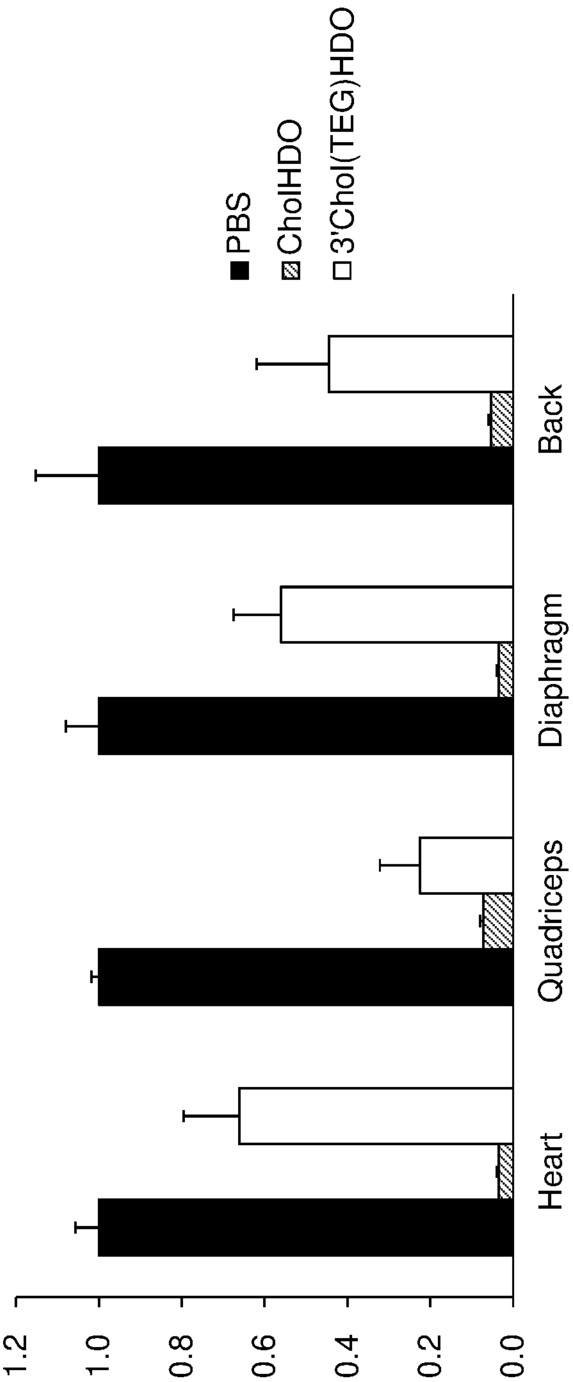
PBS
CholHDO
3'Chol(TEG)HDO
malat1 relative expression to actb
1.2
1.0
0.8
0.6
0.4
0.2
0.0
Heart
Quadriceps
Diaphragm
Back

Fig. 29

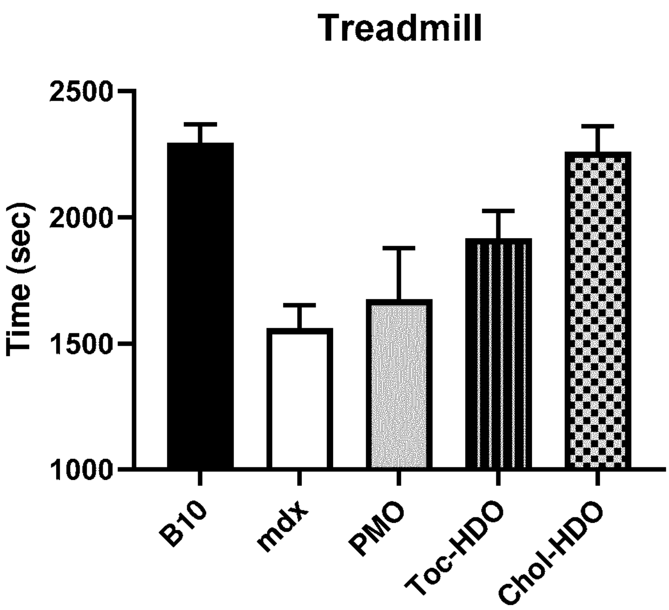
Treadmill
2500
2000
1500
1000
Time (sec)
B10
mdx
PMO
Toc-HDO
Chol-HDO

Fig. 30

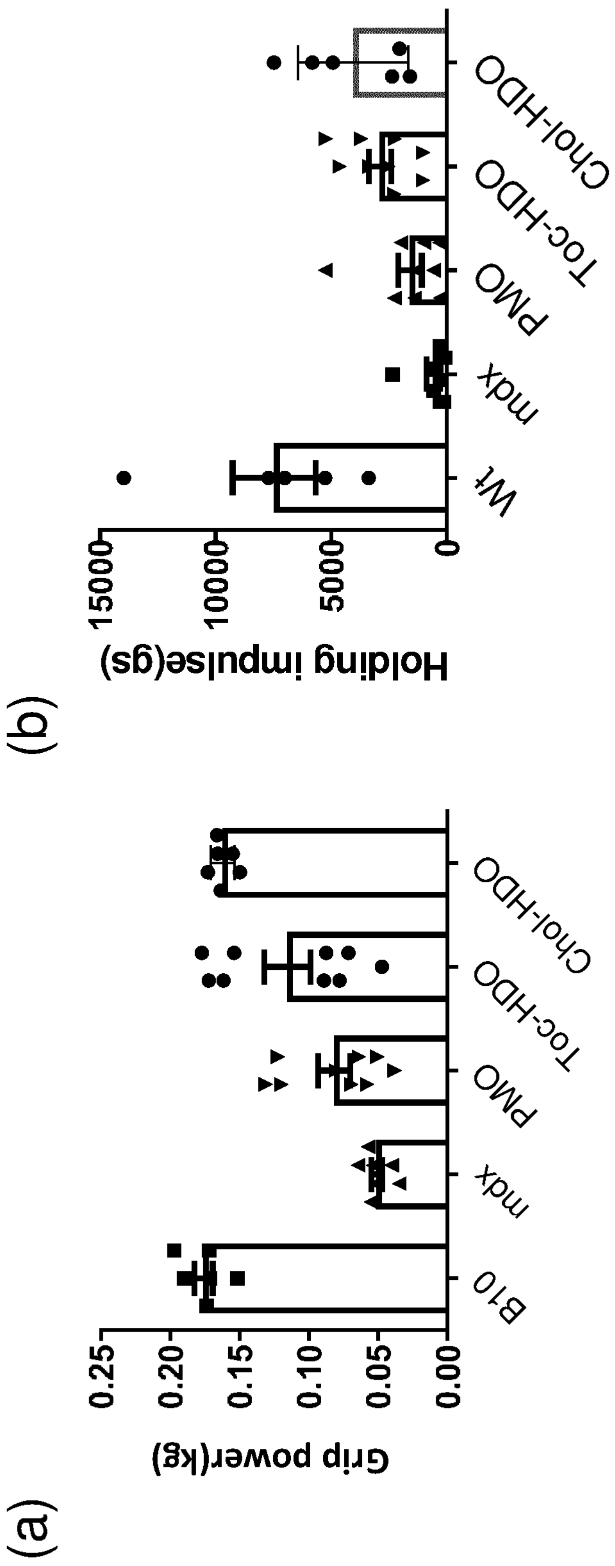
(a)
Grip power(kg)
0.25
0.20
0.15
0.10
0.05
0.00
B10
mdx
PMO
Toc-HDO
Chol-HDO
(b)
Holding impulse(gs)
15000
10000
5000
0
Wt
mdx
PMO
Toc-HDO
Chol-HDO

Fig. 31

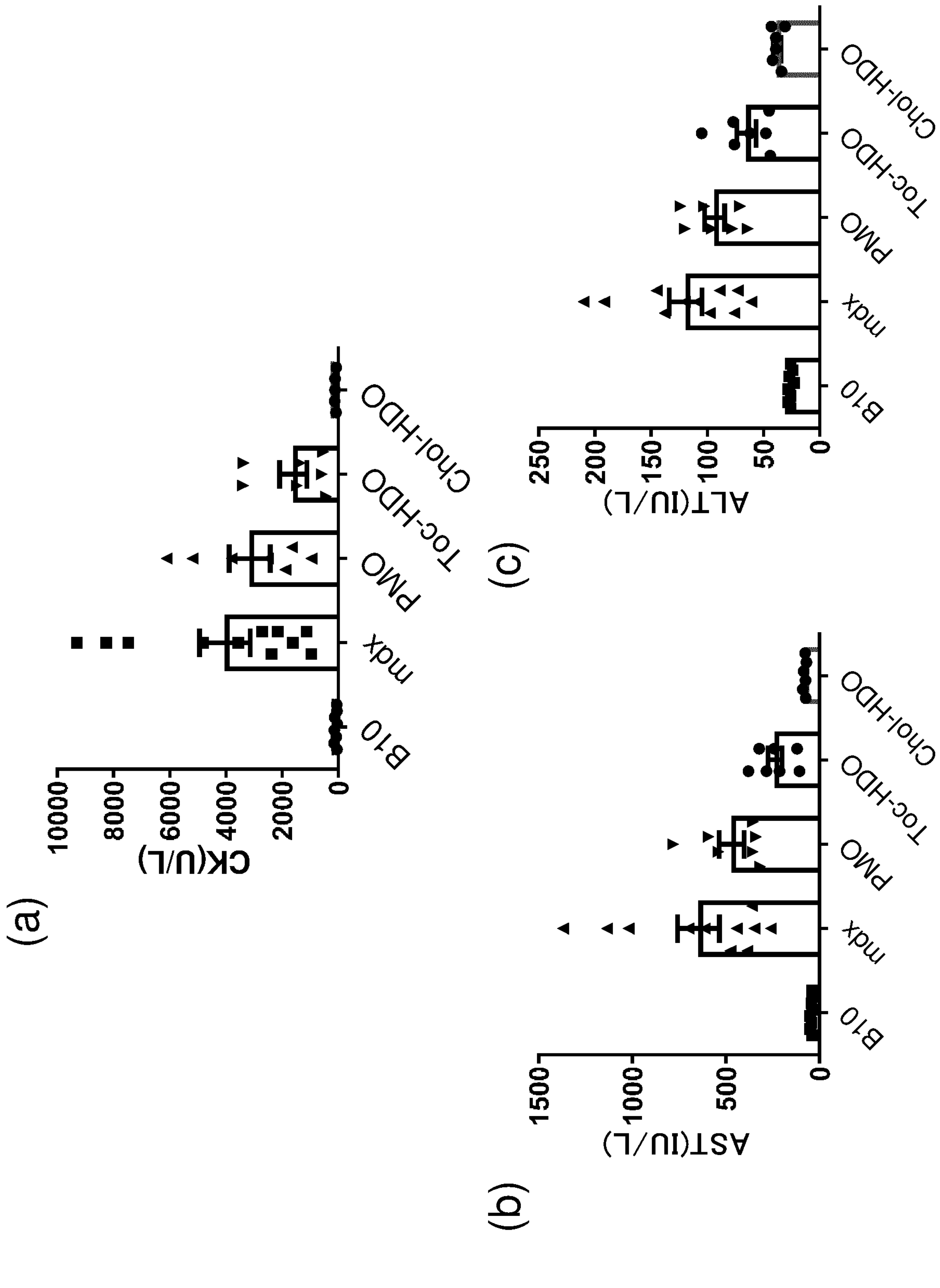
(a)
CK(U/L)
10000
8000
6000
4000
2000
0
B10
mdx
PMO
Toc-HDO
Chol-HDO
(b)
AST(IU/L)
1500
1000
500
0
B10
mdx
PMO
Toc-HDO
Chol-HDO
(c)
ALT(IU/L)
250
200
150
100
50
0
B10
mdx
PMO
Toc-HDO
Chol-HDO

Fig. 32

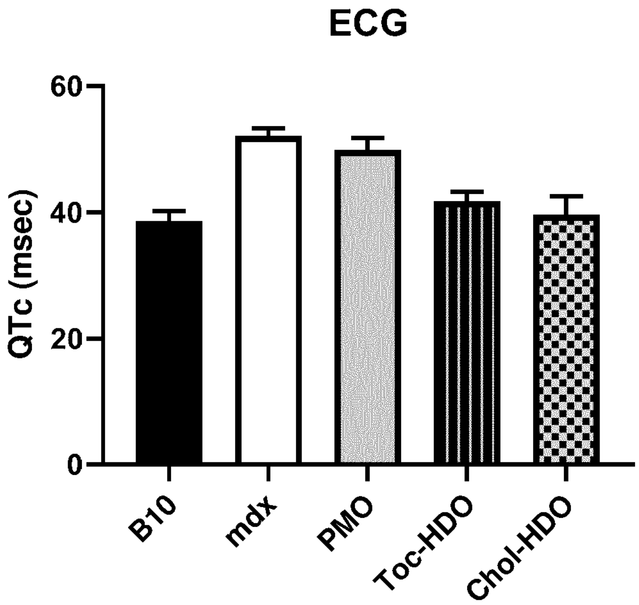

Fig. 33

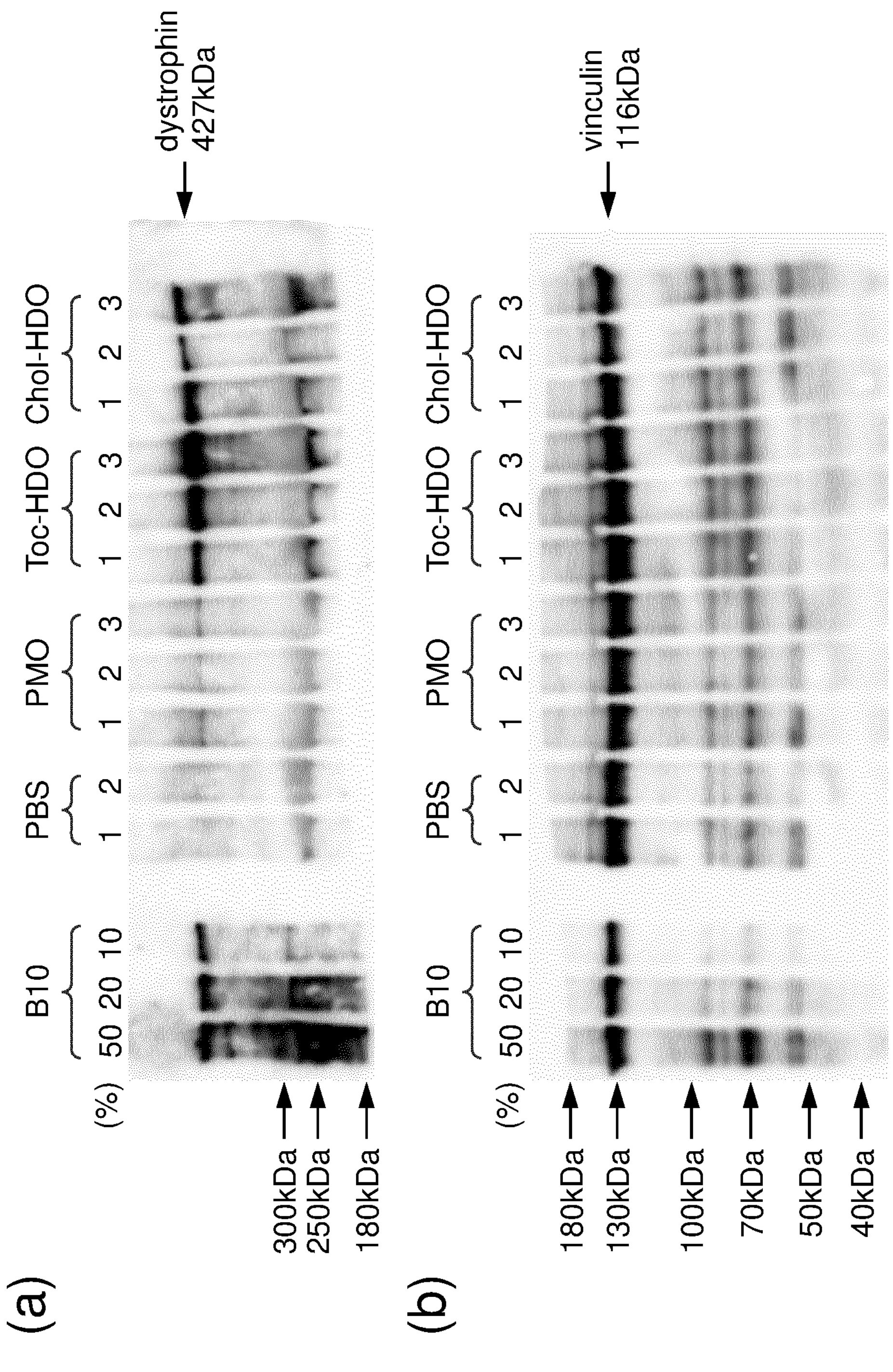
(a)
(%)
B10
50 20 10
PBS
1 2
PMO
1 2 3
Toc-HDO
1 2 3
Chol-HDO
1 2 3
dystrophin
427kDa
300kDa
250kDa
180kDa
(b)
(%)
B10
50 20 10
PBS
1 2
PMO
1 2 3
Toc-HDO
1 2 3
Chol-HDO
1 2 3
vinculin
116kDa
180kDa
130kDa
100kDa
70kDa
50kDa
40kDa

Fig. 34

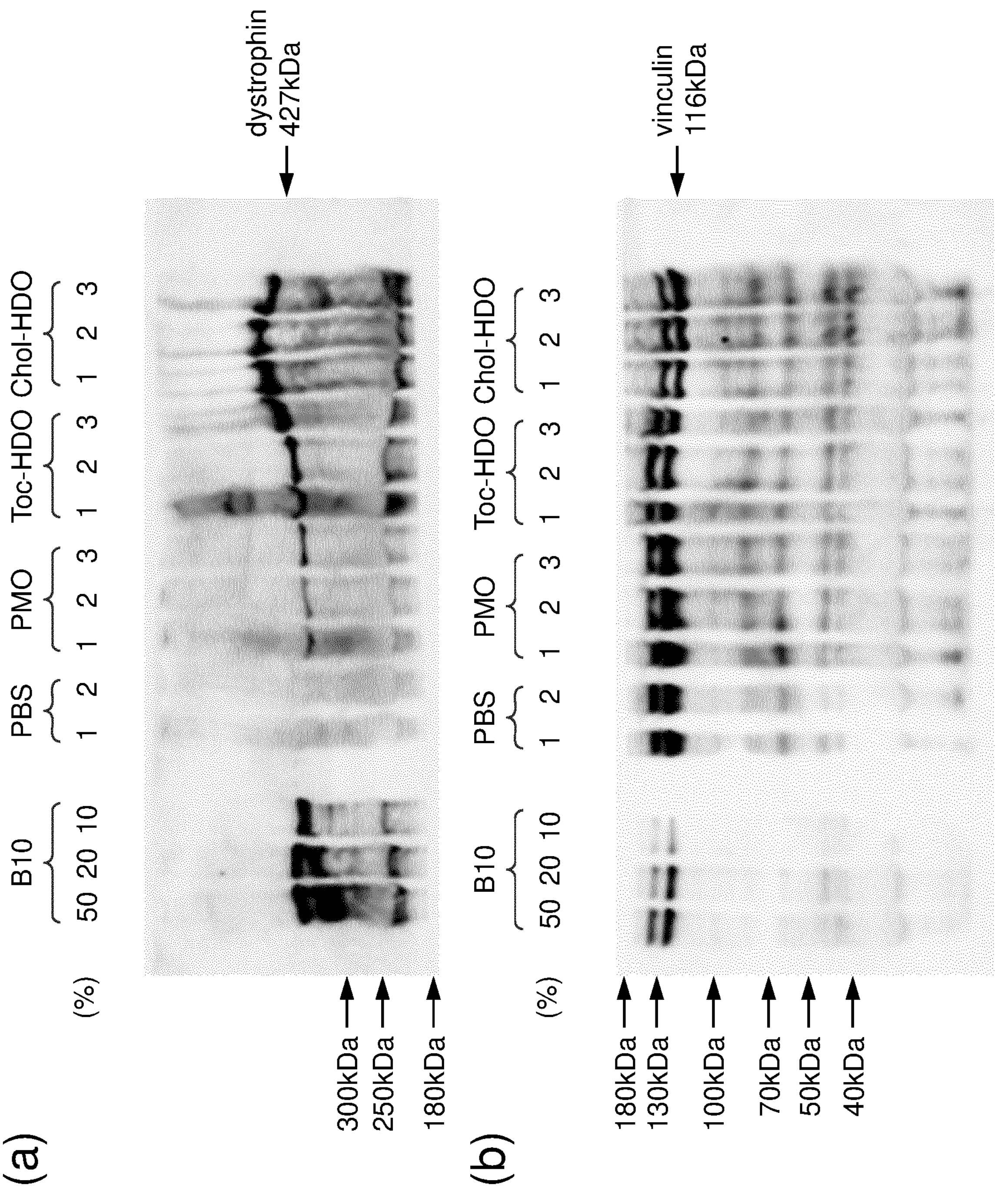
(a)
(%)
B10
50 20 10
PBS
1 2
PMO
1 2 3
Toc-HDO
1 2 3
Chol-HDO
1 2 3
dystrophin
427kDa
300kDa
250kDa
180kDa
(b)
(%)
B10
50 20 10
PBS
1 2
PMO
1 2 3
Toc-HDO
1 2 3
Chol-HDO
1 2 3
vinculin
116kDa
180kDa
130kDa
100kDa
70kDa
50kDa
40kDa

Fig. 35

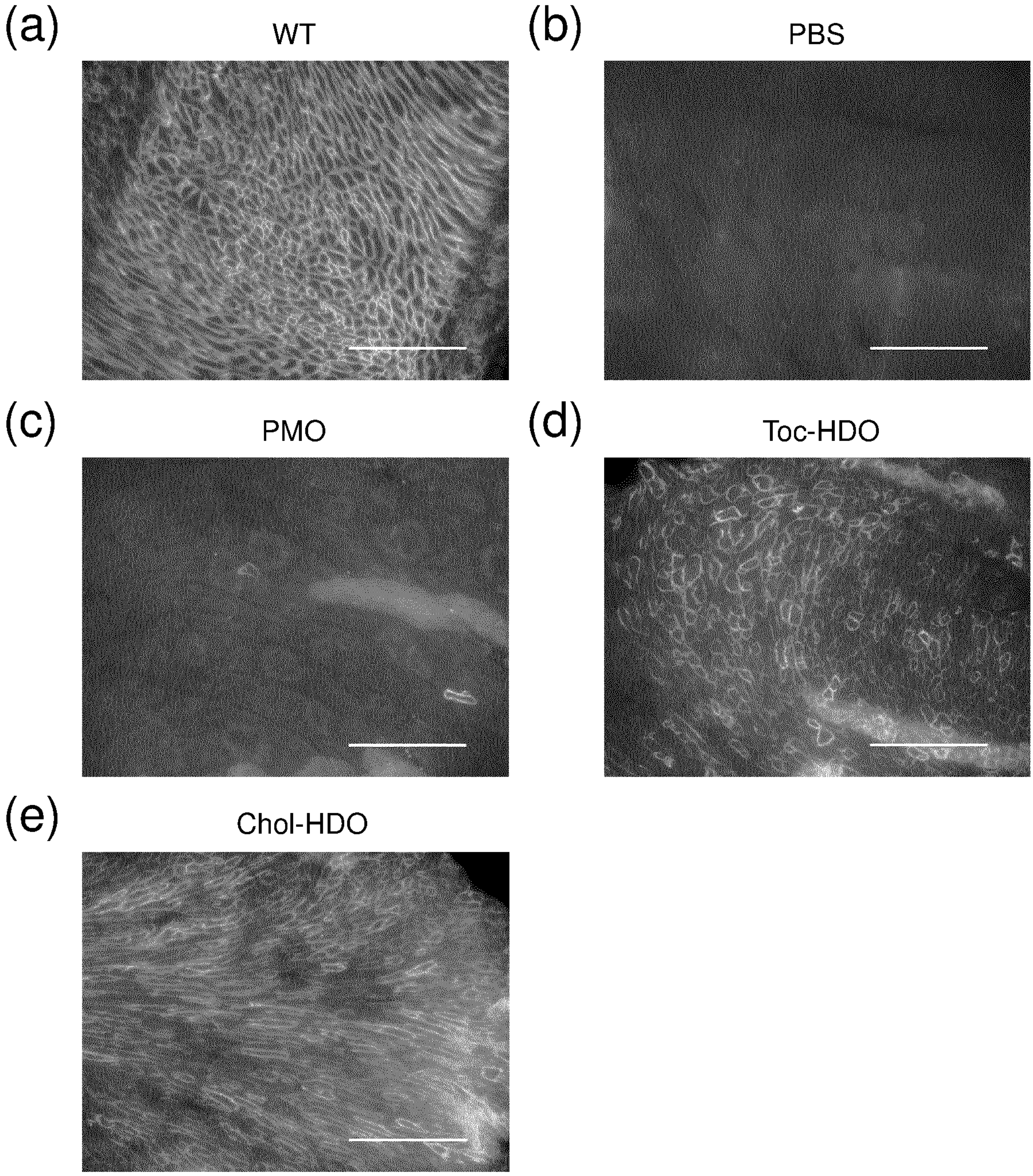

Fig. 36

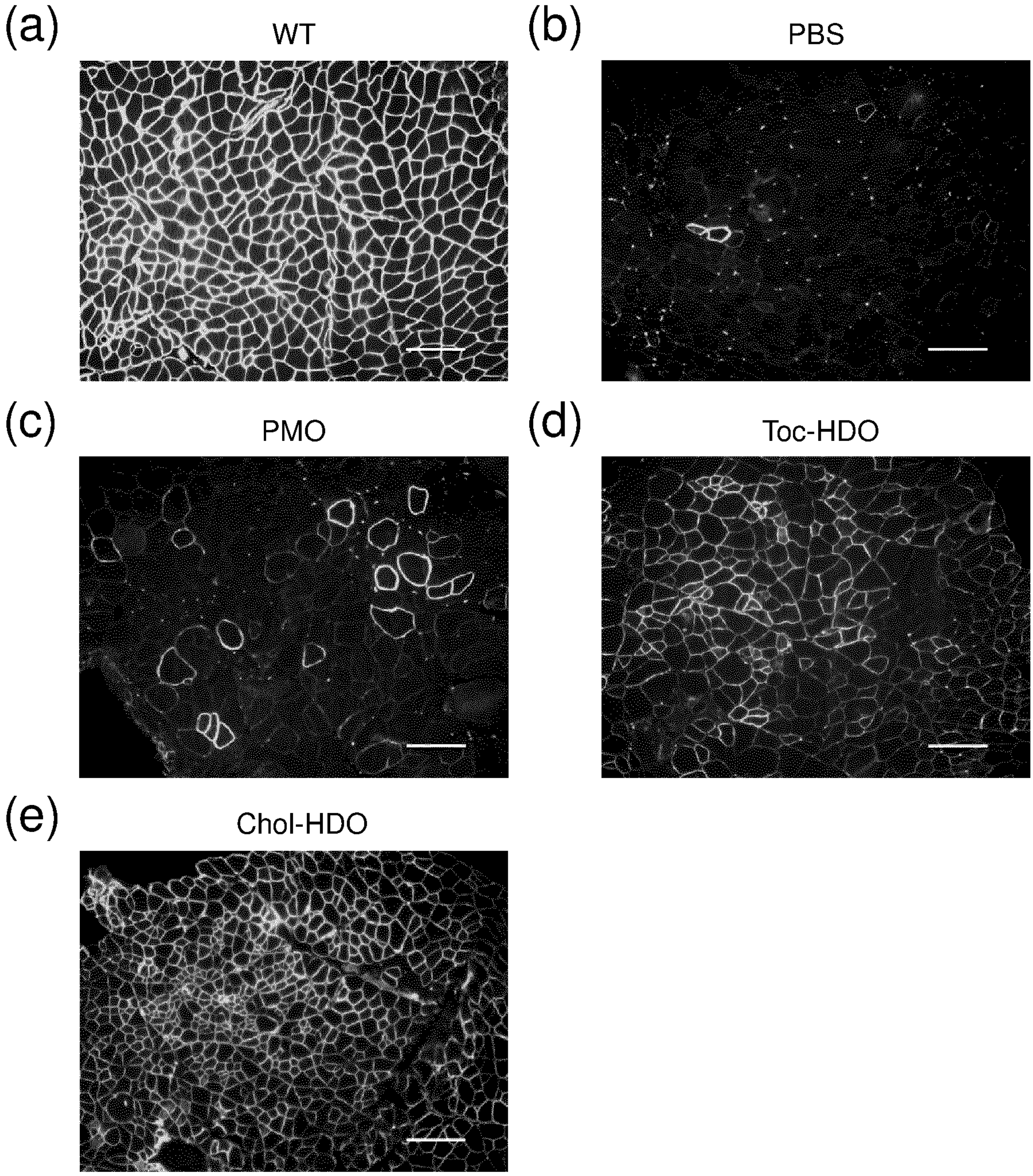

## INTERNATIONAL SEARCH REPORT

International application No.
PCT/JP2020/015818

A. CLASSIFICATION OF SUBJECT MATTER
A61K 48/00(2006.01)i; A61P 3/10(2006.01)i; A61P 7/00(2006.01)i; A61P 9/00(2006.01)i; A61P 9/06(2006.01)i; A61P 21/00(2006.01)i; A61P 21/04(2006.01)i; A61P 25/28(2006.01)i; A61P 29/00(2006.01)i; A61K 47/56(2017.01)i; C12N 15/113(2010.01)i; A61K 31/713(2006.01)i
FI: C12N15/113; A61K48/00; A61K47/56; A61P21/00; A61P9/00; A61P21/04; A61P29/00; A61P7/00; A61P3/10; A61P9/06; A61P25/28; C12N15/113 Z; A61K31/713 ZNA

According to International Patent Classification (IPC) or to both national classification and IPC

B. FIELDS SEARCHED
Minimum documentation searched (classification system followed by classification symbols)
A61K48/00; A61P3/10; A61P7/00; A61P9/00; A61P9/06; A61P21/00; A61P21/04; A61P25/28; A61P29/00; A61K47/56; C12N15/113; A61K31/713

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/REGISTRY /MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | NAGATA, T. et al., "The effect of DNA/RNA heteroduplex oligonucleotides on muscle", Journal of the Neurological Sciences, 2017, vol. 381, supplement, page 846, 2352, DOI:10.1016/j.jns.2017.08.2383, entire text | 1-40 |
| X<br>Y | WO 2018/056442 A1 (TOKYO MEDICAL AND DENTAL UNIVERSITY) 29.03.2018 (2018-03-29) claims, paragraphs [0052], [0055], [0059], [0061]-[0067], [0071], [0089]-[0092], fig. 1 | 1-21<br>1-40 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 July 2020 (03.07.2020) | 14 July 2020 (14.07.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/015818

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2015-502134 A (TOKYO MEDICAL AND DENTAL UNIVERSITY) 22.01.2015 (2015-01-22) claims, paragraph [0054], examples, fig. 5, etc. | 1-21 |
| Y | JP 2018-530560 A (SAREPTA THERAPEUTICS, INC.) 18.10.2018 (2018-10-18) claims | 40 |
| X<br><br><br>Y | JP 2018-528783 A (IONIS PHARMACEUTICALS, INC.) 04.10.2018 (2018-10-04) claims | 1-2,5,7-8,10, 12-24,26,28-31, 33-39<br>1-40 |
| A | NAGATA, Tetsuya, "Recent progress of DNA/RNA heteroduplex oligonucleotide", Clinical Neurology, 2018, vol. 58, S47, HT-02-3, DOI:10.5692/clinicalneurol.58-supplement-S1, entire text | 1-40 |
| A | NISHINA, Kazutaka et al., "DNA/RNA heteroduplex oligonucleotide for highly efficient gene silencing", Nature Communications, 2015, vol. 6, Article No. 7969, DOI:10.1038/ncomms8969, entire text | 1-40 |
| A | 馬嶋貴正ら，ヘテロ核酸の現状，最新医学， 2018, vol. 73, no. 6, pp. 781-786, entire text, (MAJIMA, Takamasa et al., "DNA/RNA Heteroduplex Oligonucleotide Has a High Efficacy for Gene Slicing", The medical frontline) | 1-40 |
| A | WO 2017/068790 A1 (RENA THERAPEUTICS INC.) 27.04.2017 (2017-04-27) entire text | 1-40 |
| P,X | NAGATA, Tetsuya, "Gene therapy with therapeutic oligonucleotide", Clinical Neurology, May 2019, vol. 59, supplement, S13 NFS-02-1, http://www.neurology-jp.org/Journal/public_pdf/59_supplement_01.pdf, entire text | 1-40 |

Form PCT/ISA/210 (continuation of second sheet) (January 2015)

**INTERNATIONAL SEARCH REPORT**
Information on patent family members

International application No.
PCT/JP2020/015818

| Patent Documents referred in the Report | Publication Date | Patent Family | Publication Date |
|---|---|---|---|
| WO 2018/056442 A1 | 29 Mar. 2018 | US 2019/0247414 A1 claims, paragraphs [0144], [0147], [0151], [0156]-[0162], [0166], [0184]-[0187], fig. 1<br>EP 3517610 A1<br>JP 2018-56442 A | |
| JP 2015-502134 A | 22 Jan. 2015 | US 2014/0302603 A1 claims, examples, fig. 5<br>EP 2791335 A1<br>CA 2859581 A<br>CN 104126010 A<br>WO 2013/089283 A1 | |
| JP 2018-530560 A | 18 Oct. 2018 | US 2019/0177723 A1 claims<br>WO 2017/062835 A2<br>EP 3359668 A2<br>CN 108699555 A<br>BR 112018007066 A | |
| JP 2018-528783 A | 04 Oct. 2018 | US 2018/0256729 A1 claims<br>WO 2017/053999 A1<br>EP 3353306 A1 | |
| WO 2017/068790 A1 | 27 Apr. 2017 | US 2018/0223280 A1 whole document<br>EP 3366773 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013089283 A
- WO 2014203518 A
- WO 2014192310 A
- JP 2019073832 A
- WO 2007143315 A
- WO 2008043753 A
- WO 2008049085 A


### Non-patent literature cited in the description


- Principle of Myology. Nankodo Co., Ltd, 1995, 469-550

SEQUENCE LISTING

<110> National University Corporation Tokyo Medical and Dental University

<120> Pharmaceutical Composition for Treating Muscle Disease

<130> PH-8312-PCT

<150> JP 2019-073832
<151> 2019-04-08

<160> 30

<170> PatentIn version 3.5

<210> 1
<211> 2534
<212> DNA
<213> Mus musculus

<220>
<223> SR-B1 mRNA

<400> 1
ggaatcccgc gccgaactcg ggggcgggct gcccgggcca tggcgcataa agcctctggc 60

cacctgcagg gctactgctg ctccggccac cgccaggcac acaccttgct gctgagggag 120

tctcggcttc tgtcatctct gtggcctccg tcacctctgt ctccgtctcc ttcaggtcct 180

gagccccgag agccccttcc gcgcacgcgg acatgggcgg cagctccagg gcgcgctggg 240

tggccttggg gttgggcgcc ctggggctgc tgtttgctgc gctcggcgtt gtcatgatcc 300

tcatggtgcc ctccctcatc aagcagcagg tgctcaagaa tgtccgcata gacccgagca 360

gcctgtcctt cgggatgtgg aaggagatcc ccgtcccttt ctacttgtct gtctacttct 420

tcgaagtggt caacccaaac gaggtcctca acggccagaa gccagtagtc cgggagcgtg 480

gaccctatgt ctacagggag ttcagacaaa aggtcaacat caccttcaat gacaacgaca 540

ccgtgtcctt cgtggagaac cgcagcctcc atttccagcc tgacaagtcg catggctcag 600

agagtgacta cattgtactg cctaacatct tggtcctggg gggctcgata ttgatggaga 660

gcaagcctgt gagcctgaag ctgatgatga ccttggcgct ggtcaccatg ggccagcgtg 720

cttttatgaa ccgcacagtt ggtgagatcc tgtggggcta tgacgatccc ttcgtgcatt 780

ttctcaacac gtacctccca gacatgcttc ccataaaggg caaatttggc ctgtttgttg 840

ggatgaacaa ctcgaattct ggggtcttca ctgtcttcac gggcgtccag aatttcagca 900

ggatccatct ggtggacaaa tggaacggac tcagcaagat cgattattgg cattcagagc 960

agtgtaacat gatcaatggg acttccgggc agatgtgggc acccttcatg acacccgaat 1020

cctcgctgga attcttcagc ccggaggcat gcaggtccat gaagctgacc tacaacgaat 1080

caagggtgtt tgaaggcatt cccacgtatc gcttcacggc ccccgatact ctgtttgcca 1140

acgggtccgt ctacccaccc aacgaaggct tctgcccatg ccgagagtct ggcattcaga 1200

atgtcagcac ctgcaggttt ggtgcgcctc tgtttctctc ccaccccccac ttttacaacg 1260

ccgaccctgt gttgtcagaa gctgttcttg gtctgaaccc taacccaaag gagcattcct 1320

tgttcctaga catccatccg gtcactggga tccccatgaa ctgttctgtg aagatgcagc 1380

tgagcctcta catcaaatct gtcaagggca tcgggcaaac agggaagatc gagccagtag 1440

ttctgccgtt gctgtggttc gaacagagcg gagcaatggg tggcaagccc ctgagcacgt 1500

tctacacgca gctggtgctg atgccccagg ttcttcacta cgcgcagtat gtgctgctgg 1560

ggcttggagg cctcctgttg ctggtgccca tcatctgcca actgcgcagc caggagaaat 1620

gctttttgtt ttggagtggt agtaaaaagg gctcccagga taaggaggcc attcaggcct 1680

actctgagtc cctgatgtca ccagctgcca agggcacggt gctgcaagaa gccaagctat 1740

agggtcctga agacactata agccccccaa acctgatagc ttggtcagac cagccaccca 1800

gtccctacac cccgcttctt gaggactctc tcagcggaca gcccaccagt gccatggcct 1860

gagcccccag atgtcacacc tgtccgcacg cacggcacat ggatgcccac gcatgtgcaa 1920

aaacaactca gggaccaggg acagacctgc tgccaagtga gcctgatggg ccacaggtgt 1980

gctcttctaa atggcctgtg agccaggctg tgggaactct agctgctgtc agcccctcct 2040

gtaggagctg gccctgccca ggctcctgac ttccctcagg aagtctttct gtctttctcc 2100

atcagtctga aagccttagt tcccacagag gacggatctg tcactcctag ggctgggca 2160

tatgtcggcc tcttgtgcca aggccaggca agcagctcca ggtcctgacc agtttgcaca 2220

cacactctgg agctgtatct ggcgcttttt ctatcgtctc tgctatgtca ctgaattaac 2280

cactgtacgt ggcagaggtg gcaggcccct cagggtcctt atttttcagg catggggtca 2340

aagctagagg tatgggccgt ctacaccccc ccgccccccg gcatctagtg tacctcacca 2400

gagggtattc ggaggcccag catcctgcaa ccgacccctt ttttctactg gaagagaaat 2460

tttatcatct tttgaaagga agtcatgact gaagcaataa accttttcac tgattcaaca 2520

aaaaaaaaaa aaaa 2534

<210> 2
<211> 2759
<212> DNA
<213> Homo sapiens

<220>
<223> SR-B1 mRNA

<400> 2
gctcaggccc cgcccctgcc gccggaatcc tgaagcccaa ggctgcccgg gggcggtccg 60

gcggcgccgg cgatggggca taaaaccact ggccacctgc cgggctgctc ctgcgtgcgc 120

tgccgtcccg gatccaccgt gcctctgcgg cctgcgtgcc cggagtcccc gcctgtgtcg 180

```
tctctgtcgc cgtccccgtc tcctgccagg cgcggagccc tgcgagccgc gggtgggccc      240
caggcgcgca gacatgggct gctccgccaa agcgcgctgg gctgccgggg cgctgggcgt      300
cgcggggcta ctgtgcgctg tgctgggcgc tgtcatgatc gtgatggtgc cgtcgctcat      360
caagcagcag gtccttaaga acgtgcgcat cgaccccagt agcctgtcct tcaacatgtg      420
gaaggagatc cctatcccct tctatctctc cgtctacttc tttgacgtca tgaaccccag      480
cgagatcctg aagggcgaga agccgcaggt gcgggagcgc gggccctacg tgtacaggga      540
gttcaggcac aaaagcaaca tcaccttcaa caacaacgac accgtgtcct tcctcgagta      600
ccgcaccttc cagttccagc cctccaagtc ccacggctcg gagagcgact acatcgtcat      660
gcccaacatc ctggtcttgg gtgcggcggt gatgatggag aataagccca tgaccctgaa      720
gctcatcatg accttggcat tcaccaccct cggcgaacgt gccttcatga accgcactgt      780
gggtgagatc atgtggggct acaaggaccc ccttgtgaat ctcatcaaca agtactttcc      840
aggcatgttc cccttcaagg acaagttcgg attatttgct gagctcaaca actccgactc      900
tgggctcttc acggtgttca cgggggtcca gaacatcagc aggatccacc tcgtggacaa      960
gtggaacggg ctgagcaagg ttgacttctg gcattccgat cagtgcaaca tgatcaatgg     1020
aacttctggg caaatgtggc cgcccttcat gactcctgag tcctcgctgg agttctacag     1080
cccggaggcc tgccgatcca tgaagctaat gtacaaggag tcaggggtgt ttgaaggcat     1140
ccccacctat cgcttcgtgg ctcccaaaac cctgtttgcc aacgggtcca tctacccacc     1200
caacgaaggc ttctgcccgt gcctggagtc tggaattcag aacgtcagca cctgcaggtt     1260
cagtgccccc ttgtttctct cccatcctca cttcctcaac gctgacccgg ttctggcaga     1320
agcggtgact ggcctgcacc ctaaccagga ggcacactcc ttgttcctgg acatccaccc     1380
ggtcacggga atccccatga actgctctgt gaaactgcag ctgagcctct acatgaaatc     1440
tgtcgcaggc attggacaaa ctgggaagat tgagcctgtg gtcctgccgc tgctctggtt     1500
tgcagagagc ggggccatgg agggggagac tcttcacaca ttctacactc agctggtgtt     1560
gatgcccaag gtgatgcact atgcccagta cgtcctcctg gcgctgggct gcgtcctgct     1620
gctggtccct gtcatctgcc aaatccggag ccaagagaaa tgctatttat tttggagtag     1680
tagtaaaaag ggctcaaagg ataaggaggc cattcaggcc tattctgaat ccctgatgac     1740
atcagctccc aagggctctg tgctgcagga agcaaaactg tagggtcctg aggacaccgt     1800
gagccagcca ggcctggccg ctgggcctga ccggcccccc agcccctaca ccccgcttct     1860
cccggactct cccagcggac agcccccag ccccacagcc tgagcctccc agctgccatg     1920
tgcctgttgc acacctgcac acacgccctg gcacacatac acacatgcgt gcaggcttgt     1980
gcagacactc agggatggag ctgctgctga agggacttgt agggagaggc tcgtcaacaa     2040
```

gcactgttct ggaaccttct ctccacgtgg cccacaggcc tgaccacagg ggctgtgggt 2100

cctgcgtccc cttcctcggg tgagcctggc ctgtcccgtt cagccgttgg gcccaggctt 2160

cctcccctcc aaggtgaaac actgcagtcc cggtgtggtg gctccccatg caggacgggc 2220

caggctggga gtgccgcctt cctgtgccaa attcagtggg gactcagtgc ccaggccctg 2280

gccacgagct ttggccttgg tctacctgcc aggccaggca aagcgccttt acacaggcct 2340

cggaaaacaa tggagtgagc acaagatgcc ctgtgcagct gcccgagggt ctccgcccac 2400

cccggccgga ctttgatccc cccgaagtct tcacaggcac tgcatcgggt tgtctggcgc 2460

ccttttcctc cagcctaaac tgacatcatc ctatggactg agccggccac tctctggccg 2520

aagtggccgc aggctgtgcc cccgagctgc ccccaccccc tcacagggtc cctcagatta 2580

taggtgccca ggctgaggtg aagaggcctg gggccctgc cttccgggcg ctcctggacc 2640

ctggggcaaa cctgtgaccc ttttctactg gaatagaaat gagttttatc atctttgaaa 2700

aataattcac tcttgaagta ataaacgttt aaaaaaatgg gaaaaaaaaa aaaaaaaaa 2759

<210> 3
<211> 6982
<212> DNA
<213> Mus musculus

<220>
<223> Malat1 mRNA

<400> 3
aggcattcag gcagcgagag cagagcagcg tagagcagca cagctgagct cgtgaggcag 60

gagactcagc ccgaggaaat cgcagataag tttttaatta aaaagattga gcagtaaaaa 120

gaattagaac tctaaactta agctaataga gtagcttatc gaaatattac ttagtcttaa 180

taatctaaga agatcttaag agataacatg aaggcttatt taaacagttt gaaaaaggaa 240

atgaggagaa aagtatttgt actgtataat ggaggctgac cagagcagtt taggagattg 300

taaagggagg ttttgtgaag ttctaaaagg ttctagtttg aaggtcggcc ttgtagatta 360

aaacgaaggt tacctaaata gaatctaagt ggcatttaaa acagtaaagt tgtagagaat 420

agtttgaaaa tgaggtgtag ttttaaaaga ttgagaaaag taggttaagt tgacggccgt 480

tataaaaatc cttcgactgg cgcatgtacg tttgaaggca tgagttggaa acagggaaga 540

tggaagtgtt aggctagccg ggcgatggtg gcgcacgcct ttaatcctag cacttgggag 600

gcagaggcag gcggatttct gagttcgagg ccagcctggt ctacagagtg agttccagga 660

cagccagggc tacacagaga aaccctgtct tgaaaaaaca aaaaggttag gctagtattt 720

ggagaaagaa gattagaaaa tggaagtgaa agacgaagaa gacatacagg aaggtgaaga 780

aaaagctgtt agagaagata ggaaaataga agacaaagca tctttagaag acagaaaagg 840

tacttaaagg cacaggtagt aggaagccga agaatagaag atagaaagaa gcaagataga 900

aaaacaaaat ggaagttaag acaactttgg atgccagcat tcaagatagg caaagaagat 960
aagattgagg ccaaaaggtt ggataagata taaagtcaga aggaaattat ctttaaagcc 1020
ataagttcaa atttctgatg gagcgagcag tttagaagag tctttagaca gccacataca 1080
agattgaagc tagcaatcaa agctactagg actgaagtaa aaagttaagg cagaatgcct 1140
ttgaagagtt agaagaatat taaaagcctt aacttgtagc ttaattttgc ttgatgacaa 1200
aaggactttt gataacagtt tcaagattgt cagcattttg cattggactt gagctgaggt 1260
gcttttaaaa tcctaacgac tagcattggc agctgaccca ggtctacaca gaagtgcatt 1320
cagtgaacta ggaagacagg agcggcagac aggagtcccg aagccagttt ggtgaagcta 1380
ggaaggactg aggagccagc agcagcagtg catggtgaag atagcccagg aaagagtgcg 1440
gttcggtgga ggaagctagg aagaaggagc catacggatg tggtggtgaa gctgggaaag 1500
ggttccagga tggtggagcg agagcgagtt ggtgatgaag ctagctggcg gcttggcttg 1560
tcaactgcgc ggaggaggcg agcaggcatt gtggagagga tagatagcgg ctcctagacc 1620
agcatgccag tgtgcaagaa aggctgcagg gagagcatgc ggtgcggtaa cattccttga 1680
ggtcggcaac atggtggtgg ttttctgtaa cttggatggt aacttgttta ctttgtctta 1740
atagttatgg gggagttgta ggcttctgtg taaagagata tatctggggc tgtatgtagg 1800
cctttgcggg tgttgtaggt tttctttttt cagggttatg tcctcttgca tcttgtcaga 1860
agcttttgag ggctgactgc caaggcccag aaagaagaat ggtagatggc aagttgtctt 1920
taaccgctca gaggggaatg aatggtagag ccagcacaac ctcccagttt tgtaagacgt 1980
tgtagtttga acagatgacc taccacaagc ctcactcctg tgtaggggag gtaattgggc 2040
aaagtgcttt tgggggaatg gggcaaaat atattttgag ttcttttccc cttaggtctg 2100
tctagaatcc taaaggcaga tgactcaagg gaaccagaaa aaaggaaatc cactctcagg 2160
ataagcagag ctcgccaggt ttacagtttg taggaagtag aggatggatg ctagctttca 2220
cactgagtgt ggaggagctg gccatggcgg aattgctggt agtttactct ttccccctcc 2280
cttaatgaga tttgtaaaat cctaaacact tttacttgaa atatttggga gtggtcttaa 2340
cagggaggag tgggtggggg aaacgttttt tttctaagat tttccacaga tgctatagtt 2400
gtgttgacac actgggttag agaaggcgtg tactgctatg ctgttggcac gacaccttca 2460
gggactggag ctgccttttg tccttggaag agttttccca gttgccgctg aagtcagcac 2520
agtgcggctt tggttcacag tcacctcagg agaacctcag gagcttggct aggccagagg 2580
ttgaagttaa gttttacagc accgtgattt aaaatatttc attaaagggg aggggtaaaa 2640
cttagttggc tgtggccttg tgtttgggtg ggtgggggtg ttaggtaatt gtttagttta 2700
tgatttcaga taatcatacc agagaactta aatatttgga aaaacaggaa atctcagctt 2760

tcaagttggc aagtaactcc caatccagtt tttgcttctt ttttcctttt tcttttttttg 2820

aggcgggcag ctaaggaagg ttggttcctc tgccggtccc tcgaaagcgt agggcttggg 2880

ggttggtctg gtccactggg atgatgtgat gctacagtgg ggactcttct gaagctgttg 2940

gatgaatata gattgtagtg tgtggttctc ttttgaaatt tttttcaggt gacttaatgt 3000

atcttaataa ctactatagg aacaaaggaa gtggctttaa tgaccctgaa ggaatttctt 3060

ctggtgatag cttttatatt atcaagtaag agatactatc tcagttttgt ataagcaagt 3120

ctttttccta gtgtaggaga aatgattttc cttgtgacta aacaagatgt aaaggtatgc 3180

ttttttttctt cttgtgcatt gtatacttgt gtttatttgt aacttataat ttaagaatta 3240

tgataattca gcctgaatgt cttttagagg gtgggctttt gttgatgagg gaggggaaac 3300

cttttttttt ctgtagacct ttttcagata acaccatctg agtcataacc agcctggcag 3360

tgtgatgacg tagatgcaga gggagcagct ccttggtgaa tgagtgataa gtaaaggcag 3420

aaaaaataat gtcatgtctc catggggaat gagcatgagc cagagattgt tcctactgat 3480

gaaaagctgc atatgcaaaa atttaagcaa atgaaagcaa ccagtataaa gttatggcaa 3540

tacctttaaa agttatggct tatctaccaa gctttatcca caaaagtaaa gaattgatga 3600

aaaacagtga agatcaaatg ttcatctcaa aactgctttt acaaaagcag aatagaaatg 3660

aagtgaaaat gctgcattaa gcctggagta aaaagaagct gagcttgttg agatgagtgg 3720

gatcgagcgg ctgcgaggcg gtgcagtgtg ccaatgtttc gtttgcctca gacaggtttc 3780

tcttcataag cagaagagtt gcttcattcc atctcggagc aggaaacagc agactgctgt 3840

tgacagataa gtgtaacttg gatctgcagt attgcatgtt agggatagat aagtgccttt 3900

tttctctttt tccaaaaaga cctgtagagc tgttgaatgt ttgcagctgg cccctcttag 3960

gcagttcaga attttgagta gttttcccat ccagcctctt aaaaattcct aagccttgca 4020

ccgatgggct ttcatgatgg gatagctaat aggcttttgc atcgtaaact tcaacacaaa 4080

agcctacatg attaatgcct actttaatta cattgcttac aagattaagg aatctttatc 4140

ttgaagaccc catgaaaggg atcattatgt gctgaaaatt agatgttcat attgctaaaa 4200

tttaaatgtg ctccaatgta cttgtgctta aaatcattaa attatacaaa ttaataaaat 4260

acttcactag agaatgtatg tatttagaag gctgtctcct tatttaaata aagtcttgtt 4320

tgttgtctgt agttagtgtg ggcaattttg gggggatgtt cttctctaat cttttcagaa 4380

acttgacttc gaacacttaa gtggaccaga tcaggatttg agccagaaga ccgaaattaa 4440

ctttaaggca ggaaagacaa attttattct ccatgcagtg atgagcattt aataattgca 4500

ggcctggcat agaggccgtc taactaagga ctaagtacct taggcaggtg ggagatgatg 4560

gtcagagtaa aaggtaacta catattttgt ttccagaaag tcaggggtct aatttgacca 4620

tggctaaaca tctagggtaa gacacttttc ccccacattt ccaaatatgc atgttgagtt 4680

taaatgctta cgatcatctc atccacttta gccttttgtc acctcacttg agccacgagt 4740
ggggtcaggc atgtgggttt aaagagtttt cctttgcaga gcctcatttc atccttcatg 4800
gagctgctca ggactttgca tataagcgct tgcctctgtc ttctgttctg ctagtgagtg 4860
tgtgatgtga gaccttgcag tgagtttgtt tttcctggaa tgtggaggga gggggggatg 4920
gggcttactt gttctagctt ttttttaca gaccacacag aatgcaggtg tcttgacttc 4980
aggtcatgtc tgttctttgg caagtaatat gtgcagtact gttccaatct gctgctatta 5040
gaatgcattg tgacgcgact ggagtatgat taaagaaagt tgtgtttccc caagtgtttg 5100
gagtagtggt tgttggagga aaagccatga gtaacaggct gagtgttgag gaaatggctc 5160
tctgcagctt taagtaaccc gtgtttgtga ttggagccga gtccctttgc tgtgctgcct 5220
taggtaaatg tttttgttca tttctggtga gggggggttgg gagcactgaa gcctttagtc 5280
tcttccagat tcaacttaaa atctgacaag aaataaatca gacaagcaac attcttgaag 5340
aaattttaac tggcaagtgg aaatgttttg aacagttccg tggtctttag tgcattatct 5400
ttgtgtaggt gttctctctc ccctcccttg gtcttaattc ttacatgcag gaacattgac 5460
aacagcagac atctatctat tcaaggggcc agagaatcca gacccagtaa ggaaaaatag 5520
cccatttact ttaaatcgat aagtgaagca gacatgccat tttcagtgtg gggattggga 5580
agccctagtt ctttcagatg tacttcagac tgtagaagga gcttccagtt gaattgaaat 5640
tcaccagtgg acaaaatgag gacaacaggt gaacgagcct tttcttgttt aagattagct 5700
actggtaatc tagtgttgaa tcctctccag cttcatgctg gagcagctag catgtgatgt 5760
aatgttggcc ttggggtgga ggggtgaggt gggcgctaag cctttttta agatttttca 5820
ggtacccctc actaaaggca ctgaaggctt aatgtaggac agcggagcct tcctgtgtgg 5880
caagaatcaa gcaagcagta ttgtatcgag accaaagtgg tatcatggtc ggttttgatt 5940
agcagtgggg actaccctac cgtaacacct tgttggaatt gaagcatcca aagaaaatac 6000
ttgagaggcc ctgggcttgt tttaacatct ggaaaaaagg ctgtttttat agcagcggtt 6060
accagcccaa acctcaagtt gtgcttgcag gggagggaaa agggggaaag cgggcaacca 6120
gtttccccag cttttccaga atcctgttac aaggtctccc cacaagtgat ttctctgcca 6180
catcgccacc atgggccttt ggcctaatca cagacccttc acccctcacc ttgatgcagc 6240
cagtagctgg atccttgagg tcacgttgca tatcggtttc aaggtaacca tggtgccaag 6300
gtcctgtggg ttgcaccaga aaaggccatc aattttcccc ttgcctgtaa tttaacatta 6360
aaaccatagc taagatgttt tatacatagc acctatgcag agtaaacaaa ccagtatggg 6420
tatagtatgt ttgataccag tgctgggtgg gaatgtagga agtcggatga aaagcaagcc 6480
tttgtaggaa gttgttgggg tgggattgca aaaattctct gctaagactt tttcaggtgg 6540

acataacaga cttggccaag ctagcatctt agtggaagca gattcgtcag tagggttgta 6600

aaggtttttc ttttcctgag aaaacaacct tttgttttct caggttttgc tttttggcct 6660

ttccctagct ttaaaaaaaa aaaagcaaaa gacgctggtg gctggcactc ctggtttcca 6720

ggacggggtt caagtccctg cggtgtcttt gcttgactct tatatcatga ggccattaca 6780

tttttcttgg agggttctaa aggctctggg tatggtagct gatatcactg gaacactccc 6840

cagcctcagt gttgaactct tgataattaa ctgcattgtc tttcaggtta tgcccaattc 6900

gtcttattac ctctgagtcg acacacctcc tactatttat tgaatacttt gattttatga 6960

aataaaaact aaatatctct ca 6982

<210> 4
<211> 8758
<212> DNA
<213> Homo sapiens

<220>
<223> Malat1 mRNA

<400> 4
gtaaaggact ggggccccgc aactggcctc tcctgccctc ttaagcgcag cgccatttta 60

gcaacgcaga agcccggcgc cgggaagcct cagctcgcct gaaggcaggt cccctctgac 120

gcctccggga gcccaggttt cccagagtcc ttgggacgca gcgacgagtt gtgctgctat 180

cttagctgtc cttataggct ggccattcca ggtggtggta tttagataaa accactcaaa 240

ctctgcagtt tggtcttggg gtttggagga aagcttttat ttttcttcct gctccggttc 300

agaaggtctg aagctcatac ctaaccaggc ataacacaga atctgcaaaa caaaaacccc 360

taaaaaagca gacccagagc agtgtaaaca cttctgggtg tgtccctgac tggctgccca 420

aggtctctgt gtcttcggag acaaagccat tcgcttagtt ggtctacttt aaaaggccac 480

ttgaactcgc tttccatggc gatttgcctt gtgagcactt tcaggagagc ctggaagctg 540

aaaaacggta gaaaaatttc cgtgcgggcc gtgggggct ggcggcaact gggggccgc 600

agatcagagt gggccactgg cagccaacgg cccccggggc tcaggcgggg agcagctctg 660

tggtgtggga ttgaggcgtt ttccaagagt gggttttcac gtttctaaga tttcccaagc 720

agacagcccg tgctgctccg atttctcgaa caaaaaagca aaacgtgtgg ctgtcttggg 780

agcaagtcgc aggactgcaa gcagttgggg gagaaagtcc gccattttgc cacttctcaa 840

ccgtccctgc aaggctgggg ctcagttgcg taatggaaag taaagccctg aactatcaca 900

ctttaatctt ccttcaaaag gtggtaaact atacctactg tccctcaaga gaacacaaga 960

agtgctttaa gaggtatttt aaaagttccg gggttttgt gaggtgtttg atgacccgtt 1020

taaaatatga tttccatgtt tcttttgtct aaagtttgca gctcaaatct ttccacacgc 1080

tagtaattta agtatttctg catgtgtagt ttgcattcaa gttccataag ctgttaagaa 1140

aaatctagaa aagtaaaact agaacctatt tttaaccgaa gaactacttt ttgcctccct 1200
cacaaaggcg gcggaaggtg atcgaattcc ggtgatgcga gttgttctcc gtctataaat 1260
acgcctcgcc cgagctgtgc ggtaggcatt gaggcagcca gcgcaggggc ttctgctgag 1320
ggggcaggcg gagcttgagg aaaccgcaga taagtttttt tctctttgaa agatagagat 1380
taatacaact acttaaaaaa tatagtcaat aggttactaa gatattgctt agcgttaagt 1440
ttttaacgta attttaatag cttaagattt taagagaaaa tatgaagact tagaagagta 1500
gcatgaggaa ggaaaagata aaaggtttct aaaacatgac ggaggttgag atgaagcttc 1560
ttcatggagt aaaaaatgta tttaaaagaa aattgagaga aaggactaca gagccccgaa 1620
ttaataccaa tagaagggca atgcttttag attaaaatga aggtgactta aacagcttaa 1680
agtttagttt aaaagttgta ggtgattaaa ataatttgaa ggcgatcttt taaaaagaga 1740
ttaaaccgaa ggtgattaaa agaccttgaa atccatgacg cagggagaat tgcgtcattt 1800
aaagcctagt taacgcattt actaaacgca gacgaaaatg gaaagattaa ttgggagtgg 1860
taggatgaaa caatttggag aagatagaag tttgaagtgg aaaactggaa gacagaagta 1920
cgggaaggcg aagaaaagaa tagagaagat agggaaatta gaagataaaa acatactttt 1980
agaagaaaaa agataaattt aaacctgaaa agtaggaagc agaagaaaaa agacaagcta 2040
ggaaacaaaa agctaagggc aaaatgtaca aacttagaag aaaattggaa gatagaaaca 2100
agatagaaaa tgaaaatatt gtcaagagtt tcagatagaa aatgaaaaac aagctaagac 2160
aagtattgga gaagtataga agatagaaaa atataaagcc aaaaattgga taaaatagca 2220
ctgaaaaaat gaggaaatta ttggtaacca atttatttta aaagcccatc aatttaattt 2280
ctggtggtgc agaagttaga aggtaaagct tgagaagatg agggtgttta cgtagaccag 2340
aaccaattta gaagaatact tgaagctaga aggggaagtt ggttaaaaat cacatcaaaa 2400
agctactaaa aggactggtg taatttaaaa aaaactaagg cagaaggctt ttggaagagt 2460
tagaagaatt tggaaggcct taaatatagt agcttagttt gaaaaatgtg aaggactttc 2520
gtaacggaag taattcaaga tcaagagtaa ttaccaactt aatgtttttg cattggactt 2580
tgagttaaga ttattttta aatcctgagg actagcatta attgacagct gacccaggtg 2640
ctacacagaa gtggattcag tgaatctagg aagacagcag cagacaggat tccaggaacc 2700
agtgtttgat gaagctagga ctgaggagca agcgagcaag cagcagttcg tggtgaagat 2760
aggaaaagag tccaggagcc agtgcgattt ggtgaaggaa gctaggaaga aggaaggagc 2820
gctaacgatt tggtggtgaa gctaggaaaa aggattccag gaaggagcga gtgcaatttg 2880
gtgatgaagg tagcaggcgg cttggcttgg caaccacacg gaggaggcga gcaggcgttg 2940
tgcgtagagg atcctagacc agcatgccag tgtgccaagg ccacagggaa agcgagtggt 3000

tggtaaaaat ccgtgaggtc ggcaatatgt tgtttttctg gaacttactt atggtaacct 3060
tttatttatt ttctaatata atgggggagt ttcgtactga ggtgtaaagg gatttatatg 3120
gggacgtagg ccgatttccg ggtgttgtag gtttctcttt ttcaggctta tactcatgaa 3180
tcttgtctga agcttttgag ggcagactgc caagtcctgg agaaatagta gatggcaagt 3240
ttgtgggttt ttttttttta cacgaatttg aggaaaacca aatgaatttg atagccaaat 3300
tgagacaatt tcagcaaatc tgtaagcagt ttgtatgttt agttggggta atgaagtatt 3360
tcagttttgt gaatagatga cctgttttta cttcctcacc ctgaattcgt tttgtaaatg 3420
tagagtttgg atgtgtaact gaggcggggg ggagttttca gtattttttt ttgtgggggt 3480
gggggcaaaa tatgttttca gttctttttc ccttaggtct gtctagaatc ctaaaggcaa 3540
atgactcaag gtgtaacaga aaacaagaaa atccaatatc aggataatca gaccaccaca 3600
ggtttacagt ttatagaaac tagagcagtt ctcacgttga ggtctgtgga agagatgtcc 3660
attggagaaa tggctggtag ttactctttt ttcccccccac ccccttaatc agactttaaa 3720
agtgcttaac cccttaaact tgttattttt tacttgaagc attttgggat ggtcttaaca 3780
gggaagagag agggtggggg agaaaatgtt tttttctaag attttccaca gatgctatag 3840
tactattgac aaactgggtt agagaaggag tgtaccgctg tgctgttggc acgaacacct 3900
tcagggactg gagctgcttt tatccttgga agagtattcc cagttgaagc tgaaaagtac 3960
agcacagtgc agctttggtt catattcagt catctcagga gaacttcaga agagcttgag 4020
taggccaaat gttgaagtta agttttccaa taatgtgact tcttaaaagt tttattaaag 4080
gggaggggca aatattggca attagttggc agtggcctgt tacggttggg attggtgggg 4140
tgggtttagg taattgttta gtttatgatt gcagataaac tcatgccaga gaacttaaag 4200
tcttagaatg gaaaaagtaa agaaatatca acttccaagt tggcaagtaa ctcccaatga 4260
tttagttttt ttccccccag tttgaattgg gaagctgggg gaagttaaat atgagccact 4320
gggtgtacca gtgcattaat ttgggcaagg aaagtgtcat aatttgatac tgtatctgtt 4380
ttccttcaaa gtatagagct tttggggaag gaaagtattg aactgggggt tggtctggcc 4440
tactgggctg acattaacta caattatggg aaatgcaaaa gttgtttgga tatggtagtg 4500
tgtggttctc ttttggaatt tttttcaggt gatttaataa taatttaaaa ctactataga 4560
aactgcagag caaaggaagt ggcttaatga tcctgaaggg atttcttctg atggtagctt 4620
ttgtattatc aagtaagatt ctattttcag ttgtgtgtaa gcaagttttt ttttagtgta 4680
ggagaaatac ttttccattg tttaactgca aaacaagatg ttaaggtatg cttcaaaaat 4740
tttgtaaatt gtttatttta aacttatctg tttgtaaatt gtaactgatt aagaattgtg 4800
atagttcagc ttgaatgtct cttagagggt gggcttttgt tgatgaggga ggggaaactt 4860
tttttttttc tatagacttt tttcagataa catcttctga gtcataacca gcctggcagt 4920

atgatggcct agatgcagag aaaacagctc cttggtgaat tgataagtaa aggcagaaaa 4980
gattatatgt catacctcca ttggggaata agcataaccc tgagattctt actactgatg 5040
agaacattat ctgcatatgc caaaaaattt taagcaaatg aaagctacca atttaaagtt 5100
acggaatcta ccattttaaa gttaattgct tgtcaagcta taaccacaaa aataatgaat 5160
tgatgagaaa tacaatgaag aggcaatgtc catctcaaaa tactgctttt acaaaagcag 5220
aataaaagcg aaaagaaatg aaaatgttac actacattaa tcctggaata aaagaagccg 5280
aaataaatga gagatgagtt gggatcaagt ggattgagga ggctgtgctg tgtgccaatg 5340
tttcgtttgc ctcagacagg tatctcttcg ttatcagaag agttgcttca tttcatctgg 5400
gagcagaaaa cagcaggcag ctgttaacag ataagtttaa cttgcatctg cagtattgca 5460
tgttagggat aagtgcttat ttttaagagc tgtggagttc ttaaatatca accatggcac 5520
tttctcctga ccccttccct aggggatttc aggattgaga aatttttcca tcgagccttt 5580
ttaaaattgt aggacttgtt cctgtgggct tcagtgatgg gatagtacac ttcactcaga 5640
ggcatttgca tctttaaata atttcttaaa agcctctaaa gtgatcagtg ccttgatgcc 5700
aactaaggaa atttgtttag cattgaatct ctgaaggctc tatgaaagga atagcatgat 5760
gtgctgttag aatcagatgt tactgctaaa atttacatgt tgtgatgtaa attgtgtaga 5820
aaaccattaa atcattcaaa ataataaact atttttatta gagaatgtat acttttagaa 5880
agctgtctcc ttatttaaat aaaatagtgt ttgtctgtag ttcagtgttg ggcaatctt 5940
ggggggatt cttctctaat ctttcagaaa ctttgtctgc gaacactctt taatggacca 6000
gatcaggatt tgagcggaag aacgaatgta actttaaggc aggaaagaca aattttattc 6060
ttcataaagt gatgagcata taataattcc aggcacatgg caatagaggc cctctaaata 6120
aggaataaat aacctcttag acaggtggga gattatgatc agagtaaaag gtaattacac 6180
attttatttc cagaaagtca ggggtctata aattgacagt gattagagta atactttttc 6240
acatttccaa agtttgcatg ttaactttaa atgcttacaa tcttagagtg gtaggcaatg 6300
ttttacacta ttgaccttat atagggaagg gagggggtgc ctgtggggtt ttaaagaatt 6360
ttcctttgca gaggcatttc atccttcatg aagccattca ggattttgaa ttgcatatga 6420
gtgcttggct cttccttctg ttctagtgag tgtatgagac cttgcagtga gtttatcagc 6480
atactcaaaa tttttttcct ggaatttgga gggatgggag gagggggtgg ggcttacttg 6540
ttgtagcttt tttttttttt acagacttca cagagaatgc agttgtcttg acttcaggtc 6600
tgtctgttct gttggcaagt aaatgcagta ctgttctgat cccgctgcta ttagaatgca 6660
ttgtgaaacg actggagtat gattaaaagt tgtgttcccc aatgcttgga gtagtgattg 6720
ttgaaggaaa aaatccagct gagtgataaa ggctgagtgt tgaggaaatt tctgcagttt 6780

taagcagtcg tatttgtgat tgaagctgag tacattttgc tggtgtattt ttaggtaaaa 6840

tgctttttgt tcatttctgg tggtgggagg ggactgaagc ctttagtctt ttccagatgc 6900

aaccttaaaa tcagtgacaa gaaacattcc aaacaagcaa cagtcttcaa gaaattaaac 6960

tggcaagtgg aaatgtttaa acagttcagt gatctttagt gcattgttta tgtgtgggtt 7020

tctctctccc ctcccttggt cttaattctt acatgcagga acactcagca gacacacgta 7080

tgcgaagggc cagagaagcc agacccagta agaaaaaata gcctatttac tttaaataaa 7140

ccaaacattc cattttaaat gtggggattg ggaaccacta gttctttcag atggtattct 7200

tcagactata gaaggagctt ccagttgaat tcaccagtgg acaaaatgag gaaaacaggt 7260

gaacaagctt tttctgtatt tacatacaaa gtcagatcag ttatgggaca atagtattga 7320

atagatttca gctttatgct ggagtaactg gcatgtgagc aaactgtgtt ggcgtggggg 7380

tggaggggtg aggtgggcgc taagcctttt tttaagattt ttcaggtacc cctcactaaa 7440

ggcaccgaag gcttaaagta ggacaaccat ggagccttcc tgtggcagga gagacaacaa 7500

agcgctatta tcctaaggtc aagagaagtg tcagcctcac ctgattttta ttagtaatga 7560

ggacttgcct caactccctc tttctggagt gaagcatccg aaggaatgct tgaagtaccc 7620

ctgggcttct cttaacattt aagcaagctg tttttatagc agctcttaat aataaagccc 7680

aaatctcaag cggtgcttga aggggaggga aagggggaaa gcgggcaacc acttttccct 7740

agcttttcca gaagcctgtt aaaagcaagg tctccccaca agcaacttct ctgccacatc 7800

gccaccccgt gccttttgat ctagcacaga cccttcaccc ctcacctcga tgcagccagt 7860

agcttggatc cttgtgggca tgatccataa tcggtttcaa ggtaacgatg gtgtcgaggt 7920

ctttggtggg ttgaactatg ttagaaaagg ccattaattt gcctgcaaat tgttaacaga 7980

agggtattaa aaccacagct aagtagctct attataatac ttatccagtg actaaaacca 8040

acttaaacca gtaagtggag aaataacatg ttcaagaact gtaatgctgg gtgggaacat 8100

gtaacttgta gactggagaa gataggcatt tgagtggctg agagggcttt tgggtgggaa 8160

tgcaaaaatt ctctgctaag actttttcag gtgaacataa cagacttggc caagctagca 8220

tcttagcgga agctgatctc caatgctctt cagtagggtc atgaaggttt ttcttttcct 8280

gagaaaacaa cacgtattgt tttctcaggt tttgcttttt ggcctttttc tagcttaaaa 8340

aaaaaaaaag caaaagatgc tggtggttgg cactcctggt ttccaggacg gggttcaaat 8400

ccctgcggcg tctttgcttt gactactaat ctgtcttcag gactctttct gtatttctcc 8460

ttttctctgc aggtgctagt tcttggagtt ttggggaggt gggaggtaac agcacaatat 8520

ctttgaacta tatacatcct tgatgtataa tttgtcagga gcttgacttg attgtatatt 8580

catatttaca cgagaaccta atataactgc cttgtctttt tcaggtaata gcctgcagct 8640

ggtgttttga gaagccctac tgctgaaaac ttaacaattt tgtgtaataa aaatggagaa 8700

gctctaaatt gttgtggttc ttttgtgaat aaaaaaatct tgattgggga aaaaaaa 8758

<210> 5
<211> 2761
<212> DNA
<213> Mus musculus

<220>
<223> DMPK mRNA

<400> 5
gaactggcca gagagaccca agggatagtc agggacgggc agacatgcag ctagggttct 60

ggggcctgga caggggcagc caggccctgt gacgggaaga ccccgagctc cggcccgggg 120

aggggccatg gtgttgcctg cccaacatgt cagccgaagt gcggctgagg cagctccagc 180

agctggtgct ggacccaggc ttcctgggac tggagcccct gctcgacctt ctcctgggcg 240

tccaccagga gctgggtgcc tctcacctag cccaggacaa gtatgtggcc gacttcttgc 300

agtgggtgga gcccattgca gcaaggctta aggaggtccg actgcagagg gatgattttg 360

agattttgaa ggtgatcggg cgtggggcgt tcagcgaggt agcggtggtg aagatgaaac 420

agacgggcca agtgtatgcc atgaagatta tgaataagtg ggacatgctg aagagaggcg 480

aggtgtcgtg cttccgggaa gaaagggatg tattagtgaa aggggaccgg cgctggatca 540

cacagctgca ctttgccttc caggatgaga actacctgta cctggtcatg gaatactacg 600

tgggcgggga cctgctaacg ctgctgagca agtttgggga gcggatcccc gccgagatgg 660

ctcgcttcta cctggccgag attgtcatgg ccatagactc cgtgcaccgg ctgggctacg 720

tgcacaggga catcaaacca gataacattc tgctggaccg atgtgggcac attcgcctgg 780

cagacttcgg ctcctgcctc aaactgcagc ctgatggaat ggtgaggtcg ctggtggctg 840

tgggcacccc ggactacctg tctcctgaga ttctgcaggc cgttggtgga gggcctgggg 900

caggcagcta cgggccagag tgtgactggt gggcactggg cgtgttcgcc tatgagatgt 960

tctatgggca gacccccttc tacgcggact ccacagccga gacatatgcc aagattgtgc 1020

actacaggga acacttgtcg ctgccgctgg cagacacagt tgtccccgag gaagctcagg 1080

acctcattcg tgggctgctg tgtcctgctg agataaggct aggtcgaggt ggggcaggtg 1140

atttccagaa acatcctttc ttctttggcc ttgattggga gggtctccga gacagtgtac 1200

ccccctttac accagacttc gagggtgcca cggacacatg caatttcgat gtggtggagg 1260

accggctcac tgccatggtg agcgggggcg gggagacgct gtcagacatg caggaagaca 1320

tgccccttgg ggtgcgcctg cccttcgtgg gctactccta ctgctgcatg gccttcagag 1380

acaatcaggt cccggacccc accccctatgg aactagaggc cctgcagttg cctgtgtcag 1440

acttgcaagg gcttgacttg cagcccccag tgtccccacc ggatcaagtg gctgaagagg 1500

ctgacctagt ggctgtccct gcccctgtgg ctgaggcaga gaccacggta acgctgcagc 1560

agctccagga agccctggaa gaagaggttc tcacccggca gagcctgagc cgcgagctgg 1620

aggccatccg gaccgccaac cagaacttct ccagccaact acaggaggcc gaggtccgaa 1680

accgagacct ggaggcgcat gttcggcagc tacaggaacg gatggagatg ctgcaggccc 1740

caggagccgc agccatcacg gggtccccca gtccccgggc cacggatcca ccttcccatc 1800

tagatggccc cccggccgtg gctgtgggcc agtgcccgct ggtggggcca ggccccatgc 1860

accgccgtca cctgctgctc cctgccagga tccctaggcc tggcctatcc gaggcgcgtt 1920

gcctgctcct gttcgccgct gctctggctg ctgccgccac actgggctgc actgggttgg 1980

tggcctatac cggcggtctc accccagtct ggtgtttccc gggagccacc ttcgcccccct 2040

gaaccctaag actccaagcc atctttcatt taggcctcct aggaaggtcg agcgaccagg 2100

gagcgaccca aagcgtctct gtgcccatcg cgcccccccc cccccccac cgctccgctc 2160

cacacttctg tgagcctggg tccccaccca gctccgctcc tgtgatccag gcctgccacc 2220

tggcggccgg ggagggagga acagggctcg tgcccagcac ccctggttcc tgcagagctg 2280

gtagccaccg ctgctgcagc agctgggcat tcgccgacct tgctttactc agccccgacg 2340

tggatgggca aactgctcag ctcatccgat ttcacttttt cactctccca gccatcagtt 2400

acaagccata agcatgagcc ccctatttcc agggacatcc cattcccata gtgatggatc 2460

agcaagacct ctgccagcac acacggagtc tttggcttcg gacagcctca ctcctggggg 2520

ttgctgcaac tccttccccg tgtacacgtc tgcactctaa caacggagcc acagctgcac 2580

tccccctcc cccaaagcag tgtgggtatt tattgatctt gttatctgac tcactgacag 2640

actccgggac ccacgtttta gatgcattga gactcgacat tcctcggtat ttattgtctg 2700

tccccaccta cgacctccac tcccgaccct tgcgaataaa atacttctgg tctgccctaa 2760

a 2761

<210> 6
<211> 3243
<212> DNA
<213> Homo sapiens

<220>
<223> DMPK mRNA

<400> 6
gccacaagcc tccaccccag ctggtccccc acccaggctg cccagtttaa cattcctagt 60

cataggacct tgacttctga gaggcctgat tgtcatctgt aaataagggg taggactaaa 120

gcactcctcc tggaggactg agagatgggc tggaccggag cacttgagtc tgggatatgt 180

gaccatgcta cctttgtctc cctgtcctgt tccttccccc agccccaaat ccagggtttt 240

ccaaagtgtg gttcaagaac cacctgcatc tgaatctaga ggtactggat acaaccccac 300

gtctgggccg ttacccagga cattctacat gagaacgtgg gggtggggcc ctggctgcac 360
ctgaactgtc acctggagtc agggtggaag gtggaagaac tgggtcttat ttccttctcc 420
ccttgttctt tagggtctgt ccttctgcag actccgttac cccaccctaa ccatcctgca 480
caccccttgga gccctctggg ccaatgccct gtcccgcaaa gggcttctca ggcatctcac 540
ctctatggga gggcattttt ggcccccaga accttacacg gtgtttatgt ggggaagccc 600
ctgggaagca gacagtccta gggtgaagct gagaggcaga gagaagggga gacagacaga 660
gggtggggct ttccccccttg tctccagtgc cctttctggt gaccctcggt tcttttcccc 720
caccaccccc ccagcggagc ccatcgtggt gaggcttaag gaggtccgac tgcagaggga 780
cgacttcgag attctgaagg tgatcggacg cggggcgttc agcgaggtag cggtagtgaa 840
gatgaagcag acgggccagg tgtatgccat gaagatcatg aacaagtggg acatgctgaa 900
gaggggcgag gtgtcgtgct tccgtgagga gagggacgtg ttggtgaatg gggaccggcg 960
gtggatcacg cagctgcact tcgccttcca ggatgagaac tacctgtacc tggtcatgga 1020
gtattacgtg ggcggggacc tgctgacact gctgagcaag tttggggagc ggattccggc 1080
cgagatggcg cgcttctacc tggcggagat tgtcatggcc atagactcgg tgcaccggct 1140
tggctacgtg cacagggaca tcaaacccga caacatcctg ctggaccgct gtggccacat 1200
ccgcctggcc gacttcggct cttgcctcaa gctgcgggca gatggaacgg tgcggtcgct 1260
ggtggctgtg ggcaccccag actacctgtc cccccgagatc ctgcaggctg tgggcggtgg 1320
gcctgggaca ggcagctacg ggcccgagtg tgactggtgg gcgctgggtg tattcgccta 1380
tgaaatgttc tatgggcaga cgcccttcta cgcggattcc acggcggaga cctatggcaa 1440
gatcgtccac tacaaggagc acctctctct gccgctggtg gacgaagggg tccctgagga 1500
ggctcgagac ttcattcagc ggttgctgtg tcccccggag acacggctgg gccggggtgg 1560
agcaggcgac ttccggacac atcccttctt ctttggcctc gactgggatg gtctccggga 1620
cagcgtgccc ccctttacac cggatttcga aggtgccacc gacacatgca acttcgactt 1680
ggtggaggac gggctcactg ccatggtgag cgggggcggg gagacactgt cggacattcg 1740
ggaaggtgcg ccgctagggg tccacctgcc ttttgtgggc tactcctact cctgcatggc 1800
cctcagggac agtgaggtcc caggccccac acccatggaa ctggaggccg agcagctgct 1860
tgagccacac gtgcaagcgc ccagcctgga gccctcggtg tccccacagg atgaaacagc 1920
tgaagtggca gttccagcgg ctgtccctgc ggcagaggct gaggccgagg tgacgctgcg 1980
ggagctccag gaagccctgg aggaggaggt gctcacccgg cagagcctga gccgggagat 2040
ggaggccatc cgcacggaca accagaactt cgccagtcaa ctacgcgagg cagaggctcg 2100
gaaccgggac ctagaggcac acgtccggca gttgcaggag cggatggagt tgctgcaggc 2160

agagggagcc acagctgtca cgggggtccc cagtccccgg gccacggatc caccttccca 2220

tctagatggc cccccggccg tggctgtggg ccagtgcccg ctggtggggc caggccccat 2280

gcaccgccgc cacctgctgc tccctgccag ggtccctagg cctggcctat cggaggcgct 2340

ttccctgctc ctgttcgccg ttgttctgtc tcgtgccgcc gccctgggct gcattgggtt 2400

ggtggcccac gccggccaac tcaccgcagt ctggcgccgc ccaggagccg cccgcgctcc 2460

ctgaacccta gaactgtctt cgactccggg gccccgttgg aagactgagt gcccggggca 2520

cggcacagaa gccgcgccca ccgcctgcca gttcacaacc gctccgagcg tgggtctccg 2580

cccagctcca gtcctgtgat ccgggcccgc cccctagcgg ccggggaggg aggggccggg 2640

tccgcggccg gcgaacgggg ctcgaagggt ccttgtagcc gggaatgctg ctgctgctgc 2700

tgctgctgct gctgctgctg ctgctgctgc tgctgctgct gctgctgggg ggatcacaga 2760

ccatttcttt ctttcggcca ggctgaggcc ctgacgtgga tgggcaaact gcaggcctgg 2820

gaaggcagca agccgggccg tccgtgttcc atcctccacg cacccccacc tatcgttggt 2880

tcgcaaagtg caaagctttc ttgtgcatga cgccctgctc tggggagcgt ctggcgcgat 2940

ctctgcctgc ttactcggga aatttgcttt tgccaaaccc gctttttcgg ggatcccgcg 3000

ccccccctcct cacttgcgct gctctcggag ccccagccgg ctccgcccgc ttcggcggtt 3060

tggatattta ttgacctcgt cctccgactc gctgacaggc tacaggaccc ccaacaaccc 3120

caatccacgt tttggatgca ctgagacccc gacattcctc ggtatttatt gtctgtcccc 3180

acctaggacc cccacccccg accctcgcga ataaaaggcc ctccatctgc ccaaagctct 3240

gga 3243

<210> 7
<211> 14
<212> DNA
<213> Artificial

<220>
<223> Synthetic

<220>
<221> misc_feature
<222> (1)..(1), (13)..(13)
<223> LNA

<220>
<221> misc_feature
<222> (2)..(2), (14)..(14)
<223> 5-methylcytosine LNA

<400> 7
tcagtcatga cttc 14

<210> 8
<211> 14

<212> RNA
<213> Artificial

<220>
<223> Synthetic

<220>
<221> misc_feature
<222> (1)..(2), (13)..(14)
<223> 2'-O-Me RNA

<400> 8
gaagucauga cuga 14

<210> 9
<211> 16
<212> DNA
<213> Artificial

<220>
<223> Synthetic

<220>
<221> misc_feature
<222> (1)..(1), (16)..(16)
<223> 5-methylcytosine LNA

<220>
<221> misc_feature
<222> (2)..(3), (14)..(15)
<223> LNA

<400> 9
ctagttcact gaatgc 16

<210> 10
<211> 16
<212> RNA
<213> Artificial

<220>
<223> Synthetic

<220>
<221> misc_feature
<222> (1)..(3), (14)..(16)
<223> 2'-O-Me RNA

<400> 10
gcauucagug aacuag 16

<210> 11
<211> 16
<212> DNA
<213> Artificial

<220>
<223> Synthetic

<220>

<221> misc_feature
<222> (1)..(1), (3)..(3), (14)..(16)
<223> LNA

<220>
<221> misc_feature
<222> (2)..(2)
<223> 5-methylcytosine LNA

<400> 11
acaataaata ccgagg 16

<210> 12
<211> 16
<212> RNA
<213> Artificial

<220>
<223> Synthetic

<220>
<221> misc_feature
<222> (1)..(3), (14)..(16)
<223> 2'-O-Me RNA

<400> 12
ccucgguauu uauugu 16

<210> 13
<211> 16
<212> DNA
<213> Artificial

<220>
<223> Synthetic

<220>
<221> misc_feature
<222> (1)..(3), (14)..(16)
<223> 2'-O-Me RNA

<400> 13
gcattcagtg aactag 16

<210> 14
<211> 13
<212> DNA
<213> Artificial

<220>
<223> Synthetic

<220>
<221> misc_feature
<222> (1)..(3), (12)..(12)
<223> LNA

<220>
<221> misc_feature
<222> (13)..(13)

<223> 5-methylcytosine LNA

<400> 14
gttcactgaa tgc 13

<210> 15
<211> 13
<212> RNA
<213> Artificial

<220>
<223> Synthetic

<220>
<221> misc_feature
<222> (1)..(3), (12)..(13)
<223> 2'-O-Me RNA

<400> 15
gcauucagug aac 13

<210> 16
<211> 20
<212> DNA
<213> Artificial

<220>
<223> Synthetic

<220>
<221> misc_feature
<222> (5)..(5), (20)..(20)
<223> 5-methylcytosine LNA

<220>
<221> misc_feature
<222> (6)..(7), (18)..(19)
<223> LNA

<400> 16
cttcctagtt cactgaatgc 20

<210> 17
<211> 20
<212> DNA
<213> Artificial

<220>
<223> Synthetic

<220>
<221> misc_feature
<222> (1)..(1), (16)..(16)
<223> 5-methylcytosine LNA

<220>
<221> misc_feature
<222> (2)..(3), (14)..(15)
<223> LNA

<400> 17
ctagttcact gaatgccttc 20

<210> 18
<211> 25
<212> DNA
<213> Artificial

<220>
<223> Synthetic

<220>
<221> misc_feature
<222> (1)..(25)
<223> Morpholino nucleic acid

<400> 18
ggccaaacct cggcttacct gaaat 25

<210> 19
<211> 25
<212> RNA
<213> Artificial

<220>
<223> Synthetic

<220>
<221> misc_feature
<222> (1)..(3), (23)..(25)
<223> 2′-O-Me RNA

<400> 19
auuucaggua agccgagguu uggcc 25

<210> 20
<211> 21
<212> DNA
<213> Artificial

<220>
<223> Primer

<400> 20
atccagcagt cagaaagcaa a 21

<210> 21
<211> 21
<212> DNA
<213> Artificial

<220>
<223> Primer

<400> 21
cagccatcca tttctgtaag g 21

<210> 22

<211> 13
<212> DNA
<213> Artificial

<220>
<223> Synthetic

<220>
<221> misc_feature
<222> (2)..(2), (8)..(8), (12)..(12)
<223> 5-methylcytosine LNA

<220>
<221> misc_feature
<222> (4)..(4), (6)..(6), (10)..(10)
<223> LNA

<400> 22
acctcggctt acc 13

<210> 23
<211> 13
<212> RNA
<213> Artificial

<220>
<223> Synthetic

<220>
<221> misc_feature
<222> (1)..(3), (11)..(13)
<223> 2'-O-Me RNA

<400> 23
ggtaagccga ggt 13

<210> 24
<211> 41
<212> DNA

<213> Artificial

<220>
<223> Synthetic

<220>
<221> misc_feature
<222> (1)..(3), (14)..(16)
<223> 2'-O-Me RNA

<220>
<221> misc_feature
<222> (4)..(13), (17)..(25)
<223> RNA

<220>
<221> misc_feature
<222> (26)..(26), (41)..(41)
<223> 5-methylcytosine LNA

<220>

<221>  misc_feature
<222>  (27)..(28), (39)..(40)
<223>  LNA

<400>  24
gcauucagug aacuaguuca agagactagt tcactgaatg c    41

<210>  25
<211>  30
<212>  DNA
<213>  Artificial

<220>
<223>  Synthetic

<400>  25
ctccaacatc aaggaagatg gcatttctag    30

<210>  26
<211>  25
<212>  DNA
<213>  Artificial

<220>
<223>  Synthetic

<400>  26
gttgcctccg gttctgaagg tgttc    25

<210>  27
<211>  21
<212>  DNA
<213>  Artificial

<220>
<223>  Synthetic

<400>  27
cctccggttc tgaaggtgtt c    21

<210>  28
<211>  22
<212>  DNA
<213>  Artificial

<220>
<223>  Synthetic

<400>  28
caatgccatc ctggagttcc tg    22

<210>  29
<211>  16
<212>  DNA
<213>  Artificial

<220>
<223>  Synthetic

```
<400>  29
gcattcagtg aactag                                                       16


<210>  30
<211>  16
<212>  DNA
<213>  Artificial

<220>
<223>  Synthetic

<220>
<221>  misc_feature
<222>  (4)..(13)
<223>  RNA

<400>  30
gcauucagug aacuag                                                       16
```